# EUROPEAN PATENT APPLICATION

(11) **EP 3 054 012 A1**
(43) Date of publication of application: **10.08.2016**
(21) Application number: 15153717.2
(22) Date of filing: 03.02.2015
(51) Int. Cl.: C12N 15/113, A61K 31/7088, A61K 31/712, A61P 9/00, A61P 35/00

(54) **Long non-coding RNA for the treatment of diseases associated with endothelial dysfunction**

(71) Applicant: Johann Wolfgang Goethe-Universität, Frankfurt am Main, 60323 Frankfurt am Main (DE)
(72) Inventor: Dimmeler, Stefanie, 60594 Frankfurt (DE); Jaé, Nicolas, 63540 Hanau (DE); Boon, Reinier, 60596 Frankfurt (DE); Michalik, Katharina, 60385 Frankfurt am Main (DE)
(74) Representative: Krauss, Jan

(57) **Abstract**

The present invention associates multiple long non-coding RNA (lncRNA) with key functions of endothelial cells. The lncRNA of the present invention are therefore useful as novel drug targets for the manufacturing of medicines for the treatment of cardiovascular diseases or pathological angiogenesis in context of proliferative diseases such as cancer. Modulation of the function or expression of the lncRNA of invention can induce or repress angiogenesis and vessel growth or repair in endothelial cells. Provided are further methods for the modulation of endothelial cell functions in vitro, for example, in the context of tissue engineering.

SEQUENCE LISTING

<110>  Goethe Universität Frankfurt am Main

<120>  LONG NON-CODING RNA FOR THE TREATMENT OF ENDOTHELIAL DYSFUNCTION

<130>  U30608EP

<160>  59

<170>  PatentIn version 3.5

<210>  1
<211>  307
<212>  DNA
<213>  Homo sapiens

<400>  1
ggcatgggag gctcagcagg tctgttccct tggcatgtgg gacatcttgc agtggatgca      60

gggagggacc atgcctctag cattctctgg gaagaataaa ggagaagttt atcttcccac     120

ctacctccca ttgcctgttt ttcattggtc aaaacttgtc ccactaggaa ttaactcctt     180

cacatttcca agtttatatc atatgactgc tccaactaca tgggacccca aaccccaaac     240

ctgatgtatg tatagtgttc ctgtttcatc caagcctgga aatgagggga agagccagaa     300

cctctag                                                               307

<210>  2
<211>  1316
<212>  DNA
<213>  Homo sapiens

<400>  2
gcttggtgct ctggaaaaag aattagaatt gcattcaaat agtcaggctt caagttgctt      60

ctgcctctgt gtgaagcaaa tagtaacaac tcacttcgaa actcaggctc ctggactgta     120

aatgaggata atgatactca ccctaccaac ctcagggact gttctaagag caaaatgaaa     180

tgagactgag ctctgaaaat gcttaaatgc ttttcaaagt agagtgatcg tgctttctgt     240

agcatcaaaa cagtcctcta taaatgggca aagtcaaggc atggactgga tctgacaaca     300

gagaatggct tgagggctgg aaaaagaaaa aaatgcaaat gccccaaatc tctgcttccc     360

ttacagtgtg gaaatcgccc cataacacac tgcaccaggt agggttaaat tgatcccaaa     420

tgtttcagga atttttaatg cccttcgaag agacatttcc aattcttcat ctgcccaaag     480

taatgctgct aacctactat ctgtcagttt cctcagtgct ttgaagattt ttctaaagtc     540

atctgacttg gtttcccttt gatttcaaat tcctgcttat tatatgtatg accttgagcc     600

tttttaactc atctgagcct ctctgtttcc tcatctataa attgtgaata agaagacgtt     660

actgtaggat tattagaaac tttagaaata atacttttaa taccagcata gtgcctgatc     720

cataggcatc caggacatgc cagctaagat cattgattat tgttgtcgtt tttagtatca     780

tacagtagaa agaacaacat ctttgaaacc agacaatccc gcatttaaat cagctctgtc 840

acttacgaaa cgtgtaatcc tgaataactg aagaaaactt tttgaagttc aattttgaga 900

taatgatgga tgatttcaga ctttgtgaga atttcagagg tatcggcagt aggttgaatg 960

gtgaaccctc caaaagatat atctatgttc caaactcaga atctgtgaat gtgacattat 1020

ttgggaaaag ggggttttgc agatataatt aagttaagca tcctgagatg agatcatcct 1080

ggattttcta gatagggcct aaagccaata atgagtgtct aagacagaag aagacacaaa 1140

gaagaagaga cgggaatgtg gtcacagagg cagagactgg agcaatgcag ccacaagcca 1200

aggaacacct ggacccacca gaagctggaa gaggaaagaa gggatttctt tttcttctag 1260

agccttggga gggagcatgg ccctgctgac aacttatttt cagacttcta gattct 1316

<210> 3
<211> 937
<212> DNA
<213> Homo sapiens

<400> 3
gtgagtacta ggtggcttgg cagtgtgtgt gtgtgtgtgt gtgtgtgtgt gtgtgtgtgt 60

ggttgagggg tggagagtta aggggacttc taaggtaagt ccctgattag tcccagccag 120

ttgaactgtg ttgggcaggt atggtggttg agtagttgaa ggtcctagaa gtggacagtg 180

gccacagtgt ctaaggaggc acagaagatt tgtgttgaga taatgattca agagaaaaga 240

aggacccttc atacttcagt cagagaagaa ttcttttggt gggctatcat gctcttgggt 300

taaattcttt tctctgtaac attatttaga aaatcgcctc cctctcaaca atgaagactg 360

agtaccactg gcttacagat atttctgtat attatctgag ttagtagagt ttgagtatca 420

catttctttc cttccaattc tatgatttgt catcataaag ttttggcata cgcagcccag 480

tctttggaag aggggttgag aattgtgtaa tatcaggttt agcattataa agtaacatct 540

atcatctgaa gatgagacta taaacaccag tctggacata atagagcatg cttagcccac 600

ttgtttggaa ctacaaggaa aataaggtca tacaaaagag ccctctgccc ttaaaaaaga 660

attctcaatt gcaaatcact tctgagtacc agtggggaaa tgcagtcatt tattctcaag 720

actgaaaaga gaaaggagac ctttttatca ctttgtgtgt gtgtgtgtgt gtgtgtgtgt 780

gtgtgtgtgt gagagagaga gagagagaga cagaaagaaa gagcaggaga gagagagaga 840

cagagagaga gaatatgtgt gtgtgttacc tacctaaagc taatggtagc caattttttc 900

ctttttttct tggaattttt ttcttaatct aaaacag 937

<210> 4
<211> 302
<212> DNA
<213> Homo sapiens

<400> 4
aacccccgcc tagccccttc acagcaaatg cgctttttgc cctgaagttg tgtgagaccg 60

accttaaact ttctccgacc ctatctcggg atgctacggc tcaggtcgtg gtggtttcgg 120

gaacctcaag acaacattcc ccagctgacc tttgggaact ttaactcggg tcgtcatgta 180

cggaaaggta gtctatgggg tacgcagaat ggaaaagggc ttccacatca gtcgtgtccg 240

aggacgctct ccaacttttg atgtccctgg agagtttctg atatttccct ggagagtttc 300

ag 302

<210> 5
<211> 1486
<212> DNA
<213> Homo sapiens

<400> 5
aaaggatttc ttccgacaga cgtgacccca gaaggctgcc gagggagact ttaggacaag 60

aggcgttgtt tttgtgagaa tgaattttta tactttaccc cgaccccacc ccctaccccc 120

cgcccaggta aatccaagta aatgattttc tgaaggtcgg tctcctagaa agcgtttagg 180

ctctggtttt ttgttttgtt tctattttcg tgttttccca gttagggaag gaagaggagc 240

ctgtgcagag ttgaactggg gtggagaggt gccttgtaaa gtatggtttc cagagatggt 300

gagacctcgt agctaagcgt tgtttcctct gcacgtaact gtgagttgtg aacttgtggc 360

tcctggggga gcgggacagg gcctggcagt gcgggggagg gggcaaggaa ttcaggaagt 420

tggcgagaat tcttcgtttt tcattcttcg ttgtaaattg cttctaaaca cttctgaatt 480

ttaaattgct accgagaagg tccagaaacc gttctcatcc aatttagtca cttttattac 540

gttccacttt gcctccccca atcactgcgg cggctcccag tttcagcgtg gccgcatttg 600

gaaaaggctg aaggtagaaa actctggaat ggagaggctg gcgactggag caggatccag 660

gggctttaaa ttttaacctg aatgcgatcg aatgcgcgac tcccagaaga gatagccagg 720

cgtccgcccg gccgctcaac ccccgccagg actcggaatg ccttgctttt ggttcttttc 780

gtgagttccg accaaatgtt taagtggact gggttttgca gaagctcatt tgtaagcact 840

gacggaaatg ggttgtgggc atttgatttc ctttgattcc aacgttttga aataatgttt 900

ctcggatttg ctattcgaag caattttcta aaaactcata tcttatttgc taaaaataaa 960

ctctttggct cagtttgtgt ccaggctgtg tacggcctcc aaccctgagc cccgaggaga 1020

tcgagccctg gcgcgagcct gatctctgtc ctgtgtgttt ctttgtattt gcaagtacaa 1080

ggatgtctgc gtggatatcc ctggacactc gatacccccc ggggacctgc ttttcccgca 1140

gctccgcaga cgcggcggga gggcgcgctg gccccggcgc tgctgggaca gaggtgagca 1200

gcggggtgga cattgaccga ttcgacaacg ttcgggctct aagggctcgg gaagggaccc 1260

ggcattttac cctccagatt cccaatctat ttttaaaaat aatgttcaaa acatatggga 1320

aaaataagtg attgctcctt ttcatggtca cagattgtat ttattttttc agactggttt 1380

tccaacctaa cgagggaatg ggtagtgaat gttgttgttg ttgttgttgt ttatgttggt 1440

taatttcgaa ataaaactgt ttaactcgta cagttgattc agacag 1486

<210> 6
<211> 283
<212> DNA
<213> Homo sapiens

<400> 6
gtaagtcaca gataagtcac tgcgaagagg gggtgtcacc ttccctggac ccagcgctaa 60

atgctgcttc cccaccccca cgttcatata cacaccccct cttcgctccc tccaaacagt 120

tatcacaact ctgtacaaac tgatacggtc cctagcgggc aaaagggact ggaaatgaac 180

gtcgcggggt tatcagcgcc gatctgtcag cggcaactcg cgcggccgca ggctcgtccg 240

ctgcaggagc cgtgatatat gtctcgctcc ctttcgctta cag 283

<210> 7
<211> 339
<212> DNA
<213> Homo sapiens

<400> 7
cattcttcgt tgtaaattgc ttctaaacac ttctgaattt taaattgcta ccgagaaggt 60

ccagaaaccg ttctcatcca atttagtcac ttttattacg ttccactttg cctcccccaa 120

tcactgcggc ggctcccagt ttcagcgtgg ccgcatttgg aaaaggctga aggtagaaaa 180

ctctggaatg gagaggctgg cgactggagc aggatccagg ggctttaaat tttaacctga 240

atgcgatcga atgcgcgact cccagaagag atagccaggc gtccgcccgg ccgctcaacc 300

cccgccagga ctcggaatgc cttgcttttg gttcttttc 339

<210> 8
<211> 330
<212> DNA
<213> Homo sapiens

<400> 8
tacaaggatg tctgcgtgga tatccctgga cactcgatac cccccgggga cctgcttttc 60

ccgcagctcc gcagacgcgg cgggagggcg cgctggcccc ggcgctgctg ggacagaggt 120

gagcagcggg gtggacattg accgattcga caacgttcgg gctctaaggg ctcgggaagg 180

gacccggcat tttaccctcc agattcccaa tctattttta aaaataatgt tcaaaacata 240

tgggaaaaat aagtgattgc tccttttcat ggtcacagat tgtatttatt ttttcagact 300

ggttttccaa cctaacgagg gaatgggtag 330

<210>  9
<211>  295
<212>  DNA
<213>  Homo sapiens

<400>  9
gtgagttccg accaaatgtt taagtggact gggttttgca gaagctcatt tgtaagcact      60

gacggaaatg ggttgtgggc atttgatttc ctttgattcc aacgttttga aataatgttt     120

ctcggatttg ctattcgaag caattttcta aaaactcata tcttatttgc taaaaataaa     180

ctctttggct cagtttgtgt ccaggctgtg tacggcctcc aaccctgagc cccgaggaga     240

tcgagccctg gcgcgagcct gatctctgtc ctgtgtgttt ctttgtattt gcaag          295


<210>  10
<211>  108
<212>  DNA
<213>  Homo sapiens

<400>  10
gccgtgatat atgtctcgct ccctttcgct tacagaaagg atttcttccg acagacgtga      60

ccccagaagg ctgccgaggg agactttagg acaagaggcg ttgttttt                  108


<210>  11
<211>  119
<212>  DNA
<213>  Homo sapiens

<400>  11
ttagggaagg aagaggagcc tgtgcagagt tgaactgggg tggagaggtg ccttgtaaag      60

tatggtttcc agagatggtg agacctcgta gctaagcgtt gtttcctctg cacgtaact      119


<210>  12
<211>  200
<212>  DNA
<213>  Homo sapiens

<400>  12
tttcagcgtg gccgcatttg gaaaaggctg aaggtagaaa actctggaat ggagaggctg      60

gcgactggag caggatccag gggctttaaa ttttaacctg aatgcgatcg aatgcgcgac     120

tcccagaaga gatagccagg cgtccgcccg gccgctcaac ccccgccagg actcggaatg      180

ccttgctttt ggttcttttc                                                 200


<210>  13
<211>  54
<212>  DNA
<213>  Homo sapiens

<400>  13
tacaaggatg tctgcgtgga tatccctgga cactcgatac cccccgggga cctg            54

<210>  14
<211>  148
<212>  DNA
<213>  Homo sapiens

<400>  14
   gtgagaatga atttttatac tttaccccga ccccaccccc taccccccgc ccaggtaaat      60
   ccaagtaaat gattttctga aggtcggtct cctagaaagc gtttaggctc tggttttttg      120
   ttttgtttct attttcgtgt tttcccag                                        148


<210>  15
<211>  240
<212>  DNA
<213>  Homo sapiens

<400>  15
gtgagttgtg aacttgtggc tcctggggga gcgggacagg gcctggcagt gcgggggagg      60

gggcaaggaa ttcaggaagt tggcgagaat tcttcgtttt tcattcttcg ttgtaaattg     120

cttctaaaca cttctgaatt ttaaattgct accgagaagg tccagaaacc gttctcatcc     180

aatttagtca cttttattac gttccacttt gcctccccca atcactgcgg cggctcccag     240


<210>  16
<211>  295
<212>  DNA
<213>  Homo sapiens

<400>  16
gtgagttccg accaaatgtt taagtggact gggttttgca gaagctcatt tgtaagcact      60

gacggaaatg ggttgtgggc atttgatttc ctttgattcc aacgttttga aataatgttt     120

ctcggatttg ctattcgaag caattttcta aaaactcata tcttatttgc taaaaataaa     180

ctctttggct cagtttgtgt ccaggctgtg tacggcctcc aaccctgagc cccgaggaga     240

tcgagccctg gcgcgagcct gatctctgtc ctgtgtgttt ctttgtattt gcaag          295


<210>  17
<211>  60
<212>  DNA
<213>  Homo sapiens

<400>  17
cgacagacgt gaccccagaa ggctgccgag ggagacttta ggacaagagg cgttgttttt      60


<210>  18
<211>  134
<212>  DNA
<213>  Homo sapiens

<400>  18
tttcagcgtg gccgcatttg gaaaaggctg aaggtagaaa actctggaat ggagaggctg      60

gcgactggag caggatccag gggctttaaa ttttaacctg aatgcgatcg aatgcgcgac     120

tcccagaaga gata                                                    134


<210>  19
<211>  507
<212>  DNA
<213>  Homo sapiens

<400>  19
gtgagaatga atttttatac tttaccccga ccccaccccc taccccccgc ccaggtaaat      60

ccaagtaaat gattttctga aggtcggtct cctagaaagc gtttaggctc tggttttttg     120

ttttgtttct attttcgtgt tttcccagtt agggaaggaa gaggagcctg tgcagagttg    180

aactggggtg gagaggtgcc ttgtaaagta tggtttccag agatggtgag acctcgtagc    240

taagcgttgt ttcctctgca cgtaactgtg agttgtgaac ttgtggctcc tgggggagcg    300

ggacagggcc tggcagtgcg ggggaggggg caaggaattc aggaagttgg cgagaattct    360

tcgtttttca ttcttcgttg taaattgctt ctaaacactt ctgaatttta aattgctacc    420

gagaaggtcc agaaaccgtt ctcatccaat ttagtcactt ttattacgtt ccactttgcc    480

tcccccaatc actgcggcgg ctcccag                                        507


<210>  20
<211>  666
<212>  DNA
<213>  Homo sapiens

<400>  20
tgatgtttga gccccagcat agatcagcat gccaggaagc agcccaccgg acccacccct     60

gatcatggca ctggacattg ttttccgtgc ttttctcagg ctggtttctg cctgtcccaa    120

ccctggccat ggctgacaag gggcccaggg acactggagc agagagacct gctggtcctt    180

ggggtttctt gcaaggtagg actctacatc ctcccttggg aactaattga tctgtcagta    240

aaatgtaaac ttctgactcc atgggtctgt gaatcagctg cctctaaagg gaaatatccc    300

aggagtgatg aaaacttgaa gatcgaggat tggatataag ctcagaggtg aaggagggaa    360

attcctcggg tcaactttag cggactgatc cacggggtct gccaagagac aggagctcga    420

gggttaaacc caagcactcg ggagtcagat gtatgaatct gaatctcagc ctctcggctt    480

gctgtgtaat ctcaggcaag tgacttttct gttctgtaaa tcaggctgat agcgggggaa    540

cctctctcac agggctgcta caaagattgc atgagatttt gctcaacaaa tattagctgt    600

tggctccaga gtttaatacc tctgatcact acaagctttg aaggcaagat aaaataactt    660

caacat                                                               666


<210>  21
<211>  538
<212>  DNA

<213>  Homo sapiens

<400>  21
agtgaatcat ctgtgaacac ttcctgtgaa cgtgtgacta tgattatgga acactctgtt      60

gcatcaagat ggccagagat tgtctcagag tccaggcagt gggaggaggg gggatttgtt     120

tcctggctgg gtggtcgtag ggcatcaagg gatgagcagc tttggttgtc tgggatttca     180

gcagcttgag gggagctgta gggccccaga gctgtcgctg tggctgtggt gtcagaaatg     240

taagcaaggt gcctccatgg aggttagcga gggtgcccct ctgaatgccg ggtggaatgc     300

aacctttcgc atggaggagg ccctcagcag ctgacctctg tcttccctgt gtagagggag     360

ggaggctgag gctggggctg agcgaggggt aaagtaagcc acgtgtctca tcagctagcc     420

tgcagcaagc acagccctct gggagggctg gttctatcct cctccttggt gtgaaatcca     480

tcaatctaat agggatcctt attttcccag ccaccaagtc acaatgagca ataatctc       538


<210>  22
<211>  13683
<212>  DNA
<213>  Homo sapiens

<400>  22
gtgagtacca ggaggccttg gtgaaaagca agcctgccgg gagataatgc ctggctgggt      60

caggattctt ggtgcttatt gaccactggg cacttcagag cagaactcat caaagcctct     120

gtattgggac aagggctagg gtggagggga ggattgggaa gggagagggg agactgtcct     180

tctttctact aaaagttact ggcaagagaa tgggaattaa ccaggcctgg aaaagtaatt     240

taggtattag aactttgaac tcttttttta aaaacaaaag acaaaaacag ctttattggg     300

atataattga caaataaaaa ttgtgtatat ttaaggtgta caacttgatg ttttgatata     360

tgtacacatt atgacatgat cactaccatc aagctaattc acatatccat cacctcatac     420

agtcactatt ttcttctttg tgtgtgtgag agatgagaac acttaagatc tatcctcagc     480

cggatgtggt ggctcacgtc tataatccca gcactttggg aggctgaggc aggcgaatca     540

cgaggtcaag agattgagac catcctggcc aacatggtga aaccccatct ctactaaaaa     600

tacaaaaatt agcttggcgt ggtggcgcgt gcctgtagtc ctagctactc ggaaggctga     660

gccaggagaa tcgcttgaac cggggaggcg gaggttgcag tgagctgaga tcgcaccact     720

gcactccagc ctgatgacag agtgagactc catctcaaaa aaaaaaacta tcctcttagt     780

aaatttccag tatataatac agaattgtta actatagtcc cacgctatac attagatctc     840

cacaacttat tcatattgca taactgaaac tttgtaccct ttgaccaaca tgtccccatt     900

ttcccgcccc tgagctgctg gcaacagcaa ttctactctc tgcttctatg agttcaactt     960

ttttagattt cacatataag tgacattata cagtatttgt ttttctgtat ctggcttatt     1020

tcacttaaca taatgtcctc caggttcatc catgttgccg aaaatgtcgg gattttttaa    1080

ggctgaataa tattgcattg tacgtatata ccacattttt aaaatccatt tatccattga 1140

ggtacatttg aatggtctac cctcctaacc aaggccaacc ctttagccca actaagctct 1200

tggaaaatgc ccaaagttgc tgcaaagccc ctggctaaat ccttctcatc ctgagactcc 1260

gcttcagcgt tacctcccca gggaggcctg ggctgggttt gatccctaag tcaacccata 1320

cggcactttg cattgttaaa agaaatactt cagccaaatt aaatttaaag gagtttaatt 1380

gagcaatgaa cagtcaggca gcccccaaat cacagcagat tcagagatac tccagcgcag 1440

ccacatggtg gaaggaaatt tatggacaga aagaggaaag tgacgtacag aaaatagaac 1500

attttcatat tgttcagtga gtgtcagggg ccccaaagaa tcatgattcc acagcataat 1560

cacagctaac atgtatttgg tgctctctat gtaccaggaa tggcataagt gttgtctagt 1620

gaattcactc aacaactctg tgaaatagtt tcccttctca cttggttttt agttaaaaaa 1680

aaaaaaattt gtagagatgg ggtctcacta tgttgcccag cctggtctca aattcctggg 1740

ctcaagtgat cctcctgcct tggactccca aagtgttggg atgacagatg tgagccacca 1800

tgcccagacc ccttcccact tttgcatctc taagctgcca ccttgctgac ccacacacag 1860

ctgtgggtga aatccaatgg gctcagcagc catgagctaa tagcactgct cctcctgcgt 1920

agagaaagat ttattgtttc gaacctttga cttagcttag aaaacaaaat cagtaggccc 1980

cactcttcaa tgtttttaag gtatggtctc ccaggtggat tctgagacag ctttcatctc 2040

tagggttaat tagggtctgg ccattgctca tgcgaagata gaagactaaa acacgcaaat 2100

ctaatgcccc atctttggct tccttgggaa cagggggagg ctggggtggg atggaatgga 2160

aaacacagag aagcaaatag cccacccaag aaagtgtgag ccctcctgcc ttcactacct 2220

ctggccacca aaagagacca aacaaagggg ctaggggaag gaaagaattg ggtgggggta 2280

aaagtggggg tcttagtaaa ctctaaactc ttagaaataa atgtagagtt cacattccac 2340

ctgggcttcc aatgtccttc tcagaggcag tcagggacca ccaaccaggg agcgcgtatg 2400

agggctcagc ttggttctag aacctttgta aactcatgct tgctattttg atgatcactt 2460

acctgatttt ccagaggaaa actaacccag ggaagcaggg aggagaggcc catgagcctg 2520

tgttggaaga agaggaggag aggtcagcac tccaccttca gccatgtcct agccagagag 2580

gggagtgaca gctttctcaa agagaggaaa ctgggcagat ccctgaggac ttgatcaagg 2640

atgcaaggca ggggagcaca ggggggctat gggctgagag gggcctggag ggtggcagtg 2700

gctgggccta gaggtgggct ccgtaatgac acagggctgg ggcaatggaa gcgcctagct 2760

caggagcatg gtggatgtga gtgtatatcc gtccacccct agctagcaat tcactagctg 2820

tgtgaccttc ctctatctcc actgattgtt tccaagcctg agtttcctca cctgtgaaat 2880

ggagataatc cagcaattgt gacgagaaat gtgagaaaat gcatgtctgc agctattaca 2940

gggtctgacc tggagtaaaa gctccatgaa tgccggctgt cctgtggcct ccccgacacg 3000

tcccccctgc tggaaggctt tcctcaccat ctccctcata aacaaagcca aggtcaaggc 3060

tgcagcccat ggtgagagca gggagagctt gtgggaagtt cactgccccg ccatgcccct 3120

gaatggtgag cctctttctt tgatgtgggc taacatccgt ggactcagag gcccagaaag 3180

tgcgagacac gccaaggctc ggcccctggg cgtctgtttc ctgagcagcc tgggacacag 3240

gttgccctgg tgagatgacc cacagtcagc accctaggcc ttggcattat gcgggcagct 3300

ggtatgagcc ctgcctcacc ctgcacagaa tttgccagct tcacagcatt cctgccagcc 3360

cagcctgctc ccagacccag ccaagtttca ctcaggcagc ctgcattccc attttgtgcg 3420

ccttgtcctg cccagaggca ccggctgctg agggaagcat gaggtccttg gaaaggagct 3480

gccccagcag gccggtgtgc tgggagccct cagcctcttc tacactcccg ccacccccag 3540

ccctgcatcc tgccccaagc tggctgcctg ccagcttctc gggtctcatt tacttactgc 3600

tgtttgtttt ggaggcttct gggaagcaag gctccacctg aggaatcttt ggctgagggg 3660

acttgtgaca cctgagactt tgaactttca agaaaacata aggctccgct ggccacttcc 3720

ccgggtgtgg ggacaaatgt cactgcagtg ccgtgggctt gcaccaggcc cctcctcctc 3780

tccaaagcag ctaaggcaga gctcatggac agggacctgc ctgaagcagc cgccatctgg 3840

gggttgacgg ctgcctggtc tcttgctcta tctggaaaaa gaaaccataa gcatttcata 3900

tttatcttat aattcatttg gatttgtttt catataaaag taatgtctaa gggaggatta 3960

gtcaagtaaa ttgtgcacat gttcttgtga tgacctatta gcagatgcca cagatttacc 4020

ctagtttgag aaacaaaggt gagaagtatc ttttgtagac caggtgcggt ggctcacacc 4080

tgtaatccta gcactttgga aggccgaggt gggtggatta cctgaggttg ggagttcaag 4140

accagcctgg ccaacatggt gaaactccgt ctttactaaa caaaaaaaaa aaaaaaaaaa 4200

ttaactggac gtggaggcac tcgcctgtag tcccagttac tcgggaggct gaggcagggg 4260

aattgcttga acccaggagg cagaggttac agtgagctga gattgtgcca ctgcactcca 4320

gcgagattct gactcaaaaa aaaggagtat cttttggaaa acctctgttc aataattgac 4380

ttttaaatgc acaaaaaatt ggaaatacac caatttttac tttttttttt tttttttttt 4440

ttttagagac aaggtcttgt tctgttgccc aggctggagt gcagtgacaa gatcatagtt 4500

cactttggcc tcaaacttct ggcctcaagc aatctttcca cctcagcctt ccaaacccct 4560

aagattacag gcatgagcta cccagcccag acctcaattt ttaaaaattg taaaatgaac 4620

ttttaaaatc cctattaagg ctgggcatgg tggctgactc acgtctgtaa tcccagcact 4680

ttggaaggct gagacaggta gacaaggggt ttgagactag catgggcaac atggtaaaac 4740

cccatctcta caaaaaataa aaacaaaaca aaacaaaaac acaacaatta gcccagcata 4800

gtggcatgtg cctgtagtcc cagctacttg agaggctgag gtgggaggtt tgattaaacc 4860

tgggaggtcg aggctgcagt gagccaagat catgccactg cactccagcc tgggcaatga 4920

ggcaagaccc tgtctcaaag aaaaaaaaat ccctattaaa tttggtagaa ttgtaattta 4980

agcaggttta ttatagtgag tttggaaaga ataaagaaaa agaactaagt tactccctca 5040

cacagagata acccttttgg tgtacatttt ggtatattta agatatcata caatatatac 5100

ttctttggtc atagttagca ctcttatcta agtatttctt cccaatcaat caattaatgt 5160

tcagcaagat tttttatttt tatttttttg agatagagta tcgttctgtc tcccattctg 5220

gagtgcagtg gcgcagtctc ggctcactgc aacctcacct cctaggctga agcgattctc 5280

atgcctcagc ctactgagta gctgggacta caggcatgtg ccaccatacc cagctaattt 5340

ttgtattttt agtagagatg ggggtttcac catgttgccc aggctgctct caaactcctg 5400

gcctcaaatg attcatctgc ctcggcctcc caaagtgctg gaattatagg catgagccac 5460

cgtgcccggc tggtaagatt atttttattt tttagtggca gcatagtcca ctgtgtggac 5520

gaacaaaagt tttgtttgtt ttttgagaca gaatctcact ctgtcaccca ggctggtgtg 5580

cactggcatg atctcagctc atgcaacctc tgtgattctc ctgcctcagc ctcctcagta 5640

gctgggatta caggtgccca ccaccatgcc cagctaattt ttgtattttc agtggagacg 5700

gggtttcacc aggttggcca ggctggtctt gaactcctga cctcaagcga tctgtccgcc 5760

tcagcctccc aaagtgctgg gatcacaggc gtgagccact gtgcacagcc taaaaatttt 5820

ttaaccgttc attttttgac atttaagttg ttcctatttt ttgctgttat aaaaaacatt 5880

caatagccac ttttgcacaa acatctttgc acatttatct gagcacatac ttaggataaa 5940

cttcataaaa ttggaattgc tgggtcaaag gataaggaca ttttaaggtt gttggtacac 6000

cctgccttgg ctggtttctt cctttgcttc tatgaaaacc attgccacct tgagatcagg 6060

gacgtatttg cctaaatttt cagtagtgct atgattttgt cattagatgt ttagattata 6120

attcatctgg actaatcata acactaataa taatatgagg taatacttat tgggtgggga 6180

ctatgagttg gacattgggc taagaaattc ttatgcataa tcttacttca cccctactat 6240

tatgttcagg gaactatttt tatttcatct tacataggaa gaaactaaga ttgtgagttt 6300

aacttgggat gaggcaggga tctagcttta tttttatttt cctttctttc tttcttcctt 6360

cctttcttcc ttaccttctt tccttctttt gtttccttct ttctttcttt ctttctttct 6420

tcctttcttc cttcctttct ttttctttct ttctttcttt ctctctctct gtttctttct 6480

ttcctttctt ttctttttta aagacagggt ctcactctgt cacctaggct ggagtgcagt 6540

ggcgagatct cagctcactg cagtcttgac ctccctgggc tcaagtgatc ctcagcctcc 6600

caagtacctg caaccacagg cacacgcccc ccatgcccgg ctagcttttc ttgtgtgtgt 6660

gtatgtgggt ttgtgtgtgt ttgtgtgcgt gtattttgta gagatgggat tttgccatgt 6720

tgcccaggct agtagctttg tttttccata ttattccaac atcatttatt aaacaatgaa 6780

tctttccatc actgatttga atatcccatt cagcatacac taccaatttc ttttatacaa 6840

taggatctgt tcctgggcct tctcttcttt tccactgagc tgcctgtcct ttttccattt 6900

cacacggttt tttgtttttt gtttaccact gaagctttaa aatgtatttt actgtctggg 6960

aatgcagaac caccctgttc tttttctccc agaaatttta tgattctatt catacataaa 7020

tttatttcca ggtgaattca ttttgtcaca ttaaaagtaa atcttagata tttttattag 7080

aattgcataa gacttatgca tttgggcaaa tggcatttga cagtgttgag ttctcggtgt 7140

ggtgtgtttt gccttttatt taatgtccct cagtgtcatt cagcagattt tttgttatga 7200

gttttgcaca gttcctgtat acttcattcc ttggctttgc tattgtaagt gggaactttt 7260

tgcattatat tttgtggcca gttcacccta gtgtatagaa agctatgaaa tgggtactag 7320

tgtttctcgt ttacagatga aggagtagaa attaagggca cagtgccagg tgagactcta 7380

gttgaggaca gggctattca gaggtgcggc cgtgtctgaa cctgcctcag tcataaccac 7440

tctcttagaa ggaagctgcc cgtgctctcc aaagacagtg tctggtcaga cccagcttgg 7500

tgtctgctgc ctggcttggt gctcctggct tggtgtctgc tgcctggaca gttccagatt 7560

caagggcagc caaacatttc tgggcagcaa gctccctggc ctgatcaagc tctgggccat 7620

ggctgtgccc cgcaaacccc agaacccaat aaaaggagtc cccattaggt gggcatggct 7680

gcaggtgact gttgcacagt aggactttgg accacttatt tgaaagctgc tgttcttact 7740

gttcagcatc agagagaatt tgatgaactc tatggactct cagacaaatg ccccaaatgc 7800

tgttgacact ttcagaggat gtatggacaa ccccaaaaca cacccaagga tcttgggtta 7860

aattaatatt cctgtgttaa aacaatggcc cagacaggga ttcctccagg tgggaggtgc 7920

ccagggatgg ggtcacgttc agaggacaga agtccttgtg tctgtgtgac tttggatggg 7980

tcacttccct ctctgaatcg ttattttctc catgagaaca atatctaccc agtctagtta 8040

tggtgttgtt tgggaatcgt ctggtatcct gtcttgtgac agtgctttga aaactgcagt 8100

taagtaacac aaaggtaaga aatgactcat gccaggaaag ggaatagcat gaggcccagc 8160

tctgatatgc aaaggcctct cagattctta gagaatatta ttggctctca caccaccagc 8220

tgctcccagt gaacttggcc ttcgccccca ggaatggctg ataaaggcct tcactgcttt 8280

ccacttctga gctgatggga ctgttttagc tgacccagct aaaaccatga ttaagtggct 8340

ggatttggaa aagaagtgtt ggggctcatg gttcacagat acgtttctgg gttgttataa 8400

aaattaataa ttaggctggg tgcggtggct cactcctgta atcccagcac tttgggaggc 8460

cgaagcagga ggaccacttg aggtcaggag ctcaagacta gcctggccaa catggcgaaa 8520

ccctgcctct actaataata caaaaattag ccggatgtgg tggctcatgc ctgtaatctc 8580

agctactcag gaggctgaga caggagaatc gcttgaacct gggaggtgga ggttgcagtg 8640

agctgagatt gtgccactgc actccagtct gggtgacaga gtgaaaatat gtctcaaaaa 8700

acaaaattaa taattaatcc tctcctgcat gctggagggg aactgccagc agcctccgag 8760

ttatggagag cactcctggc caccaggctc cccgtgagtc ctggctccgt gtgagcccag 8820

gccagccctc ctctctactg ccctccactg ggtccccatg gtaaaataag gggctgcact 8880

tgatgaaagt gaatttgcaa ctgaaaaaat atgtaataat aaagccagat cccttctaag 8940

agactttaaa ggtcttccaa cttgtcttct tcctgctccc agtagattat gtcccctgtc 9000

ctcatgcccc agcgtgaact ttaggccctg gacacatcag cttgcacaca catgtgtgtt 9060

tgtgtggggg tgggtgggtg tgcatgtgtg tccttggggg aagctggggg aaaggggcaa 9120

aagagaaccc tcactgggaa cctttttcta aatacaaatg gctgccccct tctccctggg 9180

aaatatctga atggggtgag ggatatactg ctttggtctt ccgaccatcc agatccccat 9240

ctccagactc tccagccaga gggcagggca gtagggggac tggcaggact attttgaacg 9300

cctggttctc agaaccacct ccctgcagtg ctcctgttca gaggtcagag gtgtgcagcg 9360

tcctttcatc ctctccttgg agagcaagcc tgatgaggca gggaatatct ttctgactcc 9420

ccggttcctg gctccatggc aggcatgcaa cagaagctct cggacaaata aatgggacct 9480

tcccccatac tttgcctaaa ccttcttatg tcccttctct gctccctctt taccaatgtg 9540

ggtatctcct ctcctcccaa cttctgctca aattgtagct tctggggatg cctccagctg 9600

aacccccagg cctccccaga tctgtggccc cagccctgtg tccctgcatc tttgctgatc 9660

ccagccagaa gctgccaggt atgcattttc atcaacagta gtgaggacta tttattaatt 9720

tatttatttt tgagacagag tcttgctctg tcactcaggc ttgagtgcaa tggcctgaac 9780

tcggctcact gcaacctctg cctcccaggc tcaagagatt cttgtgcctc agcctcccga 9840

gtagctggga ttacaggcgt gtgccaccac gcccggctaa tttttgtgtt tttagtaaag 9900

acagggtttc accatgttgg ccaggctggt cttgaattcc agacctcaag tgattcatcc 9960

tccttggcct cccaaagtgc tgggattaca agtgtgagtc accatgccca gccagtaagg 10020

actattcgtt aagcagcatt atagatcacc tgtgtatgtc tgctaaaaaa aaaaaaaaaa 10080

aaaaaagggc accctctcta cccagatgag caaaggtatt ttttctgata cctgtaacaa 10140

atctaatatt gccacaattg ctacatgatt agtgagagaa gcatttgata ggctaatcaa 10200

ttgattggtc tggggtaaat attcagtgaa tccaaagttc aactctaatg agccacttca 10260

cttctcctgg gccctcttgg taatgtggta ccaggaactc tggaagtcct catggcattg 10320

ctcaagggag tggtgggatg ggtccccagc tgctctttgc acagctctct gctctaggtg 10380

actgtctggt ccatgagctg ggctggccca gtctgtctct ggcaatcctg gggacatctg 10440

ctgttgacat ccttggtgag tgtaacctcc agcttacaca acccattgga cacctcctct 10500

gctctcaaaa actctgctga cccccaaagt atggttgtga ctcccattaa gcccaagacc 10560

cacgtagacc ctatgccccc agccagatcc tcattggaaa cccccatggg aacatacaag 10620

aaagtggatc tcttccagtt cctcccacat tacgtggcat caagggcttt gggaatgtca 10680

aaccaaatac tcccctgtcc tcagcagagc accctcccct ttcccaacgc ccccccgccg 10740

ccaccaccag gctaacgaag ttctaaatgg gggtcagggc acaaaatact ctgtgtttgc 10800

cttttctctc aatctattca actgctcacc ccacccaact cccaccaatc cagtcctcta 10860

ccctgggact ttagcctgca gtaggaagat cagcccacga gtgtgaccac ccaggtcccc 10920

aacctcagcc catcttcttc ccagtttcct gggcctggag gggagctcta atccctttca 10980

gttttagcct tgatatcctt aacatctttg ccactagaca gtgggccacg gacgagctgc 11040

atcagtatcc ccagagctta ttagaaatgt agactctcaa gccccaccat ggacttgcgg 11100

aaccagggct tgtatgttaa caaggtgccc gggtgattca catgcacatt aaagtcagag 11160

aagcggtgct ggcttgcagc acatcctggc agcgtgttct ccttagctta tgtcagactc 11220

ctggatctgg tccgccaggc ctggttctta atatgttagt ggaactcagc aaatttagga 11280

aacccttctc tctcttgtca aagtttgact tctagatgct gcagactcaa ctatcttgcc 11340

tccatgcctt ggatgtcact ggctgcataa ggactttgtc tttctcttga cattcttgct 11400

gtaacaatcc cagtcctcct cagtccattc ttcttgaaaa tggcactggg gaaagaggca 11460

aatatacaga atggagaaag agagaaacac atgaacatac ttgaaaacta ctctcccagc 11520

tacaatagct tttgctgact tgagcttcaa gtgatgccag caggctacat ggagggtaga 11580

ggtgagaagg ggtggcacac acttcacaga tgtcacagag gaggacgctc cttctaccac 11640

actgtccctt gtctaaggac gcaaaggaga aaagccttgc cttagcatcc agagtggttc 11700

aatattacct gagcaagtga actgccttta ttttacaggc ctctagggca taactatcca 11760

tttgcccttt tttgttttgg ccactaggaa gcttagagac agatttgtgg tccaacaacg 11820

gtaagtgtat gatcatgtct aattctcatg caggactgat ccctggcttc accctagctt 11880

gtcttcttcc tccttgcttt taacatatgc attttattat aaccatgtat tcgtataagc 11940

cactttaaat ctctcctgaa gccaagtgaa ataactttgt aagtaggtac tgttctaggt 12000

ccttgcctat ggcgtattcc ctcttgatct ttgccattgt taacatcccc attgcacaga 12060

tgaggacact gaagcgcaga gaggtgaaac aagcaagcca ggctcagggc aacggtgagt 12120

gtccaagcca gggtatctca gctctctgtg ttcagagttt gggccccaga agggaccacc 12180

tggatcctgt cttggcagca ggcaggccaa cacggcgctg agagggagct gaggggaggt 12240

gtgggtcaga tttctggcaa gccctgaggc tgtcagtgaa ccgctgtggg gtccccccag 12300

catgtgcttt ggggccacac cctaatgggg gtcttcttta tgttatgacc actcctgcag 12360

ttaatcaacc acaaggttgt tttttgttgt tgctgttttt tctgtttgtt tttttttttt 12420

tttgagacag agtttcagtc tgttggtcaa gctggagtgc agtggcacaa tcttggctca 12480

ctgcaacctc catctcccag gttcaagtga ctctcctgcc tcagcctccc gagtagctgg 12540

gattacaggc atgcaccacc acgcctggct aatttatgta tttttagtag acacggcatt 12600

tcaccatgtt ggccaggctg gtcttgaact cctggcctca agtgatccac ccgcctcggc 12660

ctcccaaagt gctgggatta caggcatgag ccaccgagcc tggccttaag cacgagattc 12720

ttaaggatag cactgggacc ggtgattctg tgactggagc tgattaactg ctttgtggga 12780

ataagtaagg gcttcaaaga tgtgtgccca tctcggctgc caggccctga gttcttgagg 12840

tggaagctga ctctccacct ttctggctga tctaggcagg ctagttccct gaagactcaa 12900

ggtaagctgc cttctagaat gaacttggga tgaaggtaaa tgatgacaca gggcagctgt 12960

ctgaaaaaga aaggtttatt tatctaaacc aacaagaaag gattcttttt aattggaaaa 13020

acaattaaag aatagtgcat gtattcagat cctatttttc ccagtgtgtt tttgttgttg 13080

ttgttgttgt ttttgagatg aggtctcact ctgtcgccag gctggaatgc agtggcgcaa 13140

tcatagctca ctgcaggctc cacctcccag gctcaggcaa tccttccact tcagcctcct 13200

gagtagctgg caccacaggc acacaccacc acgcccagct aattttttgc atttttgggt 13260

ttcaccagtt gcctaagctg gtctcgaact tctgagctca agtgatctgc ccacctcagc 13320

ctcccatagt gctgggatta caggtgtgag ccattacacc cagcctacgt gtgttttaaa 13380

tacacatact cagatggatc cactcagaca cacacacaga cccccataca cagagacact 13440

ctatccccat cccgcaacac acacacacac acacacacac acacacacag agtctagaaa 13500

ggaccaccaa acaccaaagt gttaatagct acttccgaaa gcagagatag aaactaggct 13560

aatgggtgag gaatgtttac cttttaattt tcactatata tactttccat attgttagaa 13620

ctttttacaa cgatgttgtc acttcatgat ttaactgttt tgttttgttt tgttttttaa 13680

tag 13683

<210> 23
<211> 82
<212> DNA
<213> Homo sapiens

<400> 23
gtcccaaccc tggccatggc tgacaagggg cccagggaca ctggagcaga gagacctgct 60

ggtccttggg gtttcttgca ag 82

<210> 24
<211> 142
<212> DNA
<213> Homo sapiens

<400> 24

gctgatagcg ggggaacctc tctcacaggg ctgctacaaa gattgcatga gattttgctc 60

aacaaatatt agctgttggc tccagagttt aatacctctg atcactacaa gctttgaagg 120

caagataaaa taacttcaac at 142

<210> 25
<211> 133
<212> DNA
<213> Homo sapiens

<400> 25
agtgaatcat ctgtgaacac ttcctgtgaa cgtgtgacta tgattatgga acactctgtt 60

gcatcaagat ggccagagat tgtctcagag tccaggcagt gggaggaggg gggatttgtt 120

tcctggctgg gtg 133

<210> 26
<211> 329
<212> DNA
<213> Homo sapiens

<400> 26
gtaggactct acatcctccc ttgggaacta attgatctgt cagtaaaatg taaacttctg 60

actccatggg tctgtgaatc agctgcctct aaagggaaat atcccaggag tgatgaaaac 120

ttgaagatcg aggattggat ataagctcag aggtgaagga gggaaattcc tcgggtcaac 180

tttagcggac tgatccacgg ggtctgccaa gagacaggag ctcgagggtt aaacccaagc 240

actcgggagt cagatgtatg aatctgaatc tcagcctctc ggcttgctgt gtaatctcag 300

gcaagtgact tttctgttct gtaaatcag 329

<210> 27
<211> 13683
<212> DNA
<213> Homo sapiens

<400> 27
gtgagtacca ggaggccttg gtgaaaagca agcctgccgg gagataatgc ctggctgggt 60

caggattctt ggtgcttatt gaccactggg cacttcagag cagaactcat caaagcctct 120

gtattgggac aagggctagg gtggagggga ggattgggaa gggagagggg agactgtcct 180

tctttctact aaaagttact ggcaagagaa tgggaattaa ccaggcctgg aaaagtaatt 240

taggtattag aactttgaac tcttttttta aaaacaaaag acaaaaacag ctttattggg 300

atataattga caaataaaaa ttgtgtatat ttaaggtgta caacttgatg ttttgatata 360

tgtacacatt atgacatgat cactaccatc aagctaattc acatatccat cacctcatac 420

agtcactatt ttcttctttg tgtgtgtgag agatgagaac acttaagatc tatcctcagc 480

cggatgtggt ggctcacgtc tataatccca gcactttggg aggctgaggc aggcgaatca 540

cgaggtcaag agattgagac catcctggcc aacatggtga aaccccatct ctactaaaaa 600

tacaaaaatt agcttggcgt ggtggcgcgt gcctgtagtc ctagctactc ggaaggctga 660

gccaggagaa tcgcttgaac cggggaggcg gaggttgcag tgagctgaga tcgcaccact 720

gcactccagc ctgatgacag agtgagactc catctcaaaa aaaaaaacta tcctcttagt 780

aaatttccag tatataatac agaattgtta actatagtcc cacgctatac attagatctc 840

cacaacttat tcatattgca taactgaaac tttgtaccct ttgaccaaca tgtccccatt 900

ttcccgcccc tgagctgctg gcaacagcaa ttctactctc tgcttctatg agttcaactt 960

ttttagattt cacatataag tgacattata cagtatttgt ttttctgtat ctggcttatt 1020

tcacttaaca taatgtcctc caggttcatc catgttgccg aaaatgtcgg gattttttaa 1080

ggctgaataa tattgcattg tacgtatata ccacattttt aaaatccatt tatccattga 1140

ggtacatttg aatggtctac cctcctaacc aaggccaacc ctttagccca actaagctct 1200

tggaaaatgc ccaaagttgc tgcaaagccc ctggctaaat ccttctcatc ctgagactcc 1260

gcttcagcgt tacctcccca gggaggcctg ggctgggttt gatccctaag tcaacccata 1320

cggcactttg cattgttaaa agaaatactt cagccaaatt aaatttaaag gagtttaatt 1380

gagcaatgaa cagtcaggca gcccccaaat cacagcagat tcagagatac tccagcgcag 1440

ccacatggtg gaaggaaatt tatggacaga aagaggaaag tgacgtacag aaaatagaac 1500

attttcatat tgttcagtga gtgtcagggg ccccaaagaa tcatgattcc acagcataat 1560

cacagctaac atgtatttgg tgctctctat gtaccaggaa tggcataagt gttgtctagt 1620

gaattcactc aacaactctg tgaaatagtt tcccttctca cttggttttt agttaaaaaa 1680

aaaaaaattt gtagagatgg ggtctcacta tgttgcccag cctggtctca aattcctggg 1740

ctcaagtgat cctcctgcct tggactccca aagtgttggg atgacagatg tgagccacca 1800

tgcccagacc ccttcccact tttgcatctc taagctgcca ccttgctgac ccacacacag 1860

ctgtgggtga aatccaatgg gctcagcagc catgagctaa tagcactgct cctcctgcgt 1920

agagaaagat ttattgtttc gaacctttga cttagcttag aaaacaaaat cagtaggccc 1980

cactcttcaa tgtttttaag gtatggtctc ccaggtggat tctgagacag ctttcatctc 2040

tagggttaat tagggtctgg ccattgctca tgcgaagata gaagactaaa acacgcaaat 2100

ctaatgcccc atctttggct tccttgggaa cagggggagg ctggggtggg atggaatgga 2160

aaacacagag aagcaaatag cccacccaag aaagtgtgag ccctcctgcc ttcactacct 2220

ctggccacca aaagagacca aacaaagggg ctaggggaag gaaagaattg ggtgggggta 2280

aaagtggggg tcttagtaaa ctctaaactc ttagaaataa atgtagagtt cacattccac 2340

ctgggcttcc aatgtccttc tcagaggcag tcagggacca ccaaccaggg agcgcgtatg 2400

agggctcagc ttggttctag aacctttgta aactcatgct tgctattttg atgatcactt 2460

acctgatttt ccagaggaaa actaacccag ggaagcaggg aggagaggcc catgagcctg 2520

tgttggaaga agaggaggag aggtcagcac tccaccttca gccatgtcct agccagagag 2580

gggagtgaca gctttctcaa agagaggaaa ctgggcagat ccctgaggac ttgatcaagg 2640

atgcaaggca ggggagcaca ggggggctat gggctgagag gggcctggag ggtggcagtg 2700

gctgggccta gaggtgggct ccgtaatgac acagggctgg ggcaatggaa gcgcctagct 2760

caggagcatg gtggatgtga gtgtatatcc gtccacccct agctagcaat tcactagctg 2820

tgtgaccttc ctctatctcc actgattgtt tccaagcctg agtttcctca cctgtgaaat 2880

ggagataatc cagcaattgt gacgagaaat gtgagaaaat gcatgtctgc agctattaca 2940

gggtctgacc tggagtaaaa gctccatgaa tgccggctgt cctgtggcct ccccgacacg 3000

tcccccctgc tggaaggctt tcctcaccat ctccctcata aacaaagcca aggtcaaggc 3060

tgcagcccat ggtgagagca gggagagctt gtgggaagtt cactgccccg ccatgcccct 3120

gaatggtgag cctctttctt tgatgtgggc taacatccgt ggactcagag gcccagaaag 3180

tgcgagacac gccaaggctc ggcccctggg cgtctgtttc ctgagcagcc tgggacacag 3240

gttgccctgg tgagatgacc cacagtcagc accctaggcc ttggcattat gcgggcagct 3300

ggtatgagcc ctgcctcacc ctgcacagaa tttgccagct tcacagcatt cctgccagcc 3360

cagcctgctc ccagacccag ccaagtttca ctcaggcagc ctgcattccc attttgtgcg 3420

ccttgtcctg cccagaggca ccggctgctg agggaagcat gaggtccttg gaaaggagct 3480

gccccagcag gccggtgtgc tgggagccct cagcctcttc tacactcccg ccacccccag 3540

ccctgcatcc tgccccaagc tggctgcctg ccagcttctc gggtctcatt tacttactgc 3600

tgtttgtttt ggaggcttct gggaagcaag gctccacctg aggaatcttt ggctgagggg 3660

acttgtgaca cctgagactt tgaactttca agaaaacata aggctccgct ggccacttcc 3720

ccgggtgtgg ggacaaatgt cactgcagtg ccgtgggctt gcaccaggcc cctcctcctc 3780

tccaaagcag ctaaggcaga gctcatggac agggacctgc ctgaagcagc cgccatctgg 3840

gggttgacgg ctgcctggtc tcttgctcta tctggaaaaa gaaaccataa gcatttcata 3900

tttatcttat aattcatttg gatttgtttt catataaaag taatgtctaa gggaggatta 3960

gtcaagtaaa ttgtgcacat gttcttgtga tgacctatta gcagatgcca cagatttacc 4020

ctagtttgag aaacaaaggt gagaagtatc ttttgtagac caggtgcggt ggctcacacc 4080

tgtaatccta gcactttgga aggccgaggt gggtggatta cctgaggttg ggagttcaag 4140

accagcctgg ccaacatggt gaaactccgt ctttactaaa caaaaaaaaa aaaaaaaaaa 4200

ttaactggac gtggaggcac tcgcctgtag tcccagttac tcgggaggct gaggcagggg 4260

aattgcttga acccaggagg cagaggttac agtgagctga gattgtgcca ctgcactcca 4320

gcgagattct gactcaaaaa aaaggagtat cttttggaaa acctctgttc aataattgac 4380

ttttaaatgc acaaaaaatt ggaaatacac caatttttac tttttttttt tttttttttt 4440

ttttagagac aaggtcttgt tctgttgccc aggctggagt gcagtgacaa gatcatagtt 4500

cactttggcc tcaaacttct ggcctcaagc aatctttcca cctcagcctt ccaaacccct 4560

aagattacag gcatgagcta cccagcccag acctcaattt ttaaaaattg taaaatgaac 4620

ttttaaaatc cctattaagg ctgggcatgg tggctgactc acgtctgtaa tcccagcact 4680

ttggaaggct gagacaggta gacaaggggt ttgagactag catgggcaac atggtaaaac 4740

cccatctcta caaaaaataa aaacaaaaca aaacaaaaac acaacaatta gcccagcata 4800

gtggcatgtg cctgtagtcc cagctacttg agaggctgag gtgggaggtt tgattaaacc 4860

tgggaggtcg aggctgcagt gagccaagat catgccactg cactccagcc tgggcaatga 4920

ggcaagaccc tgtctcaaag aaaaaaaaat ccctattaaa tttggtagaa ttgtaattta 4980

agcaggttta ttatagtgag tttggaaaga ataaagaaaa agaactaagt tactccctca 5040

cacagagata acccttttgg tgtacatttt ggtatattta agatatcata caatatatac 5100

ttctttggtc atagttagca ctcttatcta agtatttctt cccaatcaat caattaatgt 5160

tcagcaagat tttttatttt tatttttttg agatagagta tcgttctgtc tcccattctg 5220

gagtgcagtg gcgcagtctc ggctcactgc aacctcacct cctaggctga agcgattctc 5280

atgcctcagc ctactgagta gctgggacta caggcatgtg ccaccatacc cagctaattt 5340

ttgtattttt agtagagatg gggtttcacc catgttgccc aggctgctct caaactcctg 5400

gcctcaaatg attcatctgc ctcggcctcc caaagtgctg gaattatagg catgagccac 5460

cgtgcccggc tggtaagatt attttttatt tttagtggca gcatagtcca ctgtgtggac 5520

gaacaaaagt tttgtttgtt ttttgagaca gaatctcact ctgtcaccca ggctggtgtg 5580

cactggcatg atctcagctc atgcaacctc tgtgattctc ctgcctcagc ctcctcagta 5640

gctgggatta caggtgccca ccaccatgcc cagctaattt ttgtattttc agtggagacg 5700

gggtttcacc aggttggcca ggctggtctt gaactcctga cctcaagcga tctgtccgcc 5760

tcagcctccc aaagtgctgg gatcacaggc gtgagccact gtgcacagcc taaaaatttt 5820

ttaaccgttc attttttgac atttaagttg ttcctatttt ttgctgttat aaaaaacatt 5880

caatagccac ttttgcacaa acatctttgc acatttatct gagcacatac ttaggataaa 5940

cttcataaaa ttggaattgc tgggtcaaag gataaggaca ttttaaggtt gttggtacac 6000

cctgccttgg ctggtttctt cctttgcttc tatgaaaacc attgccacct tgagatcagg 6060

gacgtatttg cctaaatttt cagtagtgct atgattttgt cattagatgt ttagattata 6120

attcatctgg actaatcata acactaataa taatatgagg taatacttat tgggtgggga 6180

ctatgagttg gacattgggc taagaaattc ttatgcataa tcttacttca cccctactat 6240

tatgttcagg gaactatttt tatttcatct tacataggaa gaaactaaga ttgtgagttt 6300

aacttgggat gaggcaggga tctagcttta tttttatttt cctttctttc tttcttcctt 6360

cctttcttcc ttaccttctt tccttctttt gtttccttct ttctttcttt ctttctttct 6420

tcctttcttc cttcctttct ttttctttct ttctttcttt ctctctctct gtttctttct 6480

ttcctttctt ttctttttta aagacagggt ctcactctgt cacctaggct ggagtgcagt 6540

ggcgagatct cagctcactg cagtcttgac ctccctgggc tcaagtgatc ctcagcctcc 6600

caagtacctg caaccacagg cacacgcccc ccatgcccgg ctagcttttc ttgtgtgtgt 6660

gtatgtgggt ttgtgtgtgt ttgtgtgcgt gtattttgta gagatgggat tttgccatgt 6720

tgcccaggct agtagctttg tttttccata ttattccaac atcatttatt aaacaatgaa 6780

tctttccatc actgatttga atatcccatt cagcatacac taccaatttc tttttataca 6840

taggatctgt tcctgggcct tctcttcttt tccactgagc tgcctgtcct ttttccattt 6900

cacacggttt tttgtttttt gtttaccact gaagctttaa aatgtatttt actgtctggg 6960

aatgcagaac caccctgttc tttttctccc agaaatttta tgattctatt catacataaa 7020

tttatttcca ggtgaattca ttttgtcaca ttaaaagtaa atcttagata tttttattag 7080

aattgcataa gacttatgca tttgggcaaa tggcatttga cagtgttgag ttctcggtgt 7140

ggtgtgtttt gccttttatt taatgtccct cagtgtcatt cagcagattt tttgttatga 7200

gttttgcaca gttcctgtat acttcattcc ttggctttgc tattgtaagt gggaactttt 7260

tgcattatat tttgtggcca gttcacccta gtgtatagaa agctatgaaa tgggtactag 7320

tgtttctcgt ttacagatga aggagtagaa attaagggca cagtgccagg tgagactcta 7380

gttgaggaca gggctattca gaggtgcggc cgtgtctgaa cctgcctcag tcataaccac 7440

tctcttagaa ggaagctgcc cgtgctctcc aaagacagtg tctggtcaga cccagcttgg 7500

tgtctgctgc ctggcttggt gctcctggct tggtgtctgc tgcctggaca gttccagatt 7560

caagggcagc caaacatttc tgggcagcaa gctccctggc ctgatcaagc tctgggccat 7620

ggctgtgccc cgcaaacccc agaacccaat aaaaggagtc cccattaggt gggcatggct 7680

gcaggtgact gttgcacagt aggactttgg accacttatt tgaaagctgc tgttcttact 7740

gttcagcatc agagagaatt tgatgaactc tatggactct cagacaaatg ccccaaatgc 7800

tgttgacact ttcagaggat gtatggacaa ccccaaaaca cacccaagga tcttgggtta 7860

aattaatatt cctgtgttaa aacaatggcc cagacaggga ttcctccagg tgggaggtgc 7920

ccagggatgg ggtcacgttc agaggacaga agtccttgtg tctgtgtgac tttggatggg 7980

tcacttccct ctctgaatcg ttattttctc catgagaaca atatctaccc agtctagtta 8040

tggtgttgtt tgggaatcgt ctggtatcct gtcttgtgac agtgctttga aaactgcagt 8100

taagtaacac aaaggtaaga aatgactcat gccaggaaag ggaatagcat gaggcccagc 8160

tctgatatgc aaaggcctct cagattctta gagaatatta ttggctctca caccaccagc 8220

tgctcccagt gaacttggcc ttcgccccca ggaatggctg ataaaggcct tcactgcttt 8280

ccacttctga gctgatggga ctgttttagc tgacccagct aaaaccatga ttaagtggct 8340

ggatttggaa aagaagtgtt ggggctcatg gttcacagat acgtttctgg gttgttataa 8400

aaattaataa ttaggctggg tgcggtggct cactcctgta atcccagcac tttgggaggc 8460

cgaagcagga ggaccacttg aggtcaggag ctcaagacta gcctggccaa catggcgaaa 8520

ccctgcctct actaataata caaaaattag ccggatgtgg tggctcatgc ctgtaatctc 8580

agctactcag gaggctgaga caggagaatc gcttgaacct gggaggtgga ggttgcagtg 8640

agctgagatt gtgccactgc actccagtct gggtgacaga gtgaaaatat gtctcaaaaa 8700

acaaaattaa taattaatcc tctcctgcat gctggagggg aactgccagc agcctccgag 8760

ttatggagag cactcctggc caccaggctc cccgtgagtc ctggctccgt gtgagcccag 8820

gccagccctc ctctctactg ccctccactg ggtccccatg gtaaaataag gggctgcact 8880

tgatgaaagt gaatttgcaa ctgaaaaaat atgtaataat aaagccagat cccttctaag 8940

agactttaaa ggtcttccaa cttgtcttct tcctgctccc agtagattat gtcccctgtc 9000

ctcatgcccc agcgtgaact ttaggccctg gacacatcag cttgcacaca catgtgtgtt 9060

tgtgtggggg tgggtgggtg tgcatgtgtg tccttggggg aagctggggg aaaggggcaa 9120

aagagaaccc tcactgggaa cctttttcta aatacaaatg gctgccccct tctccctggg 9180

aaatatctga atggggtgag ggatatactg ctttggtctt ccgaccatcc agatccccat 9240

ctccagactc tccagccaga gggcagggca gtagggggac tggcaggact attttgaacg 9300

cctggttctc agaaccacct ccctgcagtg ctcctgttca gaggtcagag gtgtgcagcg 9360

tcctttcatc ctctccttgg agagcaagcc tgatgaggca gggaatatct ttctgactcc 9420

ccggttcctg gctccatggc aggcatgcaa cagaagctct cggacaaata aatgggacct 9480

tcccccatac tttgcctaaa ccttcttatg tcccttctct gctccctctt taccaatgtg 9540

ggtatctcct ctcctcccaa cttctgctca aattgtagct tctggggatg cctccagctg 9600

aacccccagg cctccccaga tctgtggccc cagccctgtg tccctgcatc tttgctgatc 9660

ccagccagaa gctgccaggt atgcattttc atcaacagta gtgaggacta tttattaatt 9720

tatttatttt tgagacagag tcttgctctg tcactcaggc ttgagtgcaa tggcctgaac 9780

tcggctcact gcaacctctg cctcccaggc tcaagagatt cttgtgcctc agcctcccga 9840

gtagctggga ttacaggcgt gtgccaccac gcccggctaa tttttgtgtt tttagtaaag 9900

acagggtttc accatgttgg ccaggctggt cttgaattcc agacctcaag tgattcatcc 9960

tccttggcct cccaaagtgc tgggattaca agtgtgagtc accatgccca gccagtaagg 10020

actattcgtt aagcagcatt atagatcacc tgtgtatgtc tgctaaaaaa aaaaaaaaaa 10080

aaaaaagggc accctctcta cccagatgag caaaggtatt ttttctgata cctgtaacaa 10140

atctaatatt gccacaattg ctacatgatt agtgagagaa gcatttgata ggctaatcaa 10200

ttgattggtc tggggtaaat attcagtgaa tccaaagttc aactctaatg agccacttca 10260

cttctcctgg gccctcttgg taatgtggta ccaggaactc tggaagtcct catggcattg 10320

ctcaagggag tggtgggatg ggtccccagc tgctctttgc acagctctct gctctaggtg 10380

actgtctggt ccatgagctg ggctggccca gtctgtctct ggcaatcctg gggacatctg 10440

ctgttgacat ccttggtgag tgtaacctcc agcttacaca acccattgga cacctcctct 10500

gctctcaaaa actctgctga cccccaaagt atggttgtga ctcccattaa gcccaagacc 10560

cacgtagacc ctatgccccc agccagatcc tcattggaaa cccccatggg aacatacaag 10620

aaagtggatc tcttccagtt cctcccacat tacgtggcat caagggcttt gggaatgtca 10680

aaccaaatac tcccctgtcc tcagcagagc accctcccct ttcccaacgc ccccccgccg 10740

ccaccaccag gctaacgaag ttctaaatgg gggtcagggc acaaaatact ctgtgtttgc 10800

cttttctctc aatctattca actgctcacc ccacccaact cccaccaatc cagtcctcta 10860

ccctgggact ttagcctgca gtaggaagat cagcccacga gtgtgaccac ccaggtcccc 10920

aacctcagcc catcttcttc ccagtttcct gggcctggag gggagctcta atccctttca 10980

gttttagcct tgatatcctt aacatctttg ccactagaca gtgggccacg gacgagctgc 11040

atcagtatcc ccagagctta ttagaaatgt agactctcaa gccccaccat ggacttgcgg 11100

aaccagggct tgtatgttaa caaggtgccc gggtgattca catgcacatt aaagtcagag 11160

aagcggtgct ggcttgcagc acatcctggc agcgtgttct ccttagctta tgtcagactc 11220

ctggatctgg tccgccaggc ctggttctta atatgttagt ggaactcagc aaatttagga 11280

aacccttctc tctcttgtca aagtttgact tctagatgct gcagactcaa ctatcttgcc 11340

tccatgcctt ggatgtcact ggctgcataa ggactttgtc tttctcttga cattcttgct 11400

gtaacaatcc cagtcctcct cagtccattc ttcttgaaaa tggcactggg gaaagaggca 11460

aatatacaga atggagaaag agagaaacac atgaacatac ttgaaaacta ctctcccagc 11520

tacaatagct tttgctgact tgagcttcaa gtgatgccag caggctacat ggagggtaga 11580

ggtgagaagg ggtggcacac acttcacaga tgtcacagag gaggacgctc cttctaccac 11640

actgtccctt gtctaaggac gcaaaggaga aaagccttgc cttagcatcc agagtggttc 11700

aatattacct gagcaagtga actgccttta ttttacaggc ctctagggca taactatcca 11760

tttgcccttt tttgttttgg ccactaggaa gcttagagac agatttgtgg tccaacaacg 11820

gtaagtgtat gatcatgtct aattctcatg caggactgat ccctggcttc accctagctt 11880

gtcttcttcc tccttgcttt taacatatgc attttattat aaccatgtat tcgtataagc 11940

cactttaaat ctctcctgaa gccaagtgaa ataactttgt aagtaggtac tgttctaggt 12000

ccttgcctat ggcgtattcc ctcttgatct ttgccattgt taacatcccc attgcacaga 12060

tgaggacact gaagcgcaga gaggtgaaac aagcaagcca ggctcagggc aacggtgagt 12120

gtccaagcca gggtatctca gctctctgtg ttcagagttt gggccccaga agggaccacc 12180

tggatcctgt cttggcagca ggcaggccaa cacggcgctg agagggagct gaggggaggt 12240

gtgggtcaga tttctggcaa gccctgaggc tgtcagtgaa ccgctgtggg gtccccccag 12300

catgtgcttt ggggccacac cctaatgggg gtcttcttta tgttatgacc actcctgcag 12360

ttaatcaacc acaaggttgt tttttgttgt tgctgttttt tctgtttgtt tttttttttt 12420

tttgagacag agtttcagtc tgttggtcaa gctggagtgc agtggcacaa tcttggctca 12480

ctgcaacctc catctcccag gttcaagtga ctctcctgcc tcagcctccc gagtagctgg 12540

gattacaggc atgcaccacc acgcctggct aatttatgta tttttagtag acacggcatt 12600

tcaccatgtt ggccaggctg gtcttgaact cctggcctca agtgatccac ccgcctcggc 12660

ctcccaaagt gctgggatta caggcatgag ccaccgagcc tggccttaag cacgagattc 12720

ttaaggatag cactgggacc ggtgattctg tgactggagc tgattaactg ctttgtggga 12780

ataagtaagg gcttcaaaga tgtgtgccca tctcggctgc caggccctga gttcttgagg 12840

tggaagctga ctctccacct ttctggctga tctaggcagg ctagttccct gaagactcaa 12900

ggtaagctgc cttctagaat gaacttggga tgaaggtaaa tgatgacaca gggcagctgt 12960

ctgaaaaaga aaggtttatt tatctaaacc aacaagaaag gattcttttt aattggaaaa 13020

acaattaaag aatagtgcat gtattcagat cctatttttc ccagtgtgtt tttgttgttg 13080

ttgttgttgt ttttgagatg aggtctcact ctgtcgccag gctggaatgc agtggcgcaa 13140

tcatagctca ctgcaggctc cacctcccag gctcaggcaa tccttccact tcagcctcct 13200

gagtagctgg caccacaggc acacaccacc acgcccagct aattttttgc atttttgggt 13260

ttcaccagtt gcctaagctg gtctcgaact tctgagctca agtgatctgc ccacctcagc 13320

ctcccatagt gctgggatta caggtgtgag ccattacacc cagcctacgt gtgttttaaa 13380

tacacatact cagatggatc cactcagaca cacacacaga cccccataca cagagacact 13440

ctatccccat cccgcaacac acacacacac acacacacac acacacacag agtctagaaa 13500

ggaccaccaa acaccaaagt gttaatagct acttccgaaa gcagagatag aaactaggct 13560

aatgggtgag gaatgtttac cttttaattt tcactatata tactttccat attgttagaa 13620

ctttttacaa cgatgttgtc acttcatgat ttaactgttt tgttttgttt tgttttttaa 13680

tag 13683

<210>  28
<211>  470
<212>  DNA
<213>  Homo sapiens

<400>  28
gcgagccacg ggcctcgctg cgggttcaag tgcgtccggg tttggacgtc gcggctccag      60

gagtgtgctc tctctcctgc ccatcccctc cccggagttc agcgcatccg gggccccggg     120

gacccctctt cccggccctc ctggccgcgc tggcccgccc gcatggggcc gaggttcctg     180

gccgacgcgc gagggcgcgg gcgggtaccg ggttcccgtt tttcccaggc cccgatcccc     240

gcccacgcac gaggcccccg gcccacgcac gaggcgccca cgcccatcgt ggaggctccg     300

cctggaaaag aagtccgact tcctctccaa gctgctccga ggggaatggg gaatcggcag     360

gaaatgaccc gaactgccag cctgcgcctt tgcagccggc cctcgctttg ctgaagacga     420

gcagctccca ccaaagtctt gcctcccctt accccgaaag ccccgctcag                470

<210>  29
<211>  93
<212>  DNA
<213>  Homo sapiens

<400>  29
ttcaatattt caagtgaaaa tctgcctgtt cgaaaagaaa atattcacaa atgagcagtg      60

tattagaaga caactgaatt gcttctaaga atg                                   93

<210>  30
<211>  844
<212>  DNA
<213>  Homo sapiens

<400>  30
ggtctcactc tgccatcacc caggctggag tgtggtggca ccatcatggc tcactgcagc      60

cttgaactcc tgagctcaag tcatcctccc acctcagcct tccgagtagc tggaactgca     120

ggtgtgagcc accatgcctg gctgatttgt ctttttaaat tttttataga gaccgagtct     180

caactatatt gtccaggctg gaaaagaact tctttccctc caagtgataa ccttttccag     240

acaagtcagc ttaaaaactg acccaaaggc cctgaagtac acagttttct tggagaaaga     300

aaaaaataag tgaaaaggac ttgctgactt gctaattcat tttggggcct gtgggtaaca     360

tcagaatggc aaagcacttg gggctttttt ctaatttgta tttttttaat cttctgagaa     420

atgactttta tatgccaaga ttcattccat gattagtttt tatgaccaag ttatgaacgc     480

ttttgaatat ggggacctag aatagaaatg ctaacctacc tgcaaggaga gctctgtgat     540

tggcatcccc atcatgctgt aactgacaca aaataaactt agggtaattg ccgcatagtg     600

taactttaat gtgtgagcat actgtaacat ctgacaagct tcttgatcta gatcagcaat     660

gtgtaaccca ttcattgttg tcactctcaa caaggcctta tggtcaaaca tcatcactct     720

cactaaagag atagtttact gaaatatact gagattttga aactggagtc aagaggtagc 780

acactgtttt atgttgatcc atttaatggt ccttgacagc caataaaaag acactggaag 840

aata 844

<210> 31
<211> 4336
<212> DNA
<213> Homo sapiens

<400> 31
gtttgtaaag aggggtgcga gatctgaaaa gtatttgtct tctagggaga gagatgttgg 60

taaggggccc agagaaagac gggtcctttt tagctcatca acgcgccacg tcttgctttg 120

cagctccaaa atgatgccag cgctgctgtc cgaagggtca atattaatgc gttaagtgaa 180

atgggaaata aatggacagt ggcacatttc gtggcatcat ttttacaagg ggatgtcgaa 240

agttttaagc tgcatttaag aattagccac atcttgcctg aaagttaata ttttttgata 300

aactcgttaa taaagcctag aagaggtaag ttaaagttag ttaccacgta gatcctccag 360

tctcttgtag agacttgcgc aggttactag gatgaggacg agttgtattt ttaattttct 420

agactaagtg caaaggcatt gagttttgag gtggcagggt taatgttcaa gggcaaatgg 480

aaccgccaag gatctcttga aaagaattca cttactatta atacttaagc ctgaaataat 540

tataattaat ttacagtggg aattatatgc ccatgtttta gacttttttg ttatattagc 600

tatatgaaaa gggatgttct ttagtgatta aaagaaaaaa ccacctgttt attgtcattt 660

gcaatttgaa atagccgaca gcagtaaggc tagctacaaa agctaacaag tcaacttcag 720

tgtggtttga catttactgt caaagctttg agctctaaga aaaacagaat ttaaaataat 780

gtatataaat aataaaattt cattgtgact caatgtccct gcccctggag aagggacaga 840

tgacactcag tgactggctt ggaaccattc cccctgcaga tgcatttgcg gatacagtgg 900

gccagggctg cctctgagta tgtacagcaa gtgcttatgg acttacagaa acaaatgttt 960

tactatttag gatttctcaa atagtttaca ctaaaagttt tgcatgtaga aatgtgggat 1020

aaatatttct ttgaaaggaa aagtttcgaa aggaaacttt atacttgtat ttttttgtgt 1080

gttcacattt ctaagttcat ctgtctttca aaaaacagat tttcataaat attaaaatat 1140

tgcaagtatt atttttaatt cttttgagtg ccatgtttgc tgtaagtagt tttttctttt 1200

aagtaagatt aatataatcc tgtctaagga gtttaataga gagaacagga tttccaagcc 1260

tataaatgca gtctatataa aatagtggga tcaaagtttt ttgttaagaa acttgagaat 1320

ttatacattt taaatgaaga tagaagcacg ttttaaaagt agtttttcca caccatttcc 1380

ctccccccaa aagaattcag aaactatatt ccatagtcta aaaatggcat aatcagatgt 1440

aaaaattatt accataatat atagttttct caagatggtg gatacttttt aatatattat 1500

gaagagttat attctcagcc cactgtgtgt ttgagggaat ggaaaagaca agatgtttct 1560
aaacatattt tactactgaa atccataaga ctattaagct gtgcaggtaa ttgtgttcta 1620
agtggtagaa gtatttttcc aactgctttt taaactttga aaaatttata tgtattttaa 1680
ttagcacttt tcttttaaag gttccaaata tttagctata tcagctatgt gataaaaatt 1740
attggaattt gttccctact actttctgca taaacaattg agaaatattt tatactaatc 1800
tttttatata caggggataa atttgaacat gtgtcttgtt ggtgtaataa acaatccaaa 1860
ttcatttctt tcttgaaaac ttggcatcga tcttcatctt attttttatg cacacaattt 1920
ggacatgcca gtttgtaggc aagttttaag acagtgcaag ttttatgtgg aagaatgtat 1980
attaaggaat gaatagctaa tagccaaaat taaatcagga ggtgtggcat tcactccttt 2040
ggatgattat tttaaagata gttttgcact caggaaggat tttcttatgc tctgtattta 2100
tgaatatcta aattatcctg ccttgcaact tcaaaactca ataaccaaat accaaattta 2160
tgtattttca gtgtgactaa ggctaggaat atgagaagca gattggtttt ataaaagaaa 2220
gcaagcattt acaattgtat tttaaaatat aatcctaatt tcagtttctg acagaaatct 2280
tgtggtcagc tcatcagttt ttcagatcag tttctaaata gcatataaaa actacctttt 2340
ccacatcaga agttcatatt taggtttcct taactgcaaa atcactttaa aagagagtgt 2400
tttatgtgaa gggccatttc atgtgttggt tccatcgatt acatatgaat gtataaaaat 2460
cttgtttaaa gaccagtaaa agtgaggaaa ttccaagtca ggctggctaa ttccattcat 2520
aattcatccc gagtacctga ctgaggaaac ttgcaaacag tttatagcta tacatacaaa 2580
ataagttgac gtgctttgta agctggaagc agagttaagt ttgacctccc tgcttggaag 2640
gtcttaaatt aataaaatta tggaatatgg ttaagctttt cagtttccca agaattttcc 2700
aatatcgatg ctttttggat ttttgcattt tgaaaatgta agcatggaag gacaacttct 2760
tttgtgctga gatgaagtta gcttttaccc atccatttcc ttcccactta acacatttgt 2820
tcagtaattc ttcgagtgct gggggacaga ggccctgcct tgagaaaagc atatgccatt 2880
tctctgcccc cttcaaccat ccccaccccc agccctccct ttatttcaag ccagttggac 2940
aagtatttag cattgacaca tgtttcaaac tgctgcatcc acagccctag gtagttttgg 3000
ggattggcag gaaatccatc tagactttgc cttctgagct gggtaaaact actaggaagt 3060
cctatgcctt gatattgtgg cacttctagg ctgtctttcc aattttaagc aatccatcag 3120
ttgttttcta agactgcact gtagactgat gccttctctt agttttgatg ttcagaagat 3180
tttttaaaca tgttcataga tttcttaaac attctgagta attgtaattt taaactagac 3240
taattttagg gttgaaaact gtcccagaga gagtccttac ataataactt caatctgatg 3300
tgaactcgaa tgacttttta aaaataagat gttgaaaatg tgggtttctg gtggaaaagg 3360

ctgccattca cttagagtgg gatccttagg accagttaat atccaccaga catgcttcaa 3420

taaactggaa caagcctgat gtggaattac tgactcccca ttttcctgaa tgtaggtcat 3480

tttgtgtcct tacaatttaa cgtttgaagg gatttcctgt ctttgaagtg caacagtttg 3540

tcacctgctt ggctagctgc cttaacagtt ggggctcctt tcctacattg aaagacttag 3600

tgactccatg ttcccgggct agagtttccc agctcactga atgcatgata cctgcacctc 3660

tgggactctc ctctacaagt tagtgcctgc agatcccctg ttccaagctt catttctcta 3720

ggtcaaagag cctaaaagaa aagcccgggc cgggcgcggt ggctcacgcc tgtaatccca 3780

gcactttggg aggccgaggc gggcggatca cgaggtcagg agatcgagac catcccggct 3840

aaaacggtga aaccccgtct ctactaaaaa tacaaaaaat tagccgggcg tagtggcggg 3900

cgcctgtagt cccagctcct tgggaggctg aggcaggaga atggcgtgaa cccgggaggc 3960

ggagcttgca gtgagccgag atcccgccac tgcactccag cctgggcgac agagcgagac 4020

tccgtctcaa aaaaaaaaaa aaaaaaagaa aagaaaagcc cggagccata cactcacctg 4080

gttgaagcca taaagaggta agctttcata acgtaggcta tatatcaaaa ttcatagctt 4140

tttagaaccg gagtaactag tgctcaggca ttagagtctc aggctagagc aaaacacagc 4200

aactggtgcc aaatgtacgc tagtggctga aacccattta agtaataaac aaacaaatgt 4260

ccttttaaga atgtgctatt atttttattt aagaatgcat atgactttat cgccccttat 4320

gatgtgaatc ttacag 4336

<210> 32
<211> 2600
<212> DNA
<213> Homo sapiens

<400> 32
   gtgggtatgc taatttttac caaatgatac aacatgtgca tgccaaagct ctttgtaaac 60
   tataacacac ctccaaatga aagcaatttg catatatttt aataaacttg tctgccttta 120
   ccacatccac caactaatcc ttatgctccc actgcaaaga ctctcctctt tttggagagg 180
   aacaccaatt gtttgaaagc ctggactttc aaacaatctt aaggcatctt catagtatcc 240
   gtccttaaga gtgctttcat cttaatatta ggtaaacatt tagaagatta tgaactttgg 300
   ggactttctt ttcagagaac tgaacacgtg tatataaaat tacaatatcc tgataagtga 360
   ggggaagaaa ataaaaatag gtgtcaatac agttatggct taagatcttg atgaaattga 420
   gaagaaagta gaaatcaaat gcatcaaagg aagaacggtc agactgaatt ctgtttgtaa 480
   ttttttcaaa atgaactgaa atgcagtttt tctgactata tgttttcttt tatggacatt 540
   aatcactttg attcatttaa tttctctgga agaaactgta gcttttgtaa cagaatctgc 600
   tctattgggt tctgtatggg cacctaccgg tttgctttct agccagtatc atgtgaattg 660
   tctagagaaa atgcctttag caaacacctt caaaaagcat ccattccaag ggcacacctc 720
   ttgtgtctct cacaaagcaa gtggtactat gtctagccaa ggaggcagac tcagattcat 780
   gcatttatct caggtttttt ggtgaccttc ttcctagatc ctgctcttgg caactgtaag 840
   ccaagtgtac ataagtatta gaatggattc tctaggtacc aagtgaaaat tctatctcta 900
   gtcacatgct cctgcacctt tcatgtggga aaaatgttcc agtctttcct tgattacatc 960
   acagatgata cgagaacagg acatgggaca gggaactgct aaacagctgg atatctctgg 1020
   aaactcagag cacttaaaaa cactttgaag ccatgcagct gctctttttt tttttttaaa 1080
   ttgtcttcag tgaaactcca aaaccccaat aatatctacc ttaaaagcat caggagtatt 1140
   ttaattgggc aataaccatc catattttcc ttctgataaa ttaaaatgaa ttaagaatga 1200
   aattttatga tgattataaa gtatattact gacccaaggg acaagaacaa ttatgaaaaa 1260
   tatgtcctac atatttggca tgaatatgaa tttagacttt aaaccattca tcttaaatgc 1320
   tcaagcatct tattaaagct tataaaataa tattcacata ataacagagt aacttataat 1380
   catgattgat attatatcat caatattgca gcaggctttg tgccaaatac tgaaattgac 1440
   ttggtctttg cctgttggac ttagaataaa aataaataca ttattaaaaa gatgaaacat 1500
   ggtaaaagat tttgtgcatt tgagattttt ttttctttct ccaattaaaa ttattcacca 1560
   tgagaacgaa aggtgatttt tgaaaaacct ggaaaaccta gtgagttgga attgtactac 1620
   taaatgttgg aagatctgat caaaatttaa ctttaatagc cagcagctgt ttgaggacaa 1680
   gttcacctta catgaatgtg ggtggtcatt tgagggtaac ccaccaagct tagcctagcg 1740
   gtggccaggc cctaaaatag gagtcatctt ggagagcaag agcatttctg tatttgagtt 1800
   aatgctaccc accagcaatc aagatcaatt aactcttgta gtaaactttt taaatgaatt 1860
   ggagatgact acagaaattg ccaaagacaa tgtgggcaaa aatggatggg ctaagtgaag 1920
   gggctgcctg tatcagcatt tacttaggcc cagccccaca aagccctagc agttgccatt 1980
   cagacacctg tatgttaaca ttaagacctt tagagcatat gtttaactgt gagtattttc 2040
   ttccacatag gtattggcac tttttagcat gacctacctt tgtgtgtcat gctaaaaagc 2100
   atatatgact aatagaacac acacatttaa cttattatgg gcctgggttt cccgggtttc 2160
   agcttcagtg tatgtcccct ggcaggtttc ttaactggtc tgagcctaag tttcctcatt 2220
   tataaaacaa aggcagtaag tggtgccttt ttgcaatatt aaaattagta ttaaatggca 2280
   taacgttcac aaaatatctg gaacagtttg ccatcagtaa atatctgcta ctgcagttgc 2340
   aagggaaaaa caagattgaa tctttgttct cagtttctca ttatataaaa ttttagagat 2400
   tatttaaaga aaagagagag agcatctaaa tgaacagttt caatagcatc ttgtttatat 2460
   tcagaaatac agccctgaac tcaaacttga aacttcagtg ttagccaacc ttccctcctc 2520
   ccatcaccac ctgaaggaaa atatctttgt gtagcacaaa agaacttaat tctaattctt 2580
   tttttttcct tttaaaacag                                                2600

<210>  33
<211>  101
<212>  DNA
<213>  Homo sapiens

<400>  33
cgaactgcca gcctgcgcct ttgcagccgg ccctcgcttt gctgaagacg agcagctccc      60

accaaagtct tgcctcccct taccccgaaa gccccgctca g                          101

<210>  34
<211>  376
<212>  DNA
<213>  Homo sapiens

<400>  34
gtcaaagagc ctaaaagaaa agcccgggcc gggcgcggtg gctcacgcct gtaatcccag      60

cactttggga ggccgaggcg ggcggatcac gaggtcagga gatcgagacc atcccggcta     120

aaacggtgaa accccgtctc tactaaaaat acaaaaaatt agccgggcgt agtggcgggc     180

gcctgtagtc ccagctcctt gggaggctga ggcaggagaa tggcgtgaac ccgggaggcg     240

gagcttgcag tgagccgaga tcccgccact gcactccagc ctgggcgaca gagcgagact     300

ccgtctcaaa aaaaaaaaaa aaaaaagaaa agaaaagccc ggagccatac actcacctgg     360

ttgaagccat aaagag                                                     376

<210>  35
<211>  93
<212>  DNA
<213>  Homo sapiens

<400>  35
ttcaatattt caagtgaaaa tctgcctgtt cgaaaagaaa atattcacaa atgagcagtg      60

tattagaaga caactgaatt gcttctaaga atg                                   93

<210>  36
<211>  268
<212>  DNA
<213>  Homo sapiens

<400>  36
ggtctcactc tgccatcacc caggctggag tgtggtggca ccatcatggc tcactgcagc      60

cttgaactcc tgagctcaag tcatcctccc acctcagcct tccgagtagc tggaactgca     120

ggtgtgagcc accatgcctg gctgatttgt ctttttaaat tttttataga gaccgagtct     180

caactatatt gtccaggctg gaaaagaact tctttccctc caagtgataa ccttttccag      240

acaagtcagc ttaaaaactg acccaaag                                        268

<210>  37
<211>  3721
<212>  DNA
<213>  Homo sapiens

<400>  37
gtttgtaaag aggggtgcga gatctgaaaa gtatttgtct tctagggaga gagatgttgg      60

taaggggccc agagaaagac gggtcctttt tagctcatca acgcgccacg tcttgctttg     120

cagctccaaa atgatgccag cgctgctgtc cgaagggtca atattaatgc gttaagtgaa     180

atgggaaata aatggacagt ggcacatttc gtggcatcat ttttacaagg ggatgtcgaa     240

agttttaagc tgcatttaag aattagccac atcttgcctg aaagttaata ttttttgata     300

aactcgttaa taaagcctag aagaggtaag ttaaagttag ttaccacgta gatcctccag     360

tctcttgtag agacttgcgc aggttactag gatgaggacg agttgtattt ttaattttct     420

agactaagtg caaaggcatt gagttttgag gtggcagggt taatgttcaa gggcaaatgg     480

aaccgccaag gatctcttga aaagaattca cttactatta atacttaagc ctgaaataat     540

tataattaat ttacagtggg aattatatgc ccatgtttta gacttttttg ttatattagc     600

tatatgaaaa gggatgttct ttagtgatta aaagaaaaaa ccacctgttt attgtcattt     660

gcaatttgaa atagccgaca gcagtaaggc tagctacaaa agctaacaag tcaacttcag     720

tgtggtttga catttactgt caaagctttg agctctaaga aaaacagaat ttaaaataat     780

gtatataaat aataaaattt cattgtgact caatgtccct gcccctggag aagggacaga     840

tgacactcag tgactggctt ggaaccattc cccctgcaga tgcatttgcg gatacagtgg     900

gccagggctg cctctgagta tgtacagcaa gtgcttatgg acttacagaa acaaatgttt     960

tactatttag gattttctca atagtttaca ctaaaagttt tgcatgtaga aatgtgggat    1020

aaatatttct ttgaaaggaa aagtttcgaa aggaaacttt atacttgtat ttttttgtgt    1080

gttcacattt ctaagttcat ctgtctttca aaaaacagat tttcataaat attaaaatat    1140

tgcaagtatt atttttaatt cttttgagtg ccatgtttgc tgtaagtagt tttttctttt    1200

aagtaagatt aatataatcc tgtctaagga gtttaataga gagaacagga tttccaagcc    1260

tataaatgca gtctatataa aatagtggga tcaaagtttt ttgttaagaa acttgagaat    1320

ttatacattt taaatgaaga tagaagcacg ttttaaaagt agtttttcca caccatttcc    1380

ctccccccaa aagaattcag aaactatatt ccatagtcta aaaatggcat aatcagatgt    1440

aaaaattatt accataatat atagttttct caagatggtg gatacttttt aatatattat    1500

gaagagttat attctcagcc cactgtgtgt ttgagggaat ggaaaagaca agatgtttct    1560

aaacatattt tactactgaa atccataaga ctattaagct gtgcaggtaa ttgtgttcta    1620

agtggtagaa gtatttttcc aactgctttt taaactttga aaaatttata tgtattttaa    1680

ttagcacttt tcttttaaag gttccaaata tttagctata tcagctatgt gataaaaatt 1740
attggaattt gttccctact actttctgca taaacaattg agaaatattt tatactaatc 1800
tttttatata caggggataa atttgaacat gtgtcttgtt ggtgtaataa acaatccaaa 1860
ttcatttctt tcttgaaaac ttggcatcga tcttcatctt attttttatg cacacaattt 1920
ggacatgcca gtttgtaggc aagttttaag acagtgcaag ttttatgtgg aagaatgtat 1980
attaaggaat gaatagctaa tagccaaaat taaatcagga ggtgtggcat tcactccttt 2040
ggatgattat tttaaagata gttttgcact caggaaggat tttcttatgc tctgtattta 2100
tgaatatcta aattatcctg ccttgcaact tcaaaactca ataaccaaat accaaattta 2160
tgtattttca gtgtgactaa ggctaggaat atgagaagca gattggtttt ataaaagaaa 2220
gcaagcattt acaattgtat tttaaaatat aatcctaatt tcagtttctg acagaaatct 2280
tgtggtcagc tcatcagttt ttcagatcag tttctaaata gcatataaaa actacctttt 2340
ccacatcaga agttcatatt taggtttcct taactgcaaa atcactttaa aagagagtgt 2400
tttatgtgaa gggccatttc atgtgttggt tccatcgatt acatatgaat gtataaaaat 2460
cttgtttaaa gaccagtaaa agtgaggaaa ttccaagtca ggctggctaa ttccattcat 2520
aattcatccc gagtacctga ctgaggaaac ttgcaaacag tttatagcta tacatacaaa 2580
ataagttgac gtgctttgta agctggaagc agagttaagt ttgacctccc tgcttggaag 2640
gtcttaaatt aataaaatta tggaatatgg ttaagctttt cagtttccca agaattttcc 2700
aatatcgatg ctttttggat ttttgcattt tgaaaatgta agcatggaag gacaacttct 2760
tttgtgctga gatgaagtta gcttttaccc atccatttcc ttcccactta acacatttgt 2820
tcagtaattc ttcgagtgct gggggacaga ggccctgcct tgagaaaagc atatgccatt 2880
tctctgcccc cttcaaccat ccccaccccc agccctccct ttatttcaag ccagttggac 2940
aagtatttag cattgacaca tgtttcaaac tgctgcatcc acagccctag gtagttttgg 3000
ggattggcag gaaatccatc tagactttgc cttctgagct gggtaaaact actaggaagt 3060
cctatgcctt gatattgtgg cacttctagg ctgtctttcc aattttaagc aatccatcag 3120
ttgttttcta agactgcact gtagactgat gccttctctt agttttgatg ttcagaagat 3180
tttttaaaca tgttcataga tttcttaaac attctgagta attgtaattt taaactagac 3240
taattttagg gttgaaaact gtcccagaga gagtccttac ataataactt caatctgatg 3300
tgaactcgaa tgacttttta aaaataagat gttgaaaatg tgggtttctg gtggaaaagg 3360
ctgccattca cttagagtgg gatccttagg accagttaat atccaccaga catgcttcaa 3420
taaactggaa caagcctgat gtggaattac tgactcccca ttttcctgaa tgtaggtcat 3480
tttgtgtcct tacaatttaa cgtttgaagg gatttcctgt ctttgaagtg caacagtttg 3540
tcacctgctt ggctagctgc cttaacagtt ggggctcctt tcctacattg aaagacttag 3600

tgactccatg ttcccgggct agagtttccc agctcactga atgcatgata cctgcacctc 3660

tgggactctc ctctacaagt tagtgcctgc agatcccctg ttccaagctt catttctcta 3720

g 3721

<210> 38
<211> 239
<212> DNA
<213> Homo sapiens

<400> 38
gtaagctttc ataacgtagg ctatatatca aaattcatag ctttttagaa ccggagtaac 60

tagtgctcag gcattagagt ctcaggctag agcaaaacac agcaactggt gccaaatgta 120

cgctagtggc tgaaacccat ttaagtaata aacaaacaaa tgtcctttta agaatgtgct 180

attattttta tttaagaatg catatgactt tatcgcccct tatgatgtga atcttacag 239

<210> 39
<211> 2600
<212> DNA
<213> Homo sapiens

<400> 39
gtgggtatgc taatttttac caaatgatac aacatgtgca tgccaaagct ctttgtaaac 60

tataacacac ctccaaatga aagcaatttg catatatttt aataaacttg tctgccttta 120

ccacatccac caactaatcc ttatgctccc actgcaaaga ctctcctctt tttggagagg 180

aacaccaatt gtttgaaagc ctggactttc aaacaatctt aaggcatctt catagtatcc 240

gtccttaaga gtgctttcat cttaatatta ggtaaacatt tagaagatta tgaactttgg 300

ggactttctt ttcagagaac tgaacacgtg tatataaaat tacaatatcc tgataagtga 360

ggggaagaaa ataaaaatag gtgtcaatac agttatggct taagatcttg atgaaattga 420

gaagaaagta gaaatcaaat gcatcaaagg aagaacggtc agactgaatt ctgtttgtaa 480

ttttttcaaa atgaactgaa atgcagtttt tctgactata tgttttcttt tatggacatt 540

aatcactttg attcatttaa tttctctgga agaaactgta gcttttgtaa cagaatctgc 600

tctattgggt tctgtatggg cacctaccgg tttgctttct agccagtatc atgtgaattg 660

tctagagaaa atgcctttag caaacacctt caaaaagcat ccattccaag ggcacacctc 720

ttgtgtctct cacaaagcaa gtggtactat gtctagccaa ggaggcagac tcagattcat 780

gcatttatct caggtttttt ggtgaccttc ttcctagatc ctgctcttgg caactgtaag 840

ccaagtgtac ataagtatta gaatggattc tctaggtacc aagtgaaaat tctatctcta 900

gtcacatgct cctgcacctt tcatgtggga aaaatgttcc agtctttcct tgattacatc 960

acagatgata cgagaacagg acatgggaca gggaactgct aaacagctgg atatctctgg 1020

aaactcagag cacttaaaaa cactttgaag ccatgcagct gctctttttt tttttttaaa 1080

ttgtcttcag tgaaactcca aaaccccaat aatatctacc ttaaaagcat caggagtatt 1140

ttaattgggc aataaccatc catattttcc ttctgataaa ttaaaatgaa ttaagaatga 1200

aattttatga tgattataaa gtatattact gacccaaggg acaagaacaa ttatgaaaaa 1260

tatgtcctac atatttggca tgaatatgaa tttagacttt aaaccattca tcttaaatgc 1320

tcaagcatct tattaaagct tataaaataa tattcacata ataacagagt aacttataat 1380

catgattgat attatatcat caatattgca gcaggctttg tgccaaatac tgaaattgac 1440

ttggtctttg cctgttggac ttagaataaa aataaataca ttattaaaaa gatgaaacat 1500

ggtaaaagat tttgtgcatt tgagattttt ttttctttct ccaattaaaa ttattcacca 1560

tgagaacgaa aggtgatttt tgaaaaacct ggaaaaccta gtgagttgga attgtactac 1620

taaatgttgg aagatctgat caaaatttaa ctttaatagc cagcagctgt ttgaggacaa 1680

gttcacctta catgaatgtg ggtggtcatt tgagggtaac ccaccaagct tagcctagcg 1740

gtggccaggc cctaaaatag gagtcatctt ggagagcaag agcatttctg tatttgagtt 1800

aatgctaccc accagcaatc aagatcaatt aactcttgta gtaaactttt taaatgaatt 1860

ggagatgact acagaaattg ccaaagacaa tgtgggcaaa aatggatggg ctaagtgaag 1920

gggctgcctg tatcagcatt tacttaggcc cagccccaca aagccctagc agttgccatt 1980

cagacacctg tatgttaaca ttaagacctt tagagcatat gtttaactgt gagtattttc 2040

ttccacatag gtattggcac tttttagcat gacctacctt tgtgtgtcat gctaaaaagc 2100

atatatgact aatagaacac acacatttaa cttattatgg gcctgggttt cccgggtttc 2160

agcttcagtg tatgtcccct ggcaggtttc ttaactggtc tgagcctaag tttcctcatt 2220

tataaaacaa aggcagtaag tggtgccttt ttgcaatatt aaaattagta ttaaatggca 2280

taacgttcac aaaatatctg gaacagtttg ccatcagtaa atatctgcta ctgcagttgc 2340

aagggaaaaa caagattgaa tctttgttct cagtttctca ttatataaaa ttttagagat 2400

tatttaaaga aaagagagag agcatctaaa tgaacagttt caatagcatc ttgtttatat 2460

tcagaaatac agccctgaac tcaaacttga aacttcagtg ttagccaacc ttccctcctc 2520

ccatcaccac ctgaaggaaa atatctttgt gtagcacaaa agaacttaat tctaattctt 2580

tttttttcct tttaaaacag 2600

<210> 40
<211> 1907
<212> DNA
<213> Homo sapiens

<400> 40
ttcccgcact caggcgccgg ttgaggctgc gctcctggcg aaggacaggt gctcctttca 60

gccggttaac gttcagctca agaccggatt aagtctcttg ctctccaaat aaaattgtaa 120

aggtttggaa atatagaacc acatttttac caaaaaccta ctagagagca ttcgtcagct 180

tgcccttttc gccccgaaaa acgacttttt cattcacagc ctgtctcttc ctctttttaa 240

atttatgccg acagaggaag aagctgatat ttaacaaata acaagaggtt gcgataaata 300

gaatttaagg tgggaaagca gccgccaaac tgacaaatcg tcttcgagac cgacaagaaa 360

atagtagtcc ggaatttggc taatggatgg agaaggtggt cgtttttcat agacccttac 420

ggagaatatc tagtgaagct aaagagatca ccataaactt atgcattcaa acgcatttct 480

ggaacagagg tcgtaaccga ttttgtttcc atcatcagtc ctgaacagtt gattatttat 540

ataatcaact gttatttata taaaggtaat aaaaatatta cctttcctcc tcctttacaa 600

tgaatatctg ttagaacacc tgccagtgta acaatcagac tacaaggcta caggattatg 660

attactgtct cggatgatga gcccggggtc ctacaaatca tgacccccgc cggcccctca 720

ttctgcccat atttgccaaa cgtctgcatc agtaatttga ataatttaaa atagttccaa 780

aacagcaaag ctcagcagtt acgcaaagaa acaagaatgg aaaacatccc ctcaggcaca 840

gagaagtgtc cacccctttg ggtggaggaa ttatacaaat caggagaaag ataaggacaa 900

tagcagttca tcacgattta tcccgcctct gcaatgttct agacaaatta actcggagcg 960

ctccaatgga cccgcatttg tctctttact attcaatcgc tgtaagatat acggagactg 1020

catcacttta aatgggagta tcaattgctt tgtggttatg ctggattttg tgttattgct 1080

cagtcactaa agtaagaaaa atctatatgg cttgtcaaat ttatgcctat tgtatttcgc 1140

cccctgctgg tttagctcgt gtgcaagctt gaatttgtgt gtgtgtagaa actagaaatt 1200

gattttaaac gaacacatgc ttgttttcat tttacagctc tactaaacag aaatatcaaa 1260

ataagaacat atgactagac cttaaagcag accctacact aagaacatgt ttaaactaaa 1320

tgaatttaaa ctgcactcca gcctgcgcga cagagtgaaa ccctgtctaa aaaaaaatta 1380

aattaaattg cacacccccc atcagcttac aattttgtaa tagggaaatt gagtacctat 1440

ttggccaact aacttgtaat tggtaatctt acatgtaatt ccgaagacag tgccctaatg 1500

ctcatgacat tttattacaa agggaaagta tttgagtgtt tttaaggtga taaatctgtg 1560

ggaaatataa agtccatttc atttggagtc aatgatagta ttgtttttat ttacctttta 1620

aaaattatgg ccataactgc attacctaag atttggcatc aagaaaagaa gaagaaaaaa 1680

attaccagtt aacatttgtt gtatttactt ctagtttttt tttcctgtgc gtacttttta 1740

caaacttgta atcataattt aagtagattt tgtgtccctt ttgtgttgta agcatttcca 1800

cccatcgttt aatgtaaaac cttaatttta attggctgct tagtagtcaa tggaatgacc 1860

actatatgga tagttttcag tattataaaa taatacttag atgaaca 1907

<210> 41
<211> 2037
<212> DNA
<213> Homo sapiens

<400> 41
atagaagctg tattacagaa acttttaatc tcttttctat gtcagtaagg ggatgctgaa 60

ataaactgtc tagaaaaaca agaagcttct gtcacaccca ggccactttc ggcagagtgg 120

tccagagcgg ccagggcacg gccagctgct ttgcatctgc attcccgttt cattaccttt 180

tgtgttgtgg gccaacagga gtgctacgta gtgggggctg gagatttgtc caggcaggag 240

gtgggcaagt ggctgtcttc tctgcagaat acctgcactg ctccatctga ccagcagtgg 300

caacagccac aaccagttct gcatctagct cctgcccaga cctttggctg ccagaatcta 360

gaaccatggc tgaccaaagc gagacttccg agtaaggttc gttaacaggc atcctgctga 420

attagaagga aacaggaacg aatccaaagc aaacggggct aaacttccct cgaggacatt 480

tcctgagctg caacatggga agcgacagca ccgaagctgg tgagctggtg caactgcaca 540

caccttttct ctggttttta gtactcacag gtcatcctgg ggagaccgct catgggctat 600

gggcagccca tgcctggatt ttttggtgaa tgagtctgaa aagcagtgac caggtgtgtt 660

gagggcctcc ccttgcctgc acctttttat ctctgagtga ctgagggctg ccaaggccag 720

acaacgcaca tgtgtcctgg atcctcccct ggcctggggc agcagcagca gcagcagctg 780

ggcttggggt gagtaggctg gcttgaggaa ggggctgccc acaagcaggg ctcatttcct 840

gggtcagggc ctgtcagact caaagtcagc tcttcagcat tctgtttaga aaatgatgat 900

gcagttcagt gagaaagaaa gctcaggata aaatatgttt tctggagggg aaatactcaa 960

gtgttctaag ccaggaacag agactctcaa gagaggaatt ctctggcatt ctctgccttg 1020

gataaatcat ggattaaaca tgagcatcct tagaataatt ttatttttgt atttcacttt 1080

acactgtaat gcaaacagct tttatgtttt ctttgacgaa gaatcataat tgagtcactt 1140

taggtctttt agctggaagc atttcaccct aaatgaaaag ttaatgcatt ttttacaagg 1200

attaacatgc taactacaac ttgccctttc agcagatgac aataagggat tctgaaaagt 1260

ttggactgtc tttttctttt tgtatacaaa taaagcagca gatcctttct ctgtgaaggt 1320

tgttgcagag ctcccgtacc agcttataaa atgcggctgt ttaagcagga cttgggtggg 1380

cagcagaatt ctgcacatga cgagtggctg cagcttagaa agcaaatttc atctgattcc 1440

agtactgtga ttttaaggaa acggtaaaac tcaaagtgcc agtaatgcca tcaaggctca 1500

gtaacatgcc atttcctcag cagctaaaga caaaaagaat tattttggtg aggggataga 1560

aatcctttcg ctttgcagtg acacccactt tttcttagct tggcttggga gtaaggagat 1620

tggagtgtgg ggggacatta gaaactcatt taaggcaaac tgaaatctcc ttcatcagca 1680

gtcttcctgc tggtgtagcc acagcagaac tccgaggctt gcaggctggg ctgtctcctt 1740

gctgcctggc ttgggtgttt atttgggcaa tgcaagctga cattgtgagg tgttgccgat 1800

gcccaggcca gctagcaggg agccctccca gggctggtag aaacctatgt gtatatatat 1860

tctcaaattt aaatagattg tacatagtga aatgtaaatg acatgtaaaa cagcaatctc 1920

tatttttctt aactattata tagcagtata tatttgttga acatgcttgt atactcttca 1980

ttaaccattt taatattttg tacagataag ttgattaaat attttattca taaaacc 2037

<210> 42
<211> 11210
<212> DNA
<213> Homo sapiens

<400> 42
ctcgcccccc cggagccgac aaatggccgc ggcggcctca ggtccggcgt cactcagaca 60

agggggcggg tggaggagga agcggccaag tccacagtcc tgctcccgtg ccgccgagca 120

ccgcccacct cagctgacca gccctgtccg ctactaggcc tcgcctagag gagggaaagg 180

ccgtggcgtc cgctccctcc gcggctcatg gcgacgacgc ttggcacatc cgggaccctc 240

aggccgctgc agtcgcgtcg ccggctgctc gggccccagc cccggccgct gtggtgactc 300

cgccgcgcct cgccgtcgcc cccgtcccgc ccgccgcccc agccgccagg gacatgtcta 360

acctcggagg ccgtaggaac gggcctgtcg aagtgcgcct gacagggaca tgccaccccc 420

gttttaggag aacttgtatt tggtttgaga aggaattgga atcacggcgt tatcctacct 480

tcaccattta cccctgataa attgtccacg tttatatttg gagtctcatt ctattgccca 540

tgctggagtg cagtggcaac agcgtgcagt gagtgatccc ggctctctgc agctttgacc 600

tcctggctca agtgcttttc ccaccttggc ttcccaagta gctgggacta caggaatcat 660

attgacaggt catgaaaatg agaccaaaag atctctggaa gcgattaatg ataaaatttc 720

atggagaaga aggccttgac tacggagggg ttgccagggt gatgacaact ccgtgagggt 780

ggagattata catctctcat catttcagca acaaggaaat aaattagtgg cagagtaagg 840

gtgacttgag atctcttgga agcgaggatc ctttaaaaat cctggaatat acactgcagt 900

aaaagaacaa agcatacctc agccttaaac gactgaagaa gaatgtcaag tggggaagtg 960

gctttggttt tcagtttgtg ggttctgaat ccacacaaag acaggattgc tttctgaaaa 1020

cctgaattaa ttattgtcct tacctcaata agacaaaaaa ttagaatcaa aatcgttagt 1080

attacagtca cagatatcac caagattagt ttgttgttat agccatatcc tggaacttct 1140

ttcgtgagct aaaaaaaaaa aaaaagaaaa aaggaaagaa caattaaaaa aagagtgaaa 1200

gctaactttt caaaacccca gtattctagt tgagtagctt acaggttctt tttttttttt 1260

ttttgagaca gggtctcact ctgtcaccca ggctggagta cagtggtgtg atcacagttc 1320

actgcagact cgacctccct gggctcaggt gatcctccca cctcagcctc tagagtagct 1380

ggaattatag gcacaagtca ccacacccaa ctaatttttg tattttttgt gaagataggg 1440

tttcactata ttggtcaagc tggtctcaaa ctcctctgac ttcaagtgat ctacccacct 1500

cggcctccca aagtgctggg tttacaggcg tgagctacca tgcccagcct acagttcatc 1560

ctgtgcacta atctgtttca ctctctaaat gaacaaagtg ggagatcact gtcatggcca 1620

aagttacatg gccaagacaa actatggcct gggagtccca ggttctcctg tgtgggcact 1680

ttcctggcat atgctaaatg atgggaaagt ctgggtctca tgtttctgtg tggtcctcac 1740

ctcactcgac ttctgctctt tctgttcact ctgggcttct gtgctttcat taatatagtt 1800

cttagttttc cactggtctg tatcccattc tgccttggac gcctttactt cctgctgctc 1860

actgagaagc ctcatcagga ggcctgtcct ggagatgagc ttggcacagg tcacttgcac 1920

ataggtctcg gagcagtccg tcttcaaggt ccagataaca tgagaaatga agcttctcac 1980

atcgttgttg gggatgacgg actgtagttg atgggttagc ggaatttcaa actgatcacc 2040

tggggatgag agcaatgggt aactgaagct tttgggctcg ggggacaggt cagtgcccac 2100

gttgttgtat tcccattttg tctcagtttg tttaacagtt ggctctaagt tgaatgcagt 2160

cctagtggga atctgcctca ggaagatgat tgtagtttgt gttttcagag atggtgcctt 2220

ctggcatagt agtgttttcc ataaaaacgt tttcttcaga ggcatttctt gcagttgtgt 2280

tttttacagt agtgttttct ataaaacatt ctgaaggagc aaatacttct ggaaaagggt 2340

tttcttgagg actcaggtct cctgaggatg aaaaagcccc ttgtgaaggg gaatttatga 2400

ggctcttcac tgcagagaac ggcggcctct ttgcgagcat cagtctattg agataacttt 2460

tctttctcaa ttttctaatc tttttggcct tgggtgttct gtgggccaca cgggagtgag 2520

ttttatgaaa gtggtatttt tttctggaat gtactactcg tttagcagcc ttcatatttt 2580

ttaactctag cctttgcatt ttctaaaatt aaaatggtgt aggctaagtc tttcaatctg 2640

tctatgacct gaggtagggc ttttgcagtt gtggaaggca gaaggcatgc ccatggagaa 2700

gggtttcagc aaagaggaga ctgttggctt ctgctcttgg gtgaatgaag gcttggtgta 2760

gacggcgttt cccgctacgt tctcgggccc ctgcactatg tgaggctatt ccagctccct 2820

tggggctgga ctcccgagcc tttttctttt ggcagcattc tccccaaatg cctgggcacc 2880

ctgttccctc ctgatactct gctttcccac cgctttgaag ggccttttct ggatgctcct 2940

tgggcccatg aggactctgt tcactctctg caggtttttg cctacacttt gaacctttgc 3000

caggctgttt tcctgtggtt ggacagtttc taatttcttt tcaaatattt ggcatttaaa 3060

tttgttacct agaaggctgg actgcataag catggcagtt ttttcttccc ttttaacttc 3120

atcgattttt ttcttgtatt tctgcatcta acatcttttc tagcaaatac acctttctca 3180

atttattttc ataagttgaa ttgttggatg caggttgaaa caaggggctc tttgtattgt 3240

ttttcaaaac aggagcttat ctccatcttg cacatttgaa aaaagcagtt taatgaatgg 3300

taacaacatt gattccgcat ctagatttcc tgctgagaaa taaggcaaga tgtaatttag 3360

cgcagtgata acatcactct tatcataagt gtctagctgc tcactcccaa agactgacaa 3420

gttgatgcca ctgctgtctg aggctgcctc cggctcagta gtcagctcaa tgctcatgtg 3480

cttcttctgg gcttgtaaca ccttcatgaa cactctttct ggattcccta cagatgcttc 3540

ttcacctgtc aaaaaaaaga gactgctttg atcatgaaag ataatggaac ggcatgcatc 3600

agtccacagc tgtatgcccc agtcacacaa agtaggagtc agcaaacatt agagtgccat 3660

tcagagagga gaaacacaca cccaagccta aatctatgaa atggcaacaa caaaaggagg 3720

aaaagacatc ttttgaaaat atggctactt acttggaaca ttcctagtgt gacatagagt 3780

aactctgctt aggattattt cgttgatccc caggggccaa ttgcccagtg ctcagtcaaa 3840

gcccaaggtg gaagacaagt gcttccctga tgagctgtcc tctctgcaga ctgctccgta 3900

ccctgtgctg tcctgcctca gatgcagaga gagcacaagc ctccctcctc atcctcagtg 3960

tgcctgtgtt cttactacca tcacagctga atgcaatgaa aggctatcct ctgagaggag 4020

cagggtggag atgctaaagt ggaggccccc tcccattgct gatagatcct catctggcat 4080

gcactccacc cacccacccc attctctgct accacatatc gtagccccat taaagaagaa 4140

gtgacatgga aaaaacactg tgcccaccct agtccttttc ttaaatttgg gcagagatcc 4200

agggtatagg ttaagagatt tttaatttgc cagatggtat gcctatgttg ttaaatatac 4260

aatgaatcta tggtatgata gcagtttctg gataaacatt acttgaggtc ctaaaatgca 4320

gaagggaaaa agcagctttt gtcagatgct tactttgctt tcatttcatc tctaatattt 4380

tggatgggga accatccaaa gcttctggcc gcatgaaggt caagtgtgcc agtgtgcagc 4440

tgggtttctt ttccagaatt aaaagtattt tgggtggtgc tgagggtcag aggaagaagt 4500

aaaaattgtg agaaaggaga aacatgggct tggggagaac ccagaattgg ggacagaaga 4560

cctggcacta agctatagca cttagcacct ctgatcttgt ttttcctcgt ccgtaaaagg 4620

agattaacaa tgcttttctg cccacctctt ggggagaagg gaataattta gttggtaaaa 4680

aaaaaacttt tgaataataa gcaactctgt ctttatataa gtagccaagc attattaatt 4740

atccacccca tatcactggt agatacctgt attcaagcta tgtggacata aaagcagtca 4800

cattttagaa gtcatgaagt tggtgctaat aaacctaatc tacagaaaaa ctcttgaaag 4860

cacttgagca tttgttctgt gaatagaaag gtttgagatt cagagcaagt tcagagttgg 4920

atggtctaag aatggaaaag ccctccattc cattagaaga gccagggagc aatttctggt 4980

tatggaacca gaagctctca ggcttcaaat aaaacagcat cacttgtact cttataaaat 5040

tgtaaaaaca aaaacaaaac tggatctaca tttgtcctaa aaggcagaga gtacttgaga 5100

cctcatgcat ataaaaccag cttacaaact acatcgcact atatgaagaa attatcactg 5160

ggggcaaatc accaagtagg gagcacaata cacagggtgt ggatgttaat gtcattccct 5220

agccttctca ttcccttctc ttggtccttt atgcatatgg aacagttcca ttattaaatt 5280

ttgtaataat aactgagaac ctgactccca gcaagggagt agttcagaag ttgagggagt 5340

ttaaatctga atgagtaaat aaagcaatta tatcattagc ttaaaatttt atcatcaatt 5400

aaaaataaat ttaaaaacaa atacttaaaa taatgtaaca atttatcacc aggcaatttg 5460

gactcacgac aatgtgtggt gtttgtcaga catgcactgt tgcaatgcag cttgactgtc 5520

ttgcagacag cctcaatgct atttttaaat tggcagaggc agcaggccat atggctaggt 5580

aagatcctat aagatgaaaa cacaaaacaa taaatagtgg taaagcagtt acttgagtag 5640

gcaaaggagg caggccaacg ctaccacagg gctgggcaga aaggtgttac tgaggcctgt 5700

ggactggaca gttgggtagg aaccagaagg gcaatgggaa ggaggacaaa ggtgcccaac 5760

tgaagggtaa gcatggcagt gagtatggta tgcctagaat aaaagtggtt gggattagaa 5820

ttgggtgaca ctgatcagta gtttaattca gaagtatctc ttcccaactc aaaagtctca 5880

ctttgggctg aaagtacaca ggaagaaagt agacttctaa gaagagtctg aataagcccc 5940

caacttctgg agtccctttc tcaatttctg ttggcagtgg gaaatattag aaattactct 6000

gggcattaaa aatagctcag tttaacctgg attgtggggt taaaaaaata acagattgca 6060

ttggccaaat ctcgacaatt tgagcattca aaagagtaac aataagctac aacataatta 6120

atatataaaa aagaatccat gaagaatgat atttaaaaag agagggagtt gttctttaat 6180

gaaataatgc cagctaatga atgtagaagg aatgacagaa ttttaaaaag tgttactttg 6240

caaccaccaa tgtaataaaa attgattcag acaaggattg tcattgatgg acacagctgg 6300

gtgagaagac atttgagaac aggatcttta ctgatctcaa agtaccacca cacagatatt 6360

ttattaatta ccaaagggaa aaattatttt ttattttttt atgagacaaa gtctcactct 6420

gtcacccagg ctgaaataca gtggcaaaat cgtagctcac aacagcctcg acccccaccc 6480

tgggctcaag tgagtctcca aattcagccc cccaagtata tgggactata gtcttgtacc 6540

accacaccca gctaattttt tttttttttt tttttttttt ttgtattttt agtagagaca 6600

gggtttcacc ttgttgctca ggctggtcta aaactcctgg actcaagcga tctgcccgtc 6660

tcggccttcc aaagtgctgg gattataagc atgagccacc acacccagca aaataactac 6720

aatggagaga tctggaagat caccttaaac aagtgatcaa acttagcatt atgagccacc 6780

tgcaatcacg aggcaggaag gatatatcac ctatgcagta tttttcccaa aaatgcttac 6840

cttgaatctc atcatgtgga aacagacaaa tccagattat gggacaattt acaaaacaac 6900

tatctttgac tcttaaaaaa tgccatgtca tgaaagatca aaaaaagtag aggaatgttt 6960

tagattaaag gaaatgaaga catgacatgc agtgcctaat ctttgattgg attctgtgct 7020

attctttcat ctttctggta tgtttgaatt ttttttgaaa tataaacttg ggcaaaagag 7080

ataaccaaga taattgatta atttattgtt atggctcatt gggggcagtt tcagagaaat 7140

aaaaacaatc tttgtaactg gagtaaattc tcaaggttaa tcttaagcaa tatagcatgg 7200

tcattaagaa tacagattcc acagccagac taggcttaaa tcccagctct gtgaataatg 7260

tgaatttggg cacattgatt aatctctgtt ccttggcctt gtcattataa tagtacctac 7320

ctctaatgag ttttgaggat tagatgaatc agtacctgaa aaatgcctga cacacagtga 7380

gtgctcaata aatgttaact actgtaatta ttacattaca gaggttgtgg gggggccttt 7440

tctgagtcct ccaaaaggat gactttatta gggccacatt aagactgtgg aaacagaaga 7500

gtattcaaca gataaatgaa gtctatgagt tgggggtaca atttatggag ttttagatta 7560

aaactgcatc caataagttg gtctgacaca tctttcaaac ctatagagga ataatcacaa 7620

gtgactagta ttcctttggg tccaatagag gtctctgatc ttcatacaga ttgaatgtac 7680

ccagaaccgt aacctagcat tttacttata tagcaacctt aactctgaca caaaagtgtt 7740

tcttttgttt tgaggccaag tctcactctg ttacacaggc tgagtgcagt ggtgcaatct 7800

cacttcactg cagcctctgc ctcctgggct caaatgatcc tcccacctca gcctcctgag 7860

tagctagaac tacaggtgag tgccaccatg cctggctcat ttttgtattt tttgtagaga 7920

tggggtttca ccatgttgcc caggctggtc tcaaactcct gagctcaagc aaccctctct 7980

ccttggcctc ccaaagtgct gggattacag gcatgagcct gaagtttaat tgttattctt 8040

ttcctgcccc cgacttttga ttttaaacat tttctttttt tcctttaagc cttaggatgg 8100

ctaggaagca ttttcagatg gtataatgaa cacctgtata actttcatct ggaatcaaca 8160

gttgctaata ctttgccaca tttgctttcc atgtgtgtat gtctatacat tttctggaca 8220

aaccatttga gagtcagttg cagacaaaat gaccctttgc cattaaagac ttcagtgtgc 8280

agcccctaag aagcaaggca ttctcctgca taaccagagg actatcatca cccaatggaa 8340

cttcatatta tcattgtcta ctatgcagcc catatacaca ttttcacaat tgtcccaatc 8400

ataacatggc ttaaaaaatt cagaatccaa tccaacatca gacattgcat ttagcgttca 8460

tgattttttt tttttttttt gtagaaagac ctttaatact tctgtttaca aaattcaggc 8520

atacatttca gtttgccctg aaccgtgccc aaggctgtgt gctcatctct gccaggatgt 8580

ctgtgccctc atgtactgct gacgtggagg gtgcagagca gagcctgggg tctggaggct 8640

tcgctgggcc tcagggggag gggaatgtga atgtggcccg gcccagagga ccctccattt 8700

catcagtttt ggattgggca acagaggaag cagatgtctg ggctgcagac ctgctggtct 8760

gggcaggggt ttgggaaccc cggggttgcc tgccatggtc tagaggagat ggctgcggct 8820

acttcccaca gggtggggtg gccactaggg gcttgaagat ttgctcactg gagcctttgt 8880

gcttggccct cagcagcaca gctggggctg tggtcccgtg caaggtgtgc tgagggttgt 8940

gtaggtggct ggtggtggca gcttgtgcca gagtgacaca ggcttctgtg ggcctggctg 9000

ggggcagtta aaaagccgaa aaggtacttg gctttgtgag ggcaaggctt gggaggcggg 9060

gccctgcagg acgtcatgtt ctctgtcctg gttaggaggg gggaggtgtt ggggggggga 9120

aggtggggca gatcccaact ggcccctgtc cctgggctgc aagctgacag caggccaggg 9180

aagcagtgag gccttctgcc ctggccttgg ggagaaggag tgcatgtgtg aggctgggat 9240

acagtggccc tggagtgggg ctagggctca tcgccagcac caggcgcggg gaggatttaa 9300

aaccagagtc ctgtctctga gccaccccac agggacgctt gatccaagcc tgttaaggcc 9360

gtggaactcg agggtaggca gtgggctggg ccaccgtggg ttcaggatgg aggtgtgggg 9420

gctgccctgg aggaacgtta gtgtttctga gagcacctta gtgttgctct gcctcagcca 9480

gctgggcaag gagttgggga gcggggagga ggccggcgcc atggagtggg gaagggagcc 9540

gtcactgttg gggatcagcc gctagtgtaa acgagcatct ccacccaccc agataagccc 9600

cccacccaag tgtggagccc tgctcaggga gtacctcaag ggccccggcc tgggtggcgc 9660

cagcaggctt ggacactgcc ctggcagcag gggccccgtg gaaggcagga tacccagcag 9720

ggggtgccac ccagtgtgga tggaggggca gggcgggggc gcccctgagt cactgggcag 9780

aggcacgagc acgtccacat agctgagcag gaagaagccc gactggccgt ccagcatgcc 9840

ctcgtatcag ttctcgttca tctggttgat cagcatgaag acatcgccct catggaagcc 9900

cagctcccta tctttctccg gctccaagtc atacagccct tgcagctcgg ctggtctagg 9960

ggcggcatgc tcctgctagg agtccagatg gacttgtcgt aagatcggaa agaggacgaa 10020

gctgcgatct tggggactgt ggtgcagggg aagccccggt tggactgctc aggctttccg 10080

aggtcaaagc actcccgggg cttgggctta tactcccgcc tgggtcgtga ggaagcttcc 10140

cggatcctgc acttgaactt ctgggccagc tggtccagga tctgcacggc ctgccggtgg 10200

tagtccagct gcgcctctgc caaggccggg agttgactca cctactggat gtcggtctcc 10260

aggaggttgt gcatgctggt ttctgccacc tccttggact cctcaaactt ctccagcgcc 10320

tggtgcagct cctcttcggg gatcttgccc tgccgcttct tttagtcaaa gtccaggcgg 10380

cggccctcca gcttctgcag gtggtgctgg atctccttca ggtctttctc acataggttc 10440

tggagggggt caatgaagtt atgcttgacc tctatgtcca gggagtcctt cacctctgcc 10500

aggtgcttca tgggctcgcc ggcatccagc agtgcatcgc tgaagttgga ctcgtcgcgc 10560

agctccttcc catggcggat caggcactcg cccaggagcc cccctgactg caggtagccg 10620

gggttcttca cctggccccg gatcttgcac gcggtgttga gcatggtcag tttagcctgc 10680

gaggctgggt tgggctgcgg gtactcgatg gtcctggaca gtacttacgt caccgccttg 10740

ctgatgacat ccaccttctc catctcttcg aagtcatcat ccagcttggt cccctcagcc 10800

cctccgacct tctcactgac cagttggctc gccttgtaga actgcttctt cagcccggca 10860

accgacatgc tgcctcctgc cgcggaacct tccgcccaga cccccctgcc cagaccccgc 10920

cagcgacagg ctcccgggca ccaccaaccc tctgcgcgcc tcagcggccc ccatcagcac 10980

cgcctccacc acccgcttgc cagctcggcg cccgccccgg cgccgcctcg agttcatgat 11040

tctttagtct ccttcaatct agatctgttc ccaggattgt ttaaaagcat actgacaaac 11100

ctttgagact gtaaatgacc tgaggtatac attatcttta cttgagaata gtaatttttc 11160

aatatgcatt aaagatcatt acacatgagc taaaaaaaaa aaaaaaaaaa 11210

<210> 43
<211> 1752
<212> DNA
<213> Homo sapiens

<400> 43
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg gggaatgggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagccaag cttcttgaaa ggcctgtcta 960

cacttgctgt cttccttcct cacctccaat ttcctcttca acccactgct tcctgactcg 1020

ctctactccg tggaagcacg ctcacaaagg gctaatctcg ggccttgtcg aaggaagagg 1080

ctgcagacgt taatgaggtt agctgctgga ttccagtatt cgtcgcataa ggatccttct 1140

ttgtctgcga aggaaaaaca cactgattat cataatgagg cacgtgggcc gtggcccggc 1200

tgggtcggct gaagaactgc ggatggaagc tgcggaagag gccctgatgg ggcccaccat 1260

cccggaccca agtcttcttc ctggcgggcc tctcgtctcc ttcctggttt gggcggaagc 1320

catcacctgg atgcctacgt gggaagggac ctcgaatgtg ggaccccagc ccctctccag 1380

ctcgaaatcc ctccacagcc acggggacac cctgcaccta ttcccacggg acaggctgga 1440

cccagagact ctggacccgg ggcctcccct tgagtagaga cccgccctct gactgatgga 1500

cgccgctgac ctggggtcag acccgtgggc tggacccctg cccaccccgc aggaaccctg 1560

aggcctaggg gagctgttga gccttcagtg tctgcatgtg ggaagtgggc tccttcacct 1620

acctcacagg gctgttgtga ggggcgctgt gatgcggttc caaagcacag ggcttggcgc 1680

accccactgt gctctcaata aatgtgtttc ctgtcttaac aaaaaaaaaa aaaaaaaaaa 1740

aaaaaaaaaa aa 1752

<210> 44
<211> 1785
<212> DNA
<213> Homo sapiens

<400> 44
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg gggaatgggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagggcta atctcgggcc ttgtcgaagg 960

aagaggctgc agacgttaat gaggttagct gctggattcc agtattcgtc gcataaggat 1020

ccttctttgt ctgcgaagga aaacacactt gattatcata atgagcaggt tcccagtgcc 1080

cccgacaagc ccctgctggt gtctccatct cctgccaagc atcctccagt gcctcctcct 1140

gtgggcctgg cctcagggct atggacagac tcctgtccca tcccagagac ccctcgtgat 1200

cgtgccctgg cacgtgggcc gtggcccggc tgggtcggct gaagaactgc ggatggaagc 1260

tgcggaagag gccctgatgg ggcccaccat cccggaccca agtcttcttc ctggcgggcc 1320

tctcgtctcc ttcctggttt gggcggaagc catcacctgg atgcctacgt gggaagggac 1380

ctcgaatgtg ggaccccagc ccctctccag ctcgaaatcc ctccacagcc acggggacac 1440

cctgcaccta ttcccacggg acaggctgga cccagagact ctggacccgg ggcctcccct 1500

tgagtagaga cccgccctct gactgatgga cgccgctgac ctggggtcag acccgtgggc 1560

tggacccctg cccaccccgc aggaaccctg aggcctaggg gagctgttga gccttcagtg 1620

tctgcatgtg ggaagtgggc tccttcacct acctcacagg gctgttgtga ggggcgctgt 1680

gatgcggttc caaagcacag ggcttggcgc accccactgt gctctcaata aatgtgtttc 1740

ctgtcttaac aaaaaaaaaa aaaaaaaaaa aaaaaaaaaa aaaaa 1785

<210> 45
<211> 1653
<212> DNA
<213> Homo sapiens

<400> 45
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg gggaatgggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggtagg ggctttgcag agaccatcga 960

cgggctgact tgaccaagct tcttgaaagg cctgtctaca cttgctgtct tccttcctca 1020

cctccaattt cctcttcaac ccactgcttc ctgactcgct ctactccgtg gaagcacgct 1080

cacaaaggca cgtgggccgt ggcccggctg ggtcggctga agaactgcgg atggaagctg 1140

cggaagaggc cctgatgggg cccaccatcc cggacccaag tcttcttcct ggcgggcctc 1200

tcgtctcctt cctggtttgg gcggaagcca tcacctggat gcctacgtgg gaagggacct 1260

cgaatgtggg accccagccc ctctccagct cgaaatccct ccacagccac ggggacaccc 1320

tgcacctatt cccacgggac aggctggacc cagagactct ggacccgggg cctccccttg 1380

agtagagacc cgccctctga ctgatggacg ccgctgacct ggggtcagac ccgtgggctg 1440

gacccctgcc caccccgcag gaaccctgag gcctagggga gctgttgagc cttcagtgtc 1500

tgcatgtggg aagtgggctc cttcacctac ctcacagggc tgttgtgagg ggcgctgtga 1560

tgcggttcca aagcacaggg cttggcgcac cccactgtgc tctcaataaa tgtgtttcct 1620

gtcttaacaa aaaaaaaaaa aaaaaaaaaa aaa 1653

<210> 46
<211> 9701
<212> DNA
<213> Homo sapiens

<400> 46
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagccaag cttcttgaaa ggcctgtcta 960

cacttgctgt cttccttcct cacctccaat ttcctcttca acccactgct tcctgactcg 1020

ctctactccg tggaagcacg ctcacaaagg cacgtgggcc gtggcccggc tgggtcggct 1080

gaagaactgc ggatggaagc tgcggaagag gccctgatgg ggcccaccat cccggaccca 1140

agtcttcttc ctggcgggcc tctcgtctcc ttcctggttt gggcggaagc catcacctgg 1200

atgcctacgt gggaagggac ctcgaatgtg ggaccccagc ccctctccag ctcgaaatcg 1260

gcagactagg atggaagtgc cctgtgagct ggggggccct tcaaagggcc aaggagaaaa 1320

cgcaggccga gggaccagcc ttccaaatgg gcttcaagct ccaatgacct ccgctcgccc 1380

cctcgaaatg tctggaaaac ataatgggca gattttctgt cttcaaagtt tccggctaaa 1440

cctcttcaag ttctttattg tttgggactg agacactcag ccatgttaat gggtagtttc 1500

ttttgtattt gccttgaaag gccaaaatat ttttatattg ccacagacaa agccacctat 1560

ttaaaaatga actccatgtc cgtcgtttcc caccaggaga ctatgtacca tgtgtgtgtc 1620

tctatgtatt ctggggtctt gaaacaggtt tctcatgggg atggtcattc accacggtcc 1680

agaggggcag aacaggcggc gcttgccttg cccagggggc ctggggaacg tgggccctca 1740

tctcagatct gcccccagta tgtttaggac gcgagcccca gaaggatctg ggagtaaact 1800

taacattcac tgtgtctctg ctctgcatcc gccatttgtg tgtgtttctg gactgtgggc 1860

tgtgtgtacc ttggttggtg actcagtgag aagaagcagg aatgccaaag atactgtgaa 1920

tgttctgagt tttgttgctg ttgttgttga gaggttgttt cactggtatc tattgcattg 1980

tataataaat gaccagatga atgaatgagt gaagcaagag agaatgaata aacaagtaaa 2040

taggtaaaga agtaagcaag ccaggatgag agtgtgtgta cacaagacca tggttcatcc 2100

gctttgatgg ctaggcaatc aatatataaa tagaaaaaaa ccagtgaatc actaagtaat 2160

agggcaacac acaaagcgat atcaggtgat tatggactaa ggggtatgtg taactcaaat 2220

atatgcctct gacatttgac aatgaaaaag aacctaaatg aaagaaagaa tggatgtatg 2280

agtagtgaag tgcagaatga gacatagatt ttgaggcccg tcaaaatgaa aagatgcaag 2340

ttagggaaca agtgatcaaa agggagaagg gaaaggtttt ttttaaaaaa ccaaaacaac 2400

aaagaaaggt taaaaaaaaa aacagactag aggatgagta atgagtaact ctgtaaggag 2460

gaccatgtca gactattgta agctaagcat taggactgat acaaataata tatgctcctg 2520

gcatagaaaa ataaaccaca gagaacgagt tcaaagaata gcaaagaaag aaagaggacc 2580

cagtgggcga aagatgagag tgtactttta ccaaaagtta tctaagcctg agcacttgaa 2640

gtctgcacat aaataaataa atgacaaaag aaagaaaaaa aggccaaaaa gtctacattg 2700

cgtgtgtgga tggatgaatg agcagtggga gtgcagcgcc aggtgacaag atgttgtgag 2760

gggttttgag tcatccagtc ctgggcactg aggtctgtta gatgaaagga tatgagaaag 2820

gtaatattgg taaataaaga aataggaaac aatgtaacaa atgttaagta cagaaataca 2880

ttaatgggtg gtaaataaag atgtaaaaga aggcaatgcg atcgatggtg gcaaaagatc 2940

atcacagatt aagggctatg gctggtccac ttctagaaaa ccacaggctg tccattaaat 3000

aatgaacatc taagtgaaca agtcagtgag tacctaaata gacaaggatg aggtgaatga 3060

gaagacatgg ccccatgggt cctcctgatg agggtgttgg ggtcccccct gggcacccca 3120

gctgcatgaa aatgaaggac aggaggtatg gaaagctatg acagaagaga gaaaggaacg 3180

gtaaaaagaa ataacaacca aatggataaa tgggtagatc cacgagaaga gttaggctag 3240

gacttgtcat aagggcacct gactccacta atagaggaat aaatgcctaa taaaaagaga 3300

gcaagcagga aggaaggatg ctatgaatgc aggaaggaag taatgagtga gacgtggaac 3360

cgcacggcca aggatggacg tttgcgggtg gctttttgat gcgtacagcc aagccactcc 3420

atggcaatga gctccgaaga caaagtgcaa gagagaatga gtgagagagt gagagagaga 3480

gaaacaataa aaaatgggaa gaaatgtaaa aaggaagaaa ggaagagagg taatatatta 3540

aggaataaat acatgcatgc agatttaaga cagagccatg ctagaacagg aatgaaaggc 3600

tgtgtgaacc aagcagaccg cttaattggc accagtgctg ctggtatggt caatcaccta 3660

ctcaactaag gaacggctca aagcatacac atgggaggga ggagtggggc cacagagaga 3720

gggcccatta gttgcagatt acgatgtatc cagttaggtg cacctgcctt cgagaagtgt 3780

aaaaataagt atttacatag aaagaaagac tgaatggatg cacggtgaat gcatgaatga 3840

ttgaacgaca gaaaagattt gcattgaccg atgaggaggg cattgtagac agggatgagg 3900

gtcattgatc ctgggtgcag atctccaaaa gaatgacaga aagaaagagg gagtggtgga 3960

aagaaacaat aggatgggaa aaaatgaaaa tagaaaaaag gaagtgaaag agataataaa 4020

taattagatc aaataagttg atgaaagggg actggtttag cacaagccat ccacattaat 4080

tcaaacctgt ggctctgaag tttgtttttt aaatgaccac aagtgtaaga ctgaatgaaa 4140

gaataaatgc gtgcattcca taggatgcaa gaaaaggagt gaggaatggg aaaattggaa 4200

gaacgagaga gggagagatg taagaaaaga aaggaaaagt gaagtaggca tatgaaagaa 4260

aaggcacttc ttggacaagc actgaaatat aatgagacag ttttacccat taaatataat 4320

aaacagtaaa cgttgaggtt catcaataaa agcacagata cctgaataga ggagtgacct 4380

gaatagaatt cgttcagccg aacgaatgag aatggatgat tttcactatc ctgtgcactc 4440

aaggcccaaa agagaaagca agagaggaga gaatatggaa acgtatgaca ggatgtatat 4500

aagcaataca aacatattga atgaataaat aaagacataa atatgtggga gagtggacca 4560

cgcaaggaca aaaagaggag agaaggcagc aagaattatg actaattcaa aactgggttc 4620

ctgagatagt taaataaatc ctgcaccaaa tccccagggg gagaaattaa caaacaaaag 4680

acagccccac acggaccagt gtgcagaagg ctccaggaac cgcagattat ggttaatcca 4740

attctgtgca cctgaggtcc ataaataaaa gaataagtat tgaaatgaaa gaatgacaga 4800

aagaatgaat ggacacatga acgactgaat tagaaatgga aatgcctggc acagccagga 4860

aggagctgcc catgggattg tcattcatct cactctgggc acctgaggtc cataagcgtg 4920

aaaagaggca ggaagagaag tgtcagggag tcaaagatag agctaaggaa aggcaaaaat 4980

gaaactaaat gaaagcgaaa gggaaaataa agaaaaacca ataaaaaaga gaacgaatac 5040

gtgggtgtat ctgtaagagt aggatctgtt aggattagtc ataagactgt cagtaatcct 5100

gaagatggat gagataatcc aggcccaggt tcccaggggg agggaaaatg gagaaaatat 5160

aaaaagatgt gaaaaaggaa aaaggaaagg taataaacaa acaaccaaag tgataaatgg 5220

atagttaagg gaggttgtct gaacagggat tataattagt ttacatacat actccttaaa 5280

cagataaata cattacacct ttcaaagaat aaatgaaaaa tagagagaca tacctggctc 5340

caaaacaagg ctgtatcttc tgccactgta ataaaataga tgcaattgag gttcataaat 5400

aaaagaataa atacttaaac gtgaaaggtg actaaatgcg gggaagaaag attgcaaata 5460

aatacatggg ccaaagatgt ttggtttgcc catggagttt taattaaaaa aattaataag 5520

gaaaacaaat acccaaaata aggaagactg acaaatgagt gagtggatga gagagtgaat 5580

ggtgcttgac gtaggagcag tagtgcttta gggaccagca tgaaggtggt gaccgggagc 5640

cctgattcat gggattctgt ccacctgact ttataagaac caagaatggc tgggaatggt 5700

ggctcacgcc tgtaatccca gcactttggg aggccgaggt gggcggatca tgaggtcagg 5760

agtttgagac cagccagttt gagatcagcc tggccaacat ggtgaaattc catctctact 5820

aaaaaaatac aaaaattatg ggcgtggtgg cacctgtctg taatcctagc tactcgggag 5880

gctgagagag gagaattgct tgaacccggg aggtggaggt tgtagtgagc caagattgca 5940

ccactgcact ccagcctggg ctacagagca agacactatc ttagatcaaa aaagaaaaaa 6000

aaaaaaagaa ggagaaagaa ccagagaaac ataaggaaga gtgagaggaa gaaagaaaga 6060

tgcaatttgg gaagaaatga aaaagaaatg aataaagaat aaaataatgt aacggtcaat 6120

aaataggact tgtgaatgga ggcctttagg ccaaaggcta tgattaattt caagctatgt 6180

tactgaagtc cataaacaaa ggactcagat ctaaatggat gaacgaatga ctggaagaaa 6240

gggtggtagg aaggtaggaa gaaaggaagg agggaaaaag ggaagagagg aaggaacctt 6300

ctttccagtc ctgtgttcta gacagtggaa tgaagtggtc cccagggagg gtggctgtag 6360

gcatgtcatg tgcttgtcac atgcacttgc cctggcaggg aggagctggc tcaggaagac 6420

cctggtcttg gggtgctgtt gccctatctt ggctgtgtgg gccatttcac tgcatctgtc 6480

tcttcctcag tttccccatc tgtaaacctg gagtggcacc agctgcctac tagagttgat 6540

cttatgtgtc tctgttgatg gtaccccatc tatggcctgg ataggcagga agggcttgga 6600

ccctgagccc cgcagaaggt tgcatgaacg agtggtgtga agcctgttgg gtagcttggc 6660

cactcccgcg gcatgggtca cctgcacagg aggttttgcc caccaggggg cagcagaggg 6720

tcagggagca ataggccctg ggtggagcat gggccccgcc tgctgtgtgc caccctgggt 6780

gtggcaccta ctcacatcca ggggttggtg cagggaaagg ccagaaggtg gccaggcgca 6840

cctgagaagg gggacccaga agccccggga cccaggagcc ctgggcaagc caccagaaac 6900

cttgttcttg caactctctg cagtgtgccc aggccaccct ctggcctggt cttccatggg 6960

gcagggcgcc cacccttctc aactcaggtt tccctgggca gcaggtgcac ctcagcaccc 7020

ctggggttgc agaagtggtc cggggaccct ggcttccttg acatgccatc cccagagcct 7080

ggttcaaggc ctctctgtct tctcggctgt ttcacgacgt gttttgtaac ttggcgggat 7140

tgcgtttcgc tgtgtcgagg ttgtctcttc tctgactcgc cctccggggg actgccgggg 7200

taaatctgga gagttgctcg tgctgacagt cctcccccag ggcctccccg gttctgttga 7260

gtctcctttc tctgtagtgg aggaaatgtg tgtagttttg tgttgtgtgc ctgtgtttgt 7320

ctgtaaaagc aaggaccaaa gtctcccttg ttgacctctc aattcctatt tgggacatat 7380

aaaaacactg gattcttaac aagcgcccgg agcagtagga gcacagcttg gatggactca 7440

ggacttgtgg cagggagcac gtgggaggca ggggagtggg gtggggccag gccatctgga 7500

gtgggaggcg tcatgctcag agtgactctg tagacgctgg gtgggatggg gagtgcgggc 7560

gcaggcatgg atggggctgt tagctagtgt gatgcttgag gtctgagctg atggcagcaa 7620

agtggggtgc tcaggaatca aagctatggg gttatagaca ggatatgaag gagggaggga 7680

ggcaagaaga agggggtggt tcccacgctt ctagctccgg ccgagtggat ggcaacagca 7740

tttggaaggc ggaggacatg gaattcatgt gtcaggagcc accttccgag cctccagtac 7800

cacgtgtcag ggccacatga gctgggcctc gtgggcctga tgtggtgctg gggcctcagg 7860

ggtctgctct tcttctcttt cagaatctgg ggctccaggc tatgccttgg ctggactgag 7920

gtctgggggt gcacttatta tccctgggga cacctgctga agcttctccc tgacaagctg 7980

tgtcactgtt ggatgaggat ggggcgggag gggttcaggg cagaagaaga ccgggagggt 8040

ctttcaaaag aactcatgta cggctgttaa aaaaagtcag cagaggctca ggaagactta 8100

aagtgtgcag aaggcgggga agggagggcc cattgcatgc accaagagga aattggaagg 8160

aacaagcgac gttggctgct aggagagcct gctcccaaca tctaggggct gtcctgacgg 8220

gtcacagtgg gtcgaactga gccaatgaga gcagctctgg ggagacccac tggtgccctg 8280

gaggctgggt gggtttgggt tggatgaatt ctgtgtgtcc ttttggaaat gtggaggcca 8340

tgagggggga tcaggctctt tagggttttg acccttaaga gttttgtatc tgtaattcaa 8400

aggttcttta gttctgggat gctgagattc gggatagggt tcctaatggc acaaaagcca 8460

gagataaaac atccttcacg tgctccctac ccggttcttt ctgtaccaga cccacaaggt 8520

ccgagttggg atcctagtgc tcctgtctgg tcagggccta tctttatgtg ttcgttaaac 8580

ttttaacaat gagaattaat tctgtctctt gacattgtca tttgcatgct ccccacacac 8640

aaatcctttc ctggtgacac caggagctac aactctcctt ggcctcctct tgtgactccc 8700

aactccctcc ttgggaagct tggcctcagg acctctggga tagacaggcc acgaatcctg 8760

ctgtgtcccg ttgtgttcct aatataaatg gtgtggatgg cacttgacct agagcagtgg 8820

gaaatgcatg caccactcaa cattctgaca tgtcacccat tttacattct tacaggcata 8880

ctttttttaa aaaaagagtg tctattcttt aatgagcatc ccttctttaa aaaaacctaa 8940

ttgccattat tcaccacata cacttttttt tttttgtatc ctgcctcttc tatttaattt 9000

tctgtcatca acattttccc ttgttccatg aatcttcata acctcacttg ctgcgttgtg 9060

ccttgttgag tggctatggc atcattcaca gaaccattct gttattctta tgtataacca 9120

ccttttaaaa atattatgaa taatgccaca actaactgct taaaacaccc tttttttcat 9180

tcttaagaat tatgttcttc cacccagaaa ttatcattgc ttcactacag atcagtttcc 9240

cctgctagac tgtgagcccc ataagggcaa ggagcttatt gaattggcct ttgtatctct 9300

gatgcccaac atgttgtaga ctataaataa atgatgaatg agtggatgga agaatggagg 9360

aaggagcgag tgagtgagtg tttggctgat ggataagagg gtggaaggat aggcggaagg 9420

atggattggt gaatgaatga atgaatttcc tttggttaag tctcttgaaa gaaaggctat 9480

ggatctttgt atggatgttg aataatttca gtaagcttac agcattttac aatgttcagc 9540

aatgtatgac cacttaatta agatatggct agtttgtctc tgttataaag tacttttgca 9600

ttactttaac ttgcattgct ttaattacta atgatgggtg aacactttga cctatgtttg 9660

ttaacaaatt gtattttatc ttctgtgaaa aaaaaaaaaa a 9701

<210> 47
<211> 1731
<212> DNA
<213> Homo sapiens

<400> 47
gcagagaggg agcgcgcctt ggctcgctgg ccttggcggc ggctcctcag gagagctggg 60

gcgcccacga gaggatccct cacccgggtc tctcctcagg gatgacatca tccgtccacc 120

tccttgtctt caaggaccac ctcctctcca tgctgagctg ctgccaaggg gcctgctgcc 180

catctacacc tcacgagggc actaggagca cggtttcctg gatcccacca acatacaaag 240

cagccactca ctgaccccca ggaccaggat ggcaaaggat gaagaggacc ggaactgacc 300

agccagctgt ccctcttacc taaagactta aaccaatgcc ctagtgaggg ggcattgggc 360

attaagccct gacctttgct atgctcatac tttgactcta tgagtacttt cctataagtc 420

tttgcttgtg ttcacctgct agcaaactgg agtgtttccc tccccaaggg ggtgtcagtc 480

tttgtcgact gactctgtca tcacccttat gatgtcctga atggaaggat ccctttggga 540

aattctcagg agggggacct gggccaaggg cttggccagc atcctgctgg caactccaag 600

gccctgggtg ggcttctgga atgagcatgc tactgaatca ccaaaggcac gcccgacctc 660

tctgaagatc ttcctatcct tttctggggg aatggggtcg atgagagcaa cctcctaggg 720

ttgttgtgag aattaaatga gataaaagag gcctcaggca ggatctggca tagaggaggt 780

gatcagcaaa tgtttgttga aaaggtttga caggtcagtc ccttcccacc cctcttgctt 840

gtcttacttg tcttatttat tctccaacag cactccaggc agcccttgtc cacgggctct 900

ccttgcatca gccaagcttc ttgaaaggcc tgtctacact tgctgtcttc cttcctcacc 960

tccaatttcc tcttcaaccc actgcttcct gactcgctct actccgtgga agcacgctca 1020

caaaggttcc cagtgccccc gacaagcccc tgctggtgtc tccatctcct gccaagcatc 1080

ctccagtgcc tcctcctgtg ggcctggcct cagggctatg gacagactcc tgtcccatcc 1140

cagagacccc tcgtgatcgt gccctggcac gtgggccgtg gcccggctgg gtcggctgaa 1200

gaactgcgga tggaagctgc ggaagaggcc ctgatggggc ccaccatccc ggacccaagt 1260

cttcttcctg gcgggcctct cgtctccttc ctggtttggg cggaagccat cacctggatg 1320

cctacgtggg aagggacctc gaatgtggga ccccagcccc tctccagctc gaaatccctc 1380

cacagccacg gggacaccct gcacctattc ccacgggaca ggctggaccc agagactctg 1440

gacccggggc ctccccttga gtagagaccc gccctctgac tgatggacgc cgctgacctg 1500

gggtcagacc cgtgggctgg acccctgccc accccgcagg aaccctgagg cctaggggag 1560

ctgttgagcc ttcagtgtct gcatgtggga agtgggctcc ttcacctacc tcacagggct 1620

gttgtgaggg gcgctgtgat gcggttccaa agcacagggc ttggcgcacc ccactgtgct 1680

ctcaataaat gtgtttcctg tcttaacaaa aaaaaaaaaa aaaaaaaaaa a 1731

<210> 48
<211> 1687
<212> DNA
<213> Homo sapiens

<400> 48
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggtagg ggctttgcag agaccatcga 960

cgggctgact tgaggctaat ctcgggcctt gtcgaaggaa gaggctgcag acgttaatga 1020

ggttagctgc tggattccag tattcgtcgc ataaggatcc ttctttgtct gcgaaggaaa 1080

aacacactga ttatcataat gaggcacgtg ggccgtggcc cggctgggtc ggctgaagaa 1140

ctgcggatgg aagctgcgga agaggccctg atggggccca ccatcccgga cccaagtctt 1200

cttcctggcg ggcctctcgt ctccttcctg gtttgggcgg aagccatcac ctggatgcct 1260

acgtgggaag ggacctcgaa tgtgggaccc cagcccctct ccagctcgaa atccctccac 1320

agccacgggg acaccctgca cctattccca cgggacaggc tggacccaga gactctggac 1380

ccggggcctc cccttgagta gagacccgcc ctctgactga tggacgccgc tgacctgggg 1440

tcagacccgt gggctggacc cctgcccacc ccgcaggaac cctgaggcct aggggagctg 1500

ttgagccttc agtgtctgca tgtgggaagt gggctccttc acctacctca cagggctgtt 1560

gtgaggggcg ctgtgatgcg gttccaaagc acagggcttg gcgcacccca ctgtgctctc 1620

aataaatgtg tttcctgtct taacaaaaaa aaaaaaaaaa aaaaaaaaaa aaaaaaaaaa 1680

aaaaaaa 1687

<210> 49
<211> 1547
<212> DNA
<213> Homo sapiens

<400> 49
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg gggaatgggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggtagg ggctttgcag agaccatcga 960

cgggctgact tgagcacgtg ggccgtggcc cggctgggtc ggctgaagaa ctgcggatgg 1020

aagctgcgga agaggccctg atggggccca ccatcccgga cccaagtctt cttcctggcg 1080

ggcctctcgt ctccttcctg gtttgggcgg aagccatcac ctggatgcct acgtgggaag 1140

ggacctcgaa tgtgggaccc cagcccctct ccagctcgaa atccctccac agccacgggg 1200

acaccctgca cctattccca cgggacaggc tggacccaga gactctggac ccggggcctc 1260

cccttgagta gagacccgcc ctctgactga tggacgccgc tgacctgggg tcagacccgt 1320

gggctggacc cctgcccacc ccgcaggaac cctgaggcct aggggagctg ttgagccttc 1380

agtgtctgca tgtgggaagt gggctccttc acctacctca cagggctgtt gtgaggggcg 1440

ctgtgatgcg gttccaaagc acagggcttg gcgcacccca ctgtgctctc aataaatgtg 1500

tttcctgtct taacaaaaaa aaaaaaaaaa aaaaaaaaaa aaaaaaa 1547

<210> 50
<211> 1656
<212> DNA
<213> Homo sapiens

<400> 50
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagcaggt tcccagtgcc cccgacaagc 960

ccctgctggt gtctccatct cctgccaagc atcctccagt gcctcctcct gtgggcctgg 1020

cctcagggct atggacagac tcctgtccca tcccagagac ccctcgtgat cgtgccctgg 1080

cacgtgggcc gtggcccggc tgggtcggct gaagaactgc ggatggaagc tgcggaagag 1140

gccctgatgg ggcccaccat cccggaccca agtcttcttc ctggcgggcc tctcgtctcc 1200

ttcctggttt gggcggaagc catcacctgg atgcctacgt gggaagggac ctcgaatgtg 1260

ggaccccagc ccctctccag ctcgaaatcc ctccacagcc acggggacac cctgcaccta 1320

ttcccacggg acaggctgga cccagagact ctggacccgg ggcctcccct tgagtagaga 1380

cccgccctct gactgatgga cgccgctgac ctggggtcag acccgtgggc tggacccctg 1440

cccaccccgc aggaaccctg aggcctaggg gagctgttga gccttcagtg tctgcatgtg 1500

ggaagtgggc tccttcacct acctcacagg gctgttgtga ggggcgctgt gatgcggttc 1560

caaagcacag ggcttggcgc accccactgt gctctcaata aatgtgtttc ctgtcttaac 1620

aaaaaaaaaa aaaaaaaaaa aaaaaaaaaa aaaaaa 1656

<210> 51
<211> 1793
<212> DNA
<213> Homo sapiens

<400> 51
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggtagg ggctttgcag agaccatcga 960

cgggctgact tgaccaagct tcttgaaagg cctgtctaca cttgctgtct tccttcctca 1020

cctccaattt cctcttcaac ccactgcttc ctgactcgct ctactccgtg gaagcacgct 1080

cacaaagggc taatctcggg ccttgtcgaa ggaagaggct gcagacgtta atgaggttag 1140

ctgctggatt ccagtattcg tcgcataagg atccttcttt gtctgcgaag gaaaaacaca 1200

ctgattatca taatgaggca cgtgggccgt ggcccggctg ggtcggctga agaactgcgg 1260

atggaagctg cggaagaggc cctgatgggg cccaccatcc cggacccaag tcttcttcct 1320

ggcgggcctc tcgtctcctt cctggtttgg gcggaagcca tcacctggat gcctacgtgg 1380

gaagggacct cgaatgtggg accccagccc ctctccagct cgaaatccct ccacagccac 1440

ggggacaccc tgcacctatt cccacgggac aggctggacc cagagactct ggacccgggg 1500

cctccccttg agtagagacc cgccctctga ctgatggacg ccgctgacct ggggtcagac 1560

ccgtgggctg gacccctgcc caccccgcag gaaccctgag gcctagggga gctgttgagc 1620

cttcagtgtc tgcatgtggg aagtgggctc cttcacctac ctcacagggc tgttgtgagg 1680

ggcgctgtga tgcggttcca aagcacaggg cttggcgcac cccactgtgc tctcaataaa 1740

tgtgtttcct gtcttaacaa aaaaaaaaaa aaaaaaaaaa aaaaaaaaaa aaa 1793

<210> 52
<211> 1506
<212> DNA
<213> Homo sapiens

<400> 52
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg gggaatgggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggcacg tgggccgtgg cccggctggg 960

tcggctgaag aactgcggat ggaagctgcg gaagaggccc tgatggggcc caccatcccg 1020

gacccaagtc ttcttcctgg cgggcctctc gtctccttcc tggtttgggc ggaagccatc 1080

acctggatgc ctacgtggga agggacctcg aatgtgggac cccagcccct ctccagctcg 1140

aaatccctcc acagccacgg ggacaccctg cacctattcc cacgggacag gctggaccca 1200

gagactctgg acccggggcc tccccttgag tagagacccg ccctctgact gatggacgcc 1260

gctgacctgg ggtcagaccc gtgggctgga cccctgccca ccccgcagga accctgaggc 1320

ctaggggagc tgttgagcct tcagtgtctg catgtgggaa gtgggctcct tcacctacct 1380

cacagggctg ttgtgagggg cgctgtgatg cggttccaaa gcacagggct tggcgcaccc 1440

cactgtgctc tcaataaatg tgtttcctgt cttaacaaaa aaaaaaaaaa aaaaaaaaaa 1500

aaaaaa 1506

<210> 53
<211> 1687
<212> DNA
<213> Homo sapiens

<400> 53
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagcaggt tcccagtgcc cccgacaagc 960

ccctgctggt gtctccatct cctgccaagc atcctccagt gcctcctcct gtgggcctgg 1020

cctcagggct atggacagac tcctgtccca tcccagagac ccctcgtgat cgtgccctgt 1080

agtcacccca ctccaccgaa aagtaaactg caggcacgtg ggccgtggcc cggctgggtc 1140

ggctgaagaa ctgcggatgg aagctgcgga agaggccctg atggggccca ccatcccgga 1200

cccaagtctt cttcctggcg ggcctctcgt ctccttcctg gtttgggcgg aagccatcac 1260

ctggatgcct acgtgggaag ggacctcgaa tgtgggaccc cagcccctct ccagctcgaa 1320

atccctccac agccacgggg acaccctgca cctattccca cgggacaggc tggacccaga 1380

gactctggac ccggggcctc cccttgagta gagacccgcc ctctgactga tggacgccgc 1440

tgacctgggg tcagacccgt gggctggacc cctgcccacc ccgcaggaac cctgaggcct 1500

aggggagctg ttgagccttc agtgtctgca tgtgggaagt gggctccttc acctacctca 1560

cagggctgtt gtgaggggcg ctgtgatgcg gttccaaagc acagggcttg gcgcacccca 1620

ctgtgctctc aataaatgtg tttcctgtct taacaaaaaa aaaaaaaaaa aaaaaaaaaa 1680

aaaaaaa 1687

<210> 54
<211> 1621
<212> DNA
<213> Homo sapiens

<400> 54
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagggcta atctcgggcc ttgtcgaagg 960

aagaggctgc agacgttaat gaggttagct gctggattcc agtattcgtc gcataaggat 1020

ccttctttgt ctgcgaagga aaaacacact gattatcata atgaggcacg tgggccgtgg 1080

cccggctggg tcggctgaag aactgcggat ggaagctgcg gaagaggccc tgatggggcc 1140

caccatcccg gacccaagtc ttcttcctgg cgggcctctc gtctccttcc tggtttgggc 1200

ggaagccatc acctggatgc ctacgtggga agggacctcg aatgtgggac cccagcccct 1260

ctccagctcg aaatccctcc acagccacgg ggacaccctg cacctattcc cacgggacag 1320

gctggaccca gagactctgg acccggggcc tccccttgag tagagacccg ccctctgact 1380

gatggacgcc gctgacctgg ggtcagaccc gtgggctgga cccctgccca ccccgcagga 1440

accctgaggc ctaggggagc tgttgagcct tcagtgtctg catgtgggaa gtgggctcct 1500

tcacctacct cacagggctg ttgtgagggg cgctgtgatg cggttccaaa gcacagggct 1560

tggcgcaccc cactgtgctc tcaataaatg tgtttcctgt cttaacaaaa aaaaaaaaaa 1620

a 1621

<210> 55
<211> 1714
<212> DNA
<213> Homo sapiens

<400> 55
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcaggacga ataatttatg gtcagtgaaa 960

atccaggccc cagtgagatt cgagtgggct gtaaaatcga gagtcctgct cccacaggtt 1020

cccagtgccc ccgacaagcc cctgctggtg tctccatctc ctgccaagca tcctccagtg 1080

cctcctcctg tgggcctggc ctcagggcta tggacagact cctgtcccat cccagagacc 1140

cctcgtgatc gtgccctggc acgtgggccg tggcccggct gggtcggctg aagaactgcg 1200

gatggaagct gcggaagagg ccctgatggg gcccaccatc ccggacccaa gtcttcttcc 1260

tggcgggcct ctcgtctcct tcctggtttg ggcggaagcc atcacctgga tgcctacgtg 1320

ggaagggacc tcgaatgtgg gaccccagcc cctctccagc tcgaaatccc tccacagcca 1380

cggggacacc ctgcacctat tcccacggga caggctggac ccagagactc tggacccggg 1440

gcctcccctt gagtagagac ccgccctctg actgatggac gccgctgacc tggggtcaga 1500

cccgtgggct ggacccctgc ccaccccgca ggaaccctga ggcctagggg agctgttgag 1560

ccttcagtgt ctgcatgtgg gaagtgggct ccttcaccta cctcacaggg ctgttgtgag 1620

gggcgctgtg atgcggttcc aaagcacagg gcttggcgca ccccactgtg ctctcaataa 1680

atgtgtttcc tgtcttaaca aaaaaaaaaa aaaa 1714

<210> 56
<211> 1735
<212> DNA
<213> Homo sapiens

<400> 56
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagccaag cttcttgaaa ggcctgtcta 960

cacttgctgt cttccttcct cacctccaat ttcctcttca acccactgct tcctgactcg 1020

ctctactccg tggaagcacg ctcacaaagg gctaatctcg ggccttgtcg aaggaagagg 1080

ctgcagacgt taatgaggtt agctgctgga ttccagtatt cgtcgcataa ggatccttct 1140

ttgtctgcga aggaaaaaca cactgattat cataatgagg cacgtgggcc gtggcccggc 1200

tgggtcggct gaagaactgc ggatggaagc tgcggaagag gccctgatgg ggcccaccat 1260

cccggaccca agtcttcttc ctggcgggcc tctcgtctcc ttcctggttt gggcggaagc 1320

catcacctgg atgcctacgt gggaagggac ctcgaatgtg ggaccccagc ccctctccag 1380

ctcgaaatcc ctccacagcc acggggacac cctgcaccta ttcccacggg acaggctgga 1440

cccagagact ctggacccgg ggcctcccct tgagtagaga cccgccctct gactgatgga 1500

cgccgctgac ctggggtcag acccgtgggc tggacccctg cccaccccgc aggaaccctg 1560

aggcctaggg gagctgttga gccttcagtg tctgcatgtg ggaagtgggc tccttcacct 1620

acctcacagg gctgttgtga ggggcgctgt gatgcggttc caaagcacag ggcttggcgc 1680

accccactgt gctctcaata aatgtgtttc ctgtcttaac aaaaaaaaaa aaaaa 1735

<210> 57
<211> 1855
<212> DNA
<213> Homo sapiens

<400> 57
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagccaag cttcttgaaa ggcctgtcta 960

cacttgctgt cttccttcct cacctccaat ttcctcttca acccactgct tcctgactcg 1020

ctctactccg tggaagcacg ctcacaaagg gctaatctcg ggccttgtcg aaggaagagg 1080

ctgcagacgt taatgaggtt agctgctgga ttccagtatt cgtcgcataa ggatccttct 1140

ttgtctgcga aggaaaaaca cactgattat cataatgagt tcctgacctg gccatcccgg 1200

ggtgcccttg accagccccg tgtctcctca gggtgtccca gcaccagcct ggcacagagt 1260

ggggctcagt tagagtatgt gggatgttgg tttcgccagg cacgtgggcc gtggcccggc 1320

tgggtcggct gaagaactgc ggatggaagc tgcggaagag gccctgatgg ggcccaccat 1380

cccggaccca agtcttcttc ctggcgggcc tctcgtctcc ttcctggttt gggcggaagc 1440

catcacctgg atgcctacgt gggaagggac ctcgaatgtg ggaccccagc ccctctccag 1500

ctcgaaatcc ctccacagcc acggggacac cctgcaccta ttcccacggg acaggctgga 1560

cccagagact ctggacccgg ggcctcccct tgagtagaga cccgccctct gactgatgga 1620

cgccgctgac ctggggtcag acccgtgggc tggacccctg cccaccccgc aggaaccctg 1680

aggcctaggg gagctgttga gccttcagtg tctgcatgtg ggaagtgggc tccttcacct 1740

acctcacagg gctgttgtga ggggcgctgt gatgcggttc caaagcacag ggcttggcgc 1800

accccactgt gctctcaata aatgtgtttc ctgtcttaac aaaaaaaaaa aaaaa 1855

<210> 58
<211> 1595
<212> DNA
<213> Homo sapiens

<400> 58
agcccctagc gcagacggcg gagagcagag agggagcgcg ccttggctcg ctggccttgg 60

cggcggctcc tcaggagagc tggggcgccc acgagaggat ccctcacccg ggtctctcct 120

cagggatgac atcatccgtc cacctccttg tcttcaagga ccacctcctc tccatgctga 180

gctgctgcca aggggcctgc tgcccatcta cacctcacga gggcactagg agcacggttt 240

cctggatccc accaacatac aaagcagcca ctcactgacc cccaggacca ggatggcaaa 300

ggatgaagag gaccggaact gaccagccag ctgtccctct tacctaaaga cttaaaccaa 360

tgccctagtg agggggcatt gggcattaag ccctgacctt tgctatgctc atactttgac 420

tctatgagta ctttcctata agtctttgct tgtgttcacc tgctagcaaa ctggagtgtt 480

tccctcccca agggggtgtc agtctttgtc gactgactct gtcatcaccc ttatgatgtc 540

ctgaatggaa ggatcccttt gggaaattct caggaggggg acctgggcca agggcttggc 600

cagcatcctg ctggcaactc caaggccctg ggtgggcttc tggaatgagc atgctactga 660

atcaccaaag gcacgcccga cctctctgaa gatcttccta tccttttctg ggggaatggg 720

gtcgatgaga gcaacctcct agggttgttg tgagaattaa atgagataaa agaggcctca 780

ggcaggatct ggcatagagg aggtgatcag caaatgtttg ttgaaaaggt ttgacaggtc 840

agtcccttcc cacccctctt gcttgtctta cttgtcttat ttattctcca acagcactcc 900

aggcagccct tgtccacggg ctctccttgc atcagccaag cttcttgaaa ggcctgtcta 960

cacttgctgt cttccttcct cacctccaat ttcctcttca acccactgct tcctgactcg 1020

ctctactccg tggaagcacg ctcacaaagg cacgtgggcc gtggcccggc tgggtcggct 1080

gaagaactgc ggatggaagc tgcggaagag gccctgatgg ggcccaccat cccggaccca 1140

agtcttcttc ctggcgggcc tctcgtctcc ttcctggttt gggcggaagc catcacctgg 1200

atgcctacgt gggaagggac ctcgaatgtg ggaccccagc ccctctccag ctcgaaatcc 1260

ctccacagcc acggggacac cctgcaccta ttcccacggg acaggctgga cccagagact 1320

ctggacccgg ggcctcccct tgagtagaga cccgccctct gactgatgga cgccgctgac 1380

ctggggtcag acccgtgggc tggacccctg cccaccccgc aggaaccctg aggcctaggg 1440

gagctgttga gccttcagtg tctgcatgtg ggaagtgggc tccttcacct acctcacagg 1500

gctgttgtga ggggcgctgt gatgcggttc caaagcacag ggcttggcgc accccactgt 1560

gctctcaata aatgtgtttc ctgtcttaac aaaaa 1595

<210> 59
<211> 231
<212> DNA
<213> Homo sapiens

<400> 59
gcggcccggg aatctacgtc acccgaaaag cgactataaa cgccggcgcc tccgtcccca 60

gccgcggctc gggaatccac ccgaagagtg gctataaacg tccgcgcctc cattgcgctc 120

tcctcttcac ttaggacact ggtcctccca cgcctgacac cgacgtcgcc aggaccgcgg 180

ggttggggga acttggctgt cccacgtctt tcaaataaag ctgttttgtc t 231

## Description

### FIELD OF THE INVENTION

The present invention associates multiple long non-coding RNA (lncRNA) with key functions of endothelial cells. The lncRNA of the present invention are therefore useful as novel drug targets for the manufacturing of medicines for the treatment of cardiovascular diseases or pathological angiogenesis in context of proliferative diseases such as cancer. Modulation of the function or expression of the lncRNA of invention can induce or repress angiogenesis and vessel growth or repair in endothelial cells. Provided are further methods for the modulation of endothelial cell functions *in vitro*, for example, in the context of tissue engineering.

### DESCRIPTION

RNA sequencing revealed that the majority of the genome is transcribed, however, most transcripts do not encode for proteins. According to their size, these so called "non-coding RNAs" are divided in small non-coding RNAs (< 200 nucleotides) and long non-coding RNAs (lncRNAs; >200 nt) such as natural antisense transcripts (NATs), long intergenic non-coding RNAs (lincRNAs) and circular RNAs. Whereas the function and mechanism of distinct non-coding RNA species is well understood and clearly defined (e.g. miRNAs), lncRNAs exhibit various molecular functions, for example by acting as scaffold or guide for proteins / RNAs or as molecular sponges. Therefore, lncRNAs can interfere with gene expression and signaling pathways at various stages. Specifically, lncRNAs were shown to recruit chromatin modifying enzymes, to act as decoys for RNA and protein binding partners, and to modulate splicing and mRNA degradation. Whereas microRNAs are well established regulators of endothelial cell function, vessel growth and remodeling, the regulation and function of lncRNAs in the endothelium is poorly understood.

Long ncRNAs vary in length from several hundred bases to tens of kilobases and may be located separate from protein coding genes (long intergenic ncRNAs or lincRNAs), or reside near or within protein coding genes (Guttman et al. (2009) Nature 458:223-227; Katayama et al. (2005) Science 309:1564-1566). Recent evidence indicates that active enhancer elements may also be transcribed as lncRNAs (Kim et al. (2010) Nature 465:182-187; De Santa et al. (2010) PLoS Biol. 8:e1000384).

Several lncRNAs have been implicated in transcriptional regulation. For example, in the CCND1 (encoding cyclin D1) promoter, an ncRNA transcribed 2 kb upstream of CCND1 is induced by ionizing radiation and regulates transcription of CCND1 in cis by forming a ribo-nucleoprotein repressor complex (Wang et al. (2008) Nature 454:126-130). This ncRNA binds to and allosterically activates the RNA-binding protein TLS (translated in liposarcoma), which inhibits histone acetyltransferases, resulting in repression of CCND1 transcription. Another example is the antisense ncRNA CDKN2B-AS1 (also known as p15AS or ANRIL), which overlaps the p15 coding sequence. Expression of CDKN2B-AS is increased in human leukemias and inversely correlated with p15 expression (Pasmant et al. (2007) Cancer Res. 67:3963-3969; Yu et al. (2008) Nature 451:202-206). CDKN2B-AS1 can transcriptionally silence p15 directly as well as through induction of heterochromatin formation. Many well-studied lncRNAs, such as those involved in dosage compensation and imprinting, regulate gene expression in cis (Lee (2009) Genes Dev. 23:1831-1842). Other lncRNAs, such as HOTAIR and linc-p21 regulate the activity of distantly located genes in trans (Rinn et al. (2007) Cell 129:1311-1323; Gupta et al. (2010) Nature 464:1071-1076; and Huarte et al. (2010) Cell 142:409-419).

Laminar shear stress provides a very important atheroprotective force by modulating gene expression of endothelial cells. For example, the exposure of endothelial cells to laminar shear stress but not to oscillating shear stress reduces inflammatory activation and prevents induction of apoptosis. Like shear stress, a reduction of oxygen availability - hypoxia - critically influences endothelial cell function and additionally induces the angiogenic response which represents an essential mechanism upon ischemic injury or during wound healing. The regulation of angiogenic activity in endothelial cells is therefore a key event in the context of several diseases. After the discovery of vascular endothelial growth factor (VEGF) many studies focused on the control of angiogenesis by growth factors, however, the control of endothelial cell functions by regulatory RNAs or epigenetic mechanisms remained unknown.

A critical role for lncRNA in endothelial cells is known. Repression of MALAT1 inhibited proliferation of endothelial cells and reduced neonatal retina vascularization and pharmacological inhibition of MALAT1 by GapmeRs reduced blood flow recovery and capillary density after hindlimb ischemia (Michalik et al. Circ Res. 2014 Apr 25;114(9):1389-97).

ANRIL is a long non-coding RNA which is transcribed from the INK/ARF locus and was shown to be expressed in tissues and cell types that are affected by atherosclerosis such as primary coronary smooth muscle cells, vascular endothelial cells, and human monocyte-derived macrophage cells. ANRIL transcripts were directly correlated with the severity of atherosclerosis (Holdt, et al. Arterioscler. Thromb. Vasc. Biol. 2010, 30, 620-627).

The lnc-Ang362 has been shown to be co expressed with to microRNAs (miRs) that are correlated with cardiovascular events. In particular, these miRs play a critical role in smooth muscle cell proliferation and neointimal hyperplasia in response to vascular injury. Interestingly, knockdown of Lnc-Ang362 reduces the expression of these miRNAs as well as cell growth (Davis, B.N et al. J. Biol. Chem. 2009, 284, 3728-3738).

In view of the state of the art it was therefore an object of the present invention to provide novel options for the treatment of diseases that are associated with endothelial dysfunction such as angiogenesis or cardiovascular disorders. The present invention seeks to provide new drug targets for these diseases based on comprehensive deep sequencing approaches of the endothelial transcriptome in response to shear stress or hypoxia.

The above problem is solved in a first aspect by a compound for use in the treatment of a disease associated with endothelial cell dysfunction, wherein the compound is an agonist or is an antagonist of a long non-coding RNA (lncRNA) selected from the group consisting of lnc-KPNA2, lnc-SERTAD2, lnc-Hyp_up_1, lnc-Hyp_down_1, lnc-Meg3, lnc-RNF151, lnc-Hyp_up_3, lnc-Hyp_up_5 and lnc-GDF7. For a better understanding of the invention, a more detailed discussion is provided below regarding the identified lncRNAs and their diagnostic and therapeutic uses for diseases associated with endothelial cell dysfunction, and regenerative medicine.

In the context of the present invention the above mentioned lncRNA identified herein are defined by containing the following exon/intron structure. Some lncRNA are known to comprise more than one splice variant. In the following, if for example referring to the lnc-Hyp_up_1, all its splice variants as explained herein below shall be included.

lncHyp_down_1 comprises the following exon/introns: the sequences as shown in SEQ ID NO: 1, 2 and 3, preferably in 5' to 3' order: SEQ ID NO: 1, 2, 3.

lncHyp_up_1 comprises the following exons/instrons:
the sequences as shown in SEQ ID NO: 4 to 6, preferably in 5' to 3' order: SEQ ID NO: 4, 5, 6; or
the sequences as shown in SEQ ID NO: 7 to 9, preferably in 5' to 3' order: SEQ ID NO: 7, 8, 9;or
the sequences as shown in SEQ ID NO: 10 to 16, preferably in 5' to 3' order: SEQ ID NO: 10, 11, 12, 13, 14, 15, 16; or
the sequences as shown in SEQ ID NO: 17 to 19, preferably in 5' to 3' order: SEQ ID NO: 17, 18, 19.
   lncHyp_up_3 comprises the following exons/instrons:
the sequences as shown in SEQ ID NO: 20 to 22, preferably in 5' to 3' order: SEQ ID NO: 20, 21, 22; or
the sequences as shown in SEQ ID NO: 23 to 27, preferably in 5' to 3' order: SEQ ID NO: 23, 24, 25, 26, 27.

lncHyp_up_5 comprises the following exons/instrons:
the sequences as shown in SEQ ID NO: 28 to 32, preferably in 5' to 3' order: SEQ ID NO: 28, 29, 30, 31, 32; or
the sequences as shown in SEQ ID NO: 33 to 39, preferably in 5' to 3' order: SEQ ID NO: 33, 34, 35, 36, 37, 38, 39.

lnc-SERTAD2 comprises the sequence shown in SEQ ID NO: 40.

lnc-GDF7 comprises the sequence shown in SEQ ID NO: 41.

lnc-KPNA2 comprises the sequence shown in SEQ ID NO: 42.

lnc-Meg3 may be selected from a lnRNA comprising any of the sequence shown in SEQ ID NO: 43 to 58.

lnc-RNF151 comprises the sequence shown in SEQ ID NO: 59.

The sequences provided in the sequence listing as part of the description are shown as DNA sequences. However, the person of skill understands that when referring to the actual lncRNA molecule the thymine residues will correspond to uracil at the respective positions in the RNA.

The term "sequence identity" or "percent identical" as applied to nucleic acid molecules is the percentage of nucleic acid residues in a candidate nucleic acid molecule sequence that are identical with a subject nucleic acid molecule sequence, after aligning the sequences to achieve the maximum percent identity, and not considering any nucleic acid residue substitutions as part of the sequence identity. No gaps are introduced into the candidate nucleic acid sequence in order to achieve the best alignment.

When assessing the identity or complementarity of a first and second nucleic acid sequence wherein one sequence is a DNA sequence and the other is an RNA sequence, it must be borne in mind that RNA sequences comprise uracil whereas DNA sequences would comprise thymine as complementary base of adenine. Therefore, whenever in context of the present invention a RNA sequence is referred to any of the sequences in the sequence protocol, these sequences have to be converted into RNA by exchanging thymine with uracil. When assessing sequence identity, an uracil residue is considered to be identical to a thymine residue (because both bind adenine) and when assessing complementarity a uracil residue is considered to be complementary to/capable of forming hydrogen bonds with an adenine residue. In this respect, the present invention when referring to a nucleic acid which is RNA in accordance with the invention contains uracil bases instead of thymine bases, and *vice versa.* This difference is well recognized in the pertinent art and easily recognized by a person of skill.

Using next generation sequencing, the inventors identified several endothelial long non-coding RNAs (lncRNAs) which regulate endothelial cell function. Atheroprotective laminar flow increased expression of lnc-KPNA2, and lnc-SERTAD2, and down-regulated lnc-GDF7. Inhibition of lnc-KPNA2 and lnc-SERTAD2 reduced angiogenic sprouting suggesting that both lncRNAs exhibit a pro-angiogenic activity. Inhibition of lnc-KPNA2 significantly increased basal and TNFa-induced endothelial adhesion molecule expression whereas inhibition of lnc-SERTAD2 reduced endothelial VCAM1 expression. Lnc-KPNA2 increased expression of key enzymes of the mevalonate pathway indicating a critical role in cholesterol metabolism. In addition the inventors surprisingly show that hypoxia regulates the expression of various lncRNAs and found that the hypoxia-regulated lncRNA Hyp_up_1 is pro-angiogenic, whereas Hyp_down_1 and Meg3 are anti-angiogenic.

The term "endothelial cell dysfunction" refers to a physiological dysfunction of normal biochemical processes carried out by endothelial cells, the cells that line the inner surface of all blood vessels including arteries and veins. For example, endothelial cell dysfunction may result in an inability of blood vessels, such as arteries and arterioles, to dilate normally in response to an appropriate stimulus.

### Modulation of lncRNA function and expression

The present invention is based on the development of lncRNA as drug targets. Therefore, a central aspect of the invention pertains to the modulation of the expression or function of endothelial lncRNAs as disclosed herein, preferably in the context of a medical treatment. For means or compounds enhancing the expression or function of a lncRNA the present invention will refer to such means or compounds as "agonists" of a respective lncRNA. Alternatively, the expression and/or function of an lncRNA may be reduced or inhibited. In this case the present invention will refer to these mediators of the effect as "antagonists" of the respective lncRNA of the invention. Since lncRNA are RNA-molecules which mediate their biological activity either in the cellular cytoplasm or in the cell nucleus the person of skill can harness all known methods that intervene with the natural RNA metabolism.

In some embodiments an agonist of a lncRNA of the invention is selected from an lncRNA molecule of the invention or a homolog thereof. An lncRNA molecule is an RNA molecule corresponding to a lncRNA sequence as disclosed herein in any of the SEQ ID NO: 1 to 59 as defined herein above (the lncRNA sequences). A homolog in the context of the invention is a nucleic acid, preferably an RNA molecule, which is homologous to any of the lncRNA of the herein described invention, and preferably comprises a sequence of at least 60% sequence identity to any one of the lncRNA sequences shown in SEQ ID NO: 1 to 59 as defined herein above (lncRNA sequences). Further preferred homologs of the invention comprise a nucleic acid sequence that is at least 70%, 80%, 90%, 95%, 96%, 97%, 98%, or most preferably 99% identical to a sequence as shown in any of the SEQ ID NO: 1 to 59 (lncRNA sequences). Alternatively, the present invention provides expression constructs of lncRNAs as agonists of the invention. An lncRNA expression construct of the invention comprises preferably an expressible sequence of lncRNA of the invention, optionally of homologs thereof, operatively linked to a promoter sequence. Since expression constructs may be used both to express an agonist or an antagonist of an lncRNA of the invention a detailed description of expression constructs is provided herein below.

In order to impair lncRNA expression/function in accordance with the herein described invention the person of skill may choose any suitable methodology for inhibiting RNA expression. In particular preferred are antisense approaches, which apply sequence complementary nucleic acid polymers (antagonists) which mediate the inhibition or destruction of a target RNAs.

According to some embodiments, an lncRNA of the invention may be targeted using an inhibiting agent or therapeutic - an antagonist of the lncRNA - targeting strategy such as antisense RNA, RNA interference (RNAi), siRNA, esiRNA, shRNA, miRNA, decoys, RNA aptamers, small molecule inhibitors, RNA/DNA-binding proteins/peptides, GapmeRs, LNA molecules or other compounds with different formulations to inhibit one or more physiological actions effected by lncRNA. The antisense antagonists of the invention may either be directly administered or used, or alternatively, may be expressed using an expression construct, for example a construct expressing a miRNA having a sequence complementary to an lncRNA of the invention.

For the inhibition of the lnc-RNA of the invention, it is in certain embodiments preferred to use antisense oligonucleotides in order to impair the lnc-RNA expression or function. Antisense oligonucleotides (ASOs) are synthetic nucleic acids that bind to a complementary target and suppress function of that target. Typically ASOs are used to reduce or alter expression of RNA targets - lnc-RNA is one preferred example of an RNA that can be targeted by ASOs. As a general principle, ASOs can suppress RNA function via two different mechanisms of action: 1) by steric blocking, wherein the ASO tightly binds the target nucleic acid and inactivates that species, preventing its participation in cellular activities, or 2) by triggering degradation, wherein the ASO binds the target and leads to activation of a cellular nuclease that degrades the targeted nucleic acid species. One class of "target degrading". ASOs may be composed of several types of nucleic acids, such as RNA, DNA or PNA.

In order to enhance the half-life of an ASO, modifications of the nucleic acids can be introduced. It is in particular useful to protect the ASO from degradation by cellular endonucleases. One modification that gained widespread use comprised DNA modified with phosphorothioate groups (PS). PS modification of internucleotide linkages confers nuclease resistance, making the ASOs more stable both in serum and in cells. As an added benefit, the PS modification also increases binding of the ASO to serum proteins, such as albumin, which decreases the rate of renal excretion following intravenous injection, thereby improving pharmacokinetics and improving functional performance. Therefore, PS modified ASOs are encompassed by the present invention.

Further modifications target the 3 '-end of an ASO molecule, for example "Gapmer" compounds having 2'-methoxyethylriboses (MOE's) providing 2'-modified "wings" at the 3' and 5' ends flanking a central 2'-deoxy gap region. ASO modifications that improve both binding affinity and nuclease resistance typically are modified nucleosides including locked nucleic acids (LNA), wherein a methyl bridge connects the 2'- oxygen and the 4'-carbon, locking the ribose in an A-form conformation; variations of LNA are also available, such as ethylene-bridged nucleic acids (ENA) that contain an additional methyl group, amino-LNA and thio-LNA. Additionally, other 2'-modifications, such as 2'-0- methoxyethyl (MOE) or 2'-fluoro (2'-F), can also be incorporated into ASOs. Such exemplary modifications are comprised by the present invention.

In the context of the present invention this means that the term "antisense oligonucleotide" pertains to a nucleotide sequence that is complementary to at least a portion of a target lnc-RNA sequence of the invention. The term "oligonucleotide" refers to an oligomer or polymer of nucleotide or nucleoside monomers consisting of naturally occurring bases, sugars, and intersugar (backbone) linkages. The term also includes modified or substituted oligomers comprising non-naturally occurring monomers or portions thereof, which function similarly. Such modified or substituted oligonucleotides may be preferred over naturally occurring forms because of properties such as enhanced cellular uptake, or increased stability in the presence of nucleases as already mentioned above. The term also includes chimeric oligonucleotides which contain two or more chemically distinct regions. For example, chimeric oligonucleotides may contain at least one region of modified nucleotides that confer beneficial properties (e.g. increased nuclease resistance, increased uptake into cells) as well as the antisense binding region. In addition, two or more antisense oligonucleotides may be linked to form a chimeric oligonucleotide.

The antisense oligonucleotides of the present invention may be ribonucleic or deoxyribonucleic acids and may contain naturally occurring bases including adenine, guanine, cytosine, thymidine and uracil. The oligonucleotides may also contain modified bases such as xanthine, hypoxanthine, 2-aminoadenine, 6-methyl, 2-propyl and other alkyl adenines, 5-halo uracil, 5-halo cytosine, 6-aza thymine, pseudo uracil, 4-thiouracil, 8-halo adenine, 8-aminoadenine, 8-thiol adenine, 8-thiolalkyl adenines, 8-hydroxyl adenine and other 8-substituted adenines, 8-halo guanines, 8-amino guanine, 8-thiol guanine, 8-thiolalkyl guanines, 8-hydrodyl guanine and other 8-substituted guanines, other aza and deaza uracils, thymidines, cytosines, adenines, or guanines, 5-tri-fluoromethyl uracil and 5-trifluoro cytosine.

Other antisense oligonucleotides of the invention may contain modified phosphorous, oxygen heteroatoms in the phosphate backbone, short chain alkyl or cycloalkyl intersugar linkages or short chain heteroatomic or heterocyclic intersugar linkages. For example, the antisense oligonucleotides may contain phosphorothioates, phosphotriesters, methyl phosphonates and phosphorodithioates. In addition, the antisense oligonucleotides may contain a combination of linkages, for example, phosphorothioate bonds may link only the four to six 3'-terminal bases, may link all the nucleotides or may link only 1 pair of bases.

The antisense oligonucleotides of the invention may also comprise nucleotide analogs that may be better suited as therapeutic or experimental reagents. An example of an oligonucleotide analogue is a peptide nucleic acid (PNA) in which the deoxribose (or ribose) phosphate backbone in the DNA (or RNA), is replaced with a polymide backbone which is similar to that found in peptides. PNA analogues have been shown to be resistant to degradation by enzymes and to have extended lives in vivo and in vitro. PNAs also form stronger bonds with a complementary DNA sequence due to the lack of charge repulsion between the PNA strand and the DNA strand. Other oligonucleotide analogues may contain nucleotides having polymer backbones, cyclic backbones, or acyclic backbones. For example, the nucleotides may have morpholino backbone structures. Oligonucleotide analogues may also contain groups such as reporter groups, protective groups and groups for improving the pharmacokinetic properties of the oligonucleotide. Antisense oligonucleotides may also incorporate sugar mimetics as will be appreciated by one of skill in the art.

Antisense nucleic acid molecules may be constructed using chemical synthesis and enzymatic ligation reactions using procedures known in the art based on a given lnc-RNA sequence such as those provided herein. The antisense nucleic acid molecules of the invention, or fragments thereof, may be chemically synthesized using naturally occurring nucleotides or variously modified nucleotides designed to increase the biological stability of the molecules or to increase the physical stability of the duplex formed with mRNA or the native gene, e.g. phosphorothioate derivatives and acridine substituted nucleotides. The antisense sequences may also be produced biologically. In this case, an antisense encoding nucleic acid is incorporated within an expression vector that is then introduced into cells in the form of a recombinant plasmid, phagemid or attenuated virus in which antisense sequences are produced under the control of a high efficiency regulatory region, the activity of which may be determined by the cell type into which the vector is introduced.

In another embodiment, siRNA technology may be applied to inhibit expression of a lnc-RNA of the invention. Application of nucleic acid fragments such as siRNA fragments that correspond with regions in lnc-RNA gene and which selectively target a lnc-RNA may be used to block lnc-RNA expression or function.

SiRNA, small interfering RNA molecules, corresponding to a region in the lnc-RNA sequence are made using well-established methods of nucleic acid syntheses as outlined above with respect to antisense oligonucleotides. Since the structure of target lnc-RNAs is known, fragments of RNA/DNA that correspond therewith can readily be made. The effectiveness of selected siRNA to impair lnc-RNA function or expression, for example via targeted degradation, can be confirmed using a lncRNA-expressing cell line. Briefly, selected siRNA may be incubated with a lncRNA-expressing cell line under appropriate growth conditions. Following a sufficient reaction time, i.e. for the siRNA to bind with lnc-RNA to result in decreased levels of the lnc-RNA, the reaction mixture is tested to determine if such a decrease has occurred, for example via quantitative PCR, northern blotting etc.

Antisense oligonucleotides in accordance with the invention may comprise at least one modification that is incorporated at the terminal end of an antisense oligonucleotide, or between two bases of the antisense oligonucleotide, wherein the modification increases binding affinity and nuclease resistance of the antisense oligonucleotide. In one embodiment, the antisense oligonucleotide comprises at least one modification that is located within three bases of a terminal nucleotide. In another embodiment, the antisense oligonucleotide comprises at least one modification that is located between a terminal base and a penultimate base of either the 3'- or the 5 '-end of the oligonucleotide. In another embodiment, the antisense oligonucleotide comprises a modification at a terminal end of the oligonucleotide. In a further embodiment, the antisense oligonucleotide comprises a modification at the terminal end or between the terminal base and the penultimate base of both the 3'- and the 5'- ends of the antisense oligonucleotide. In yet a further embodiment, the oligonucleotide contains a non-base modifier at a terminal end or between the terminal base and the penultimate base at the 5 '-end and at the 3 '-end.

### Pathological Angiogenesis

In some embodiments of the present invention it is preferred that the disease associated with endothelial cell dysfunction is pathological angiogenesis. In this embodiment the compound for use in the treatment of pathological angiogenesis is
a. An antagonist of a long non-coding RNA (lncRNA) selected from the group consisting of lnc-KPNA2, lnc-SERTAD2, and lncHyp_up_1, or
b. An agonist of a lncRNA selected from lncHyp_down_1 and lncMeg3.

The term "pathological angiogenesis" refers to the excessive formation and growth of blood vessels during the maintenance and the progression of several disease states. Examples where pathological angiogenesis can occur are blood vessels (atherosclerosis, hemangioma, hemangioendothelioma), bone and joints (rheumatoid arthritis, synovitis, bone and cartilage destruction, osteomyelitis, pannus growth, osteophyte formation, neoplasms and metastasis), skin (warts, pyogenic granulomas, hair growth, Kaposi's sarcoma, scar keloids, allergic oedema, neoplasms), liver, kidney, lung, ear and other epithelia (inflammatory and infectious processes (including hepatitis, glomerulonephritis, pneumonia), asthma, nasal polyps, otitis, transplantation, liver regeneration, neoplasms and metastasis), uterus, ovary and placenta (dysfunctional uterine bleeding (due to intrauterine contraceptive devices), follicular cyst formation, ovarian hyperstimulation syndrome, endometriosis, neoplasms), brain, nerves and eye (retinopathy of prematurity, diabetic retinopathy, choroidal and other intraocular disorders, leukomalacia, neoplasms and metastasis), heart and skeletal muscle due to work overload, adipose tissue (obesity), endocrine organs (thyroiditis, thyroid enlargement, pancreas transplantation), hematopoiesis (AIDS (Kaposi), hematologic malignancies (leukemias, etc.), tumour induced new blood vessels. The pathological angiogenesis may occur in connection with a proliferative disorder, most preferably in connection with a cancer disease. A cancer may be selected from the group consisting of liver cancer, lung cancer, breast cancer, colorectal cancer, stomach cancer and melanoma, most preferably where-in the cancer is solid cancer, even more preferably a metastatic solid cancer.

### Cardiovascular Diseases

In other preferred embodiments of the present invention the compound of the invention is for use in the treatment of a cardiovascular disease. In this embodiment the compound is
a. An agonist of a long non-coding RNA (lncRNA) selected from the group consisting of lnc-KPNA2, lnc-SERTAD2, and lncHyp_up_1, or
b. An antagonist of a lncRNA selected from lncHyp_down_1 and lncMeg3.

A cardiovascular disease in context of the present invention may be a disease associated with a pathological repressed endothelial cell repair, cell growth and/or cell division or is a disease treatable by improving endothelial cell repair, cell growth and/or cell division. Generally, the term "cardiovascular disease," as used herein, is intended to refer to all pathological states leading to a narrowing and/or occlusion of blood vessels throughout the body. In particular, the term "cardiovascular disease" refers to conditions including atherosclerosis, thrombosis and other related pathological states, especially within arteries of the heart and brain. Accordingly, the term "cardiovascular disease" encompasses, without limitation, various types of heart disease, as well as Alzheimer's disease and vascular dimension.

In preferred embodiments of the invention the cardiovascular disease is selected from the group consisting of acute coronary syndrome, acute lung injury (ALI), acute myocardial infarction (AMI), acute respiratory distress syndrome (ARDS), arterial occlusive disease, arteriosclerosis, articular cartilage defect, aseptic systemic inflammation, atherosclerot-ic cardiovascular disease, autoimmune disease, bone fracture, bone fracture, brain edema, brain hypoperfusion, Buerger's disease, burns, cancer, cardiovascular disease, cartilage damage, cerebral infarct, cerebral ischemia, cerebral stroke, cerebrovascular disease, chemotherapy-induced neuropathy, chronic infection, chronic mesenteric is-chemia, claudication, congestive heart failure, connective tissue damage, contusion, coronary artery disease (CAD), critical limb ischemia (CLI), Crohn's disease, deep vein thrombosis, deep wound, delayed ulcer healing, delayed wound-healing, diabetes (type I and type II), diabetic neuropathy, diabetes induced ischemia, disseminated in-travascular coagulation (DIC), embolic brain ischemia, frostbite, graft-versus-host dis-ease, hereditary hemorrhagic telengiectasiaischemic vascular disease, hyperoxic injury, hypoxia, inflammation, inflammatory bowel disease, inflammatory disease, injured tendons, intermittent claudication, intestinal ischemia, ischemia, ischemic brain disease, ischemic heart disease, ischemic peripheral vascular disease, ischemic placenta, ischemic renal disease, ischemic vascular disease, ischemic-reperfusion injury, laceration, left main coronary artery disease, limb ischemia, lower extremity ischemia, myocardial infarction, myocardial ischemia, organ ischemia, osteoarthritis, osteoporosis, osteosar-coma, Parkinson's disease, peripheral arterial disease (PAD), peripheral artery disease, peripheral ischemia, peripheral neuropathy, peripheral vascular disease, pre-cancer, pulmonary edema, pulmonary embolism, remodeling disorder, renal ischemia, retinal ischemia, retinopathy, sepsis, skin ulcers, solid organ transplantation, spinal cord injury, stroke, subchondral-bone cyst, thrombosis, thrombotic brain ischemia, tissue ischemia, transient ischemic attack (TIA), traumatic brain injury, ulcerative colitis, vascular dis-ease of the kidney, vascular inflammatory conditions, von Hippel-Lindau syndrome, or wounds to tissues or organs.

### Treatments

The agonists or antagonists as described herein above are useful in the treatment of the various diseases mentioned above. Therefore the present invention provides the use of the compounds of the invention in a curative or prophylactic medical treatment involving the administration of a therapeutically effective amount of the compound to a subject in need of such a treatment.

The agonists or antagonists described may be used alone or in combination with other methods for treating of the various diseases associated with endothelial cell dysfunction. For example if a subject has been diagnosed with ischemia, the one or more agents described above may be combined with administration of a therapeutically effective amount of a compound that is therapeutically active for the treatment of an ischemic event.

The term "effective amount" as used herein refers to an amount of a compound that produces a desired effect. For example, a population of cells may be contacted with an effective amount of a compound to study its effect *in vitro* (e.g., cell culture) or to produce a desired therapeutic effect *ex vivo* or *in vitro*. An effective amount of a compound may be used to produce a therapeutic effect in a subject, such as preventing or treating a target condition, alleviating symptoms associated with the condition, or producing a desired physiological effect. In such a case, the effective amount of a compound is a "therapeutically effective amount," "therapeutically effective concentration" or "therapeutically effective dose." The precise effective amount or therapeutically effective amount is an amount of the composition that will yield the most effective results in terms of efficacy of treatment in a given subject or population of cells. This amount will vary depending upon a variety of factors, including but not limited to the characteristics of the compound (including activity, pharmacokinetics, pharmacodynamics, and bioavailability), the physiological condition of the subject (including age, sex, disease type and stage, general physical condition, responsiveness to a given dosage, and type of medication) or cells, the nature of the pharmaceutically acceptable carrier or carriers in the formulation, and the route of administration. Further an effective or therapeutically effective amount may vary depending on whether the compound is administered alone or in combination with another compound, drug, therapy or other therapeutic method or modality. One skilled in the clinical and pharmacological arts will be able to determine an effective amount or therapeutically effective amount through routine experimentation, namely by monitoring a cell's or subject's response to administration of a compound and adjusting the dosage accordingly. For additional guidance, see Remington: The Science and Practice of Pharmacy, 21st Edition, Univ. of Sciences in Philadelphia (USIP), Lippincott Williams & Wilkins, Philadelphia, Pa., 2005, which is hereby incorporated by reference as if fully set forth herein.

The term "in combination" or "in combination with," as used herein, means in the course of treating the same disease in the same patient using two or more agents, drugs, treatment regimens, treatment modalities or a combination thereof, in any order. This includes simultaneous administration, as well as in a temporally spaced order of up to several days apart. Such combination treatment may also include more than a single administration of any one or more of the agents, drugs, treatment regimens or treatment modalities. Further, the administration of the two or more agents, drugs, treatment regimens, treatment modalities or a combination thereof may be by the same or different routes of administration.

The term "subject" as used herein means a human or other mammal. In some embodiments, the subject may be a patient suffering or in danger of suffering from a disease as disclosed herein.

### Expression constructs

Aspects of the present invention relate to various vehicles comprising the nucleic acid molecules, preferably the antisense or lncRNA molecules, of the present invention. By vehicle is understood an agent with which genetic material can be transferred. Herein such vehicles are exemplified as nucleic acid constructs, vectors, and delivery vehicles such as viruses and cells.

By nucleic acid construct or expression construct is understood a genetically engineered nucleic acid. The nucleic acid construct may be a non-replicating and linear nucleic acid, a circular expression vector, an autonomously replicating plasmid or viral expression vector. A nucleic acid construct may comprise several elements such as, but not limited to genes or fragments of same, promoters, enhancers, terminators, poly-A tails, linkers, markers and host homologous sequences for integration. Methods for engineering nucleic acid constructs are well known in the art (see, e.g., Molecular Cloning: A Laboratory Manual, Sambrook et al., eds., Cold Spring Harbor Laboratory, 2nd Edition, Cold Spring Harbor, N.Y., 1989). Furthermore, the present invention provides modified nucleic acids, in particular chemically modified RNA (modRNA) that can be used directly for the delivery of an lnc-RNA sequence of the invention ( Zangi L. et al, "Modified mRNA directs the fate of heart progenitor cells and induces vascular regeneration after myocardial infarction" 2013, Nat Biotechnol). Such modified RNA may be produced by use of 3'-O-Me-m7G(5')ppp(5')G cap analogs and is described in Warren L, Manos PD, Ahfeldt T, et al. Highly efficient reprogramming to pluripotency and directed differentiation of human cells with synthetic modified mRNA. Cell Stem Cell. 2011;7:618-630.

Several nucleic acid molecules may be encoded within the same construct and may be linked by an operative linker. By the term operative linker is understood to refer to a sequence of nucleotides that connects two parts of a nucleic acid construct in a manner securing the expression of the encoded nucleic acids via the construct.

The term promoter will be used here to refer to a group of transcriptional control modules that are clustered around the initiation site for RNA polymerase. The best known example of this is the TATA box, but in some promoters lacking a TATA box, such as the promoter for the mammalian terminal deoxynucleotidyl transferase gene and the promoter for the SV 40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. Depending on the promoter, it appears that individual elements can function either cooperatively or independently to activate transcription. Any promoter that can direct transcription initiation of the sequences encoded by the nucleic acid construct may be used in the invention.

An aspect of the present invention comprises the nucleic acid construct wherein the sequence of at least one nucleic acid molecule is preceded by a promoter enabling expression of at least one nucleic acid molecule.

It is a further aspect that the promoter is selected from the group of constitutive promoters, inducible promoters, organism specific promoters, tissue specific promoters and cell type specific promoters. Examples of promoters include, but are not limited to: constitutive promoters such as: simian virus 40 (SV40) early promoter, a mouse mammary tumour virus promoter, a human immunodeficiency virus long terminal repeat promoter, a Moloney virus promoter, an avian leukaemia virus promoter, an Epstein-Barr virus immediate early promoter, a Rous sarcoma virus (RSV) promoter, a human actin promoter, a human myosin promoter, a human haemoglobin promoter, cytomegalovirus (CMV) promoter and a human muscle creatine promoter, inducible promoters such as: a metallothionine promoter, a glucocorticoid promoter, a progesterone promoter, and a tetracycline promoter (tet-on or tet-off), tissue specific promoters such as: HER-2 promoter and PSA associated promoter.

### Vectors

An aspect of the present invention comprises the nucleic acid construct as described in any of the above, comprised within a delivery vehicle referred to as vector. A delivery vehicle is an entity whereby a nucleotide sequence can be transported from at least one media to another. Delivery vehicles are generally used for expression of the sequences encoded within the nucleic acid construct and/or for the intracellular delivery of the construct. It is within the scope of the present invention that the delivery vehicle is a vehicle selected from the group of: RNA based vehicles, DNA based vehicles/vectors, lipid based vehicles, virally based vehicles and cell based vehicles. Examples of such delivery vehicles include, but are not limited to: biodegradable polymer microspheres, lipid based formulations such as liposome carriers, coating the construct onto colloidal gold particles, lipopolysaccharides, polypeptides, polysaccharides, pegylation of viral vehicles.

A preferred embodiment of the present invention comprises a virus as a delivery vehicle, where the virus is selected from the non-exhaustive group of: adenoviruses, retroviruses, lentiviruses, adeno-associated viruses, herpesviruses, vaccinia viruses, foamy viruses, cytomeg-aloviruses, Semliki forest virus, poxviruses, RNA virus vector and DNA virus vector. Such viral vectors are well known in the art.

Another aspect of the present invention then pertains to a method for modulating endothelial cell function comprising contacting an endothelial cell with an agonist or antagonist of a lncRNA selected from the group consisting of lnc-KPNA2, lnc-SERTAD2, lncHyp_up_1, lnc-Hyp_down_1 and lnc-Meg3. The endothelial cell function may be selected from endothelial cell repair, endothelial cell growth and/or endothelial cell division.

Another aspect of the invention further pertains to a method for the generation of vascular tissue material, the method comprising the steps of contacting an endothelial cell with an agonist of a lncRNA selected from the group consisting of lnc-KPNA2, lnc-SERTAD2, and lncHyp_up_1, or an antagonist of a lncRNA selected from lncHyp_down_1 and lncMeg3. The vascular tissue material is preferably a blood vessel.

It is preferred that the method of the invention is an *ex-vivo* or *in-vitro* method.

In this aspect the lncRNA agonist or antagonist is preferably a compound as described herein above.

### Pharmaceutical compositions

Yet another aspect of the invention relates to a compositions, pharmaceutical compositions and kits comprising an agonist or antagonist of a long non-coding RNA (lncRNA) selected from the group consisting of lnc-KPNA2, lnc-SERTAD2, lncHyp_up_1, lnc-Hyp_down_1, lnc-Meg3, lncRNF151, Hyp_up_3, Hyp_up_5 and lncGDF7, in accordance with the afore described various aspects of the invention. In one embodiment, the composition comprises a compound, combination or composition as described herein, optionally together with a pharmaceutically acceptable carrier.

As used herein the language "pharmaceutically acceptable carrier" is intended to include any and all solvents, solubilizers, fillers, stabilizers, binders, absorbents, bases, buffering agents, lubricants, controlled release vehicles, nanoparticles, liposomes, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions. In certain embodiments, the pharmaceutically acceptable carrier comprises serum albumin.

The pharmaceutical composition of the invention is formulated to be compatible with its intended route of administration. Examples of routes of administration include parenteral, e.g., intrathecal, intra-arterial, intravenous, intradermal, subcutaneous, oral, transdermal (topical) and transmucosal administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injection include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phos-phate buffered saline (PBS). In all cases, the injectable composition should be sterile and should be fluid to the extent that easy syringability exists. It must be stable under the condi-tions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the inject-able compositions can be brought about by including in the composition an agent which delays absorption, for example, aluminum monostearate and gelatin.

Sterile injectable solutions can be prepared by incorporating the active compound (e.g., a neuregulin) in the required amount in an appropriate solvent with one or a combination of ingredi-ents enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active in-gredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or or-ange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions are formulated into ointments, salves, gels, or creams as generally known in the art.

In certain embodiments, the pharmaceutical composition is formulated for sustained or controlled release of the active ingredient. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from e.g. Alza Corporation and Nova Pharmaceuticals, Inc. Liposomal suspensions (including liposomes targeted to infected cells with monoclonal antibodies to viral antigens) or nanoparticles, including those prepared with poly(dl-lactide-co-glycolide), can also be used as pharmaceutically acceptable carriers. These can be prepared according to methods known to those skilled in the art.

It is especially advantageous to formulate oral or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser together with instructions for administration.

Finally provided is the use of an agonist or antagonist of a long non-coding RNA (lncRNA) selected from the group consisting of lnc-KPNA2, lnc-SERTAD2, lncHyp_up_1, lnc-Hyp_down_1, lnc-Meg3, lncRNF151, Hyp_up_3, Hyp_up_5 and lncGDF7, in the modulation of endothelial cell function.

The present invention will now be further described in the following examples with reference to the accompanying figures and sequences, nevertheless, without being limited thereto. For the purposes of the present invention, all references as cited herein are incorporated by reference in their entireties. In the Figures:

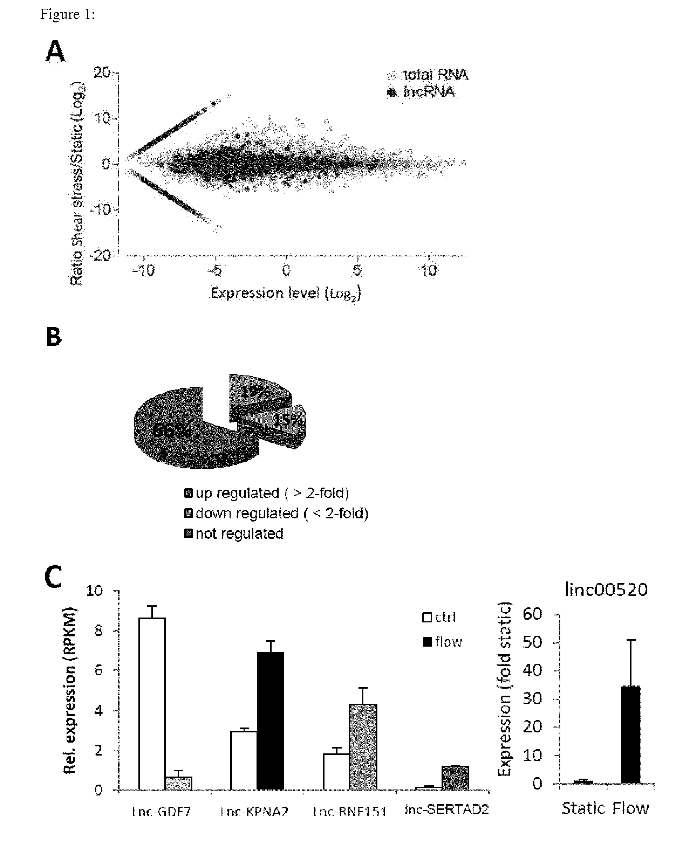

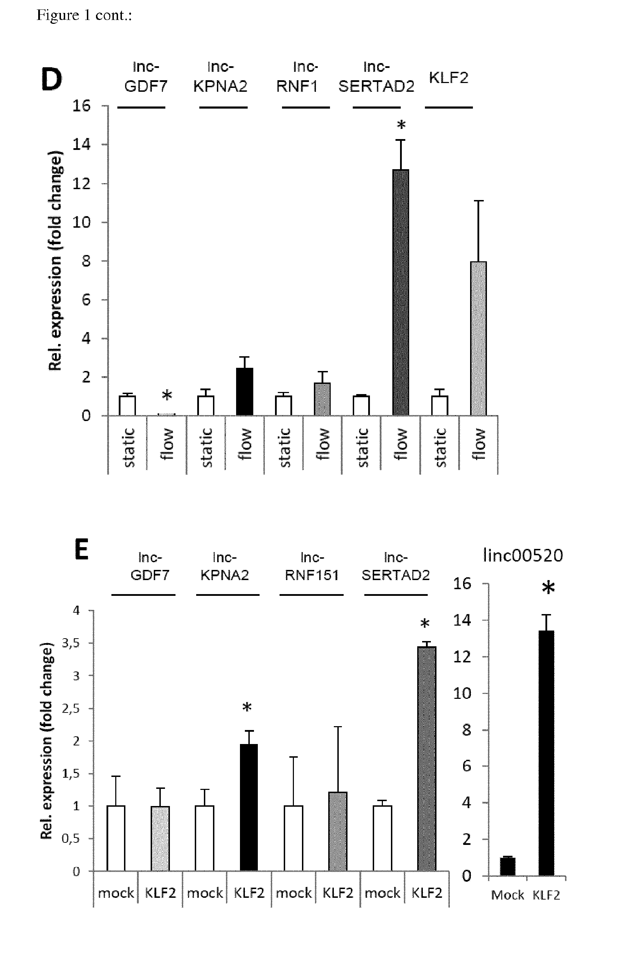

- **Figure 1:**: Regulation of lncRNAs by flow and KLF2. A/B) Expression of coding and non-coding RNAs after exposure of endothelial cells to laminar flow (12 dynes/cm²). C) Relative expression of selected flow-regulated lncRNAs comparing laminar flow and static control conditions. D) qRT-PCR confirming flow-dependent regulation of selected candidates. KLF2 was measured to confirm shear stress stimulation. E) KLF2-dependent regulation of lncRNA candidates. *p<0.05 versus static control.

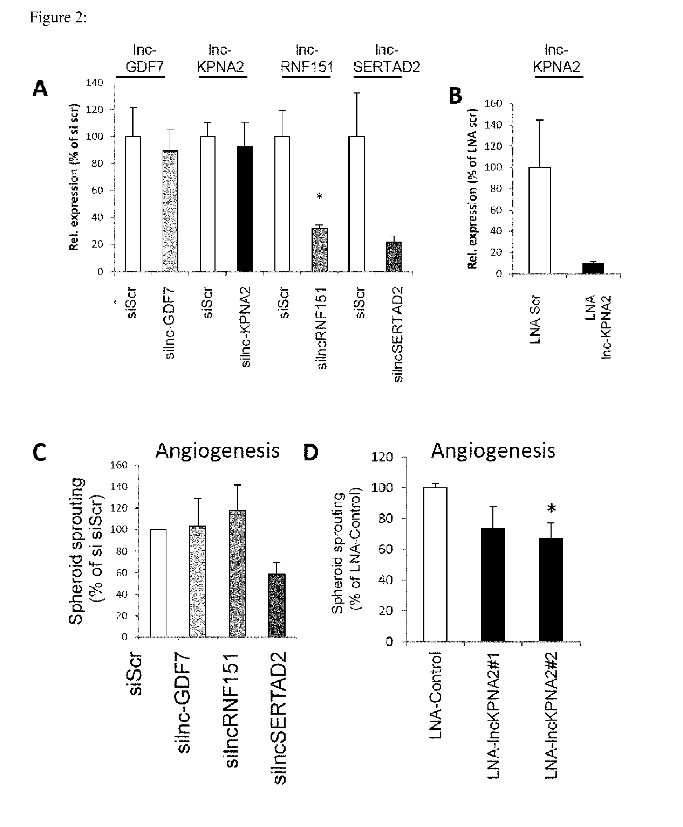

- **Figure 2:**: Angiogenic activity of flow-regulated lncRNAs. lncRNAs were silenced by siRNAs (panel A) or GapmeRs (panel B) and spheroid sprouting was determined (panel C/D).

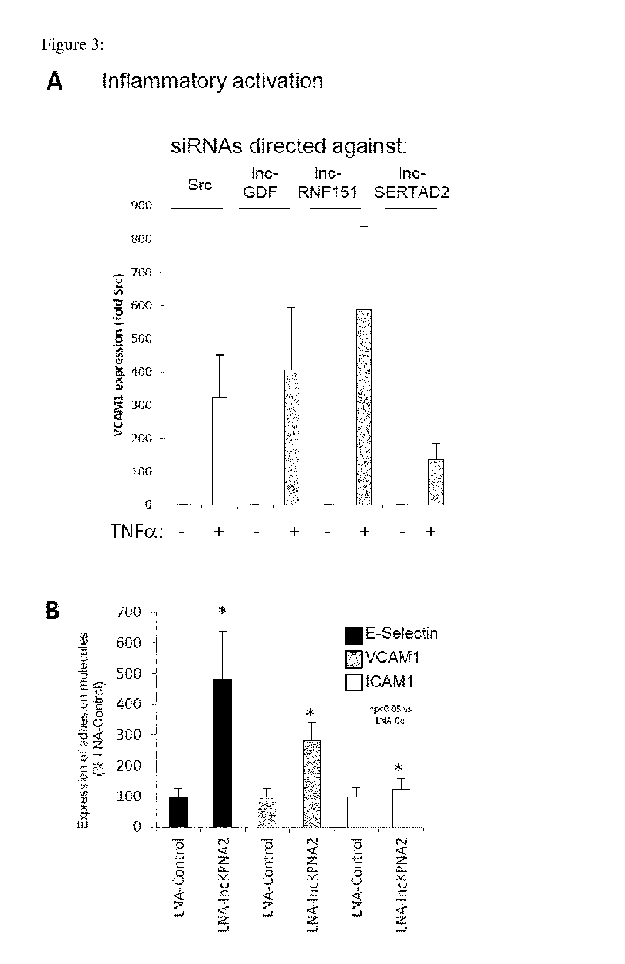

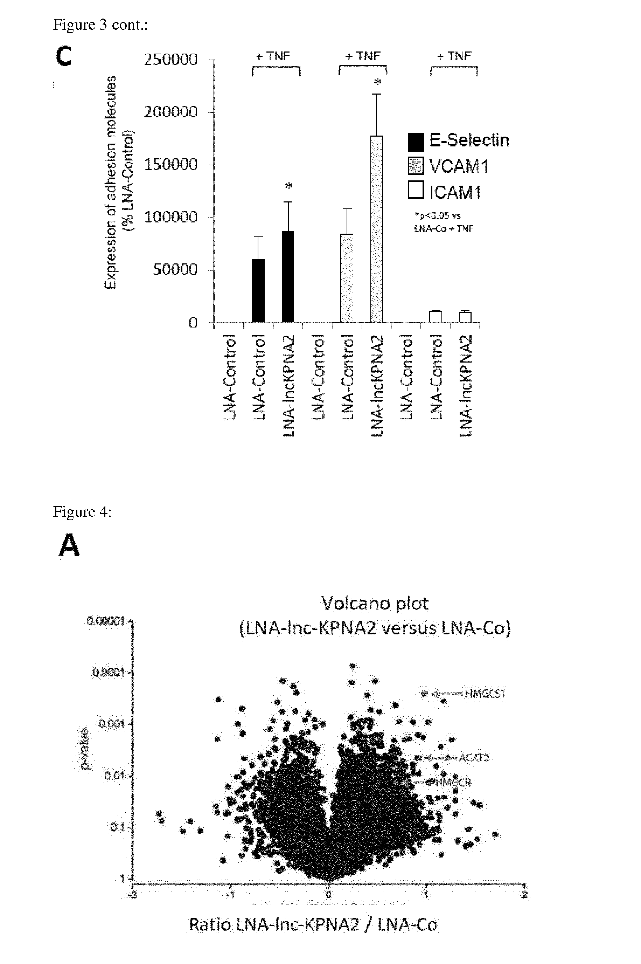

- **Figure 3:**: Regulation of endothelial inflammation by lncRNAs. A) VCAM1 expression after stimulation of siRNA transfected HUVEC with TNFa. B/C) Expression of E-Selectin, VCAM1 and ICAM1 mRNA after silencing of lnc-KPNA2 with GapmeRs. Basal expression is shown in panel B, TNFa-induction is shown in panel C.

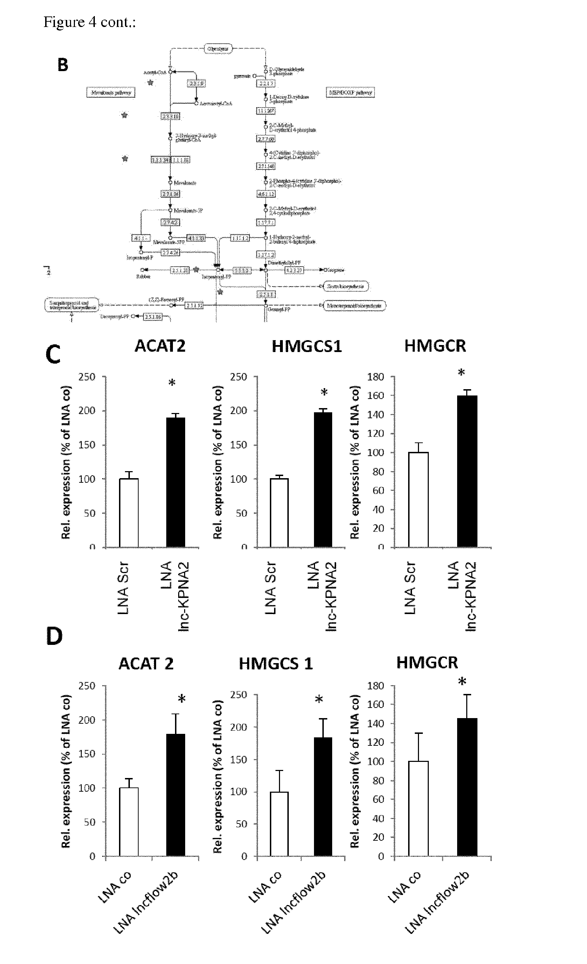

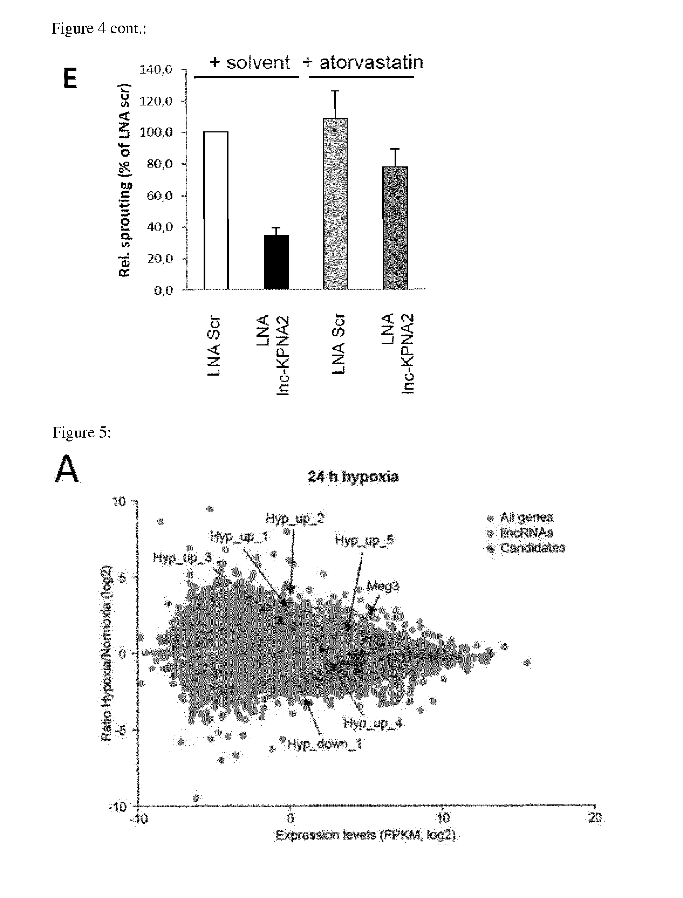

- **Figure 4:**: Gene expression analysis after silencing of lnc-KPNA2. A) Volcano blot showing gene expression changes after silencing of lnc-KPNA2 with GapmeRs. B) Bioinformatic pathway analysis showing regulated genes of the mevalonate pathway after lnc-KPNA2 silencing. C/D) Relative expression analysis of key enzymes of the mevalonate pathway by microarray (C) and validation by qRT-PCR. E) Spheroid sprouting after treatment of GapmeR control or lnc-KPNA2 silenced endothelial cells with or without atorvastatin.

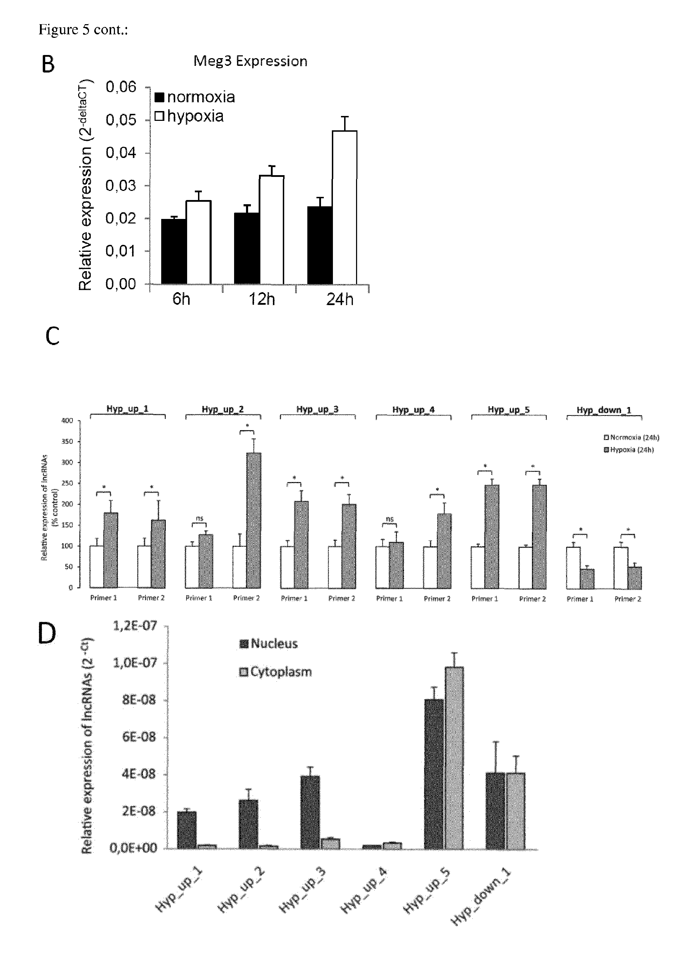

- **Figure 5:**: Regulation, validation and subcellular localization of lncRNAs in HUVEC by hypoxia. A) HUVEC were cultured under hypoxic conditions (0.2% 02) for 24 h or as control under normoxia. RNA was subjected to RNA-sequencing and hypoxia-mediated expression changes are plotted against the relative expression of the transcript. Candidate lncRNAs are highlighted in red. B/C) Validation of candidate lncRNAs by qRT-PCR. B) HUVEC were cultured under normoxic or hypoxic (0.2 % 02) conditions for 6 h, 12 h, and 24 h and the relative expression oflnc-Meg3 was analyzed by qRT-PCR. C) HUVEC were cultured under hypoxic conditions (0.2% 02) for 24 h or as control under normoxia. RNA was prepared and analyzed by qRT-PCR using two primer sets for each lncRNA. Data are n=4, *p<0.05, ns=not significant. D) Subcellular distribution of lncRNAs. Nuclear and cytoplasmic fractions were prepared from HUCEC grown under normoxic conditions and corresponding RNA samples were used for qRT-PCR detecting the indicated lncRNAs. Data are n=4.

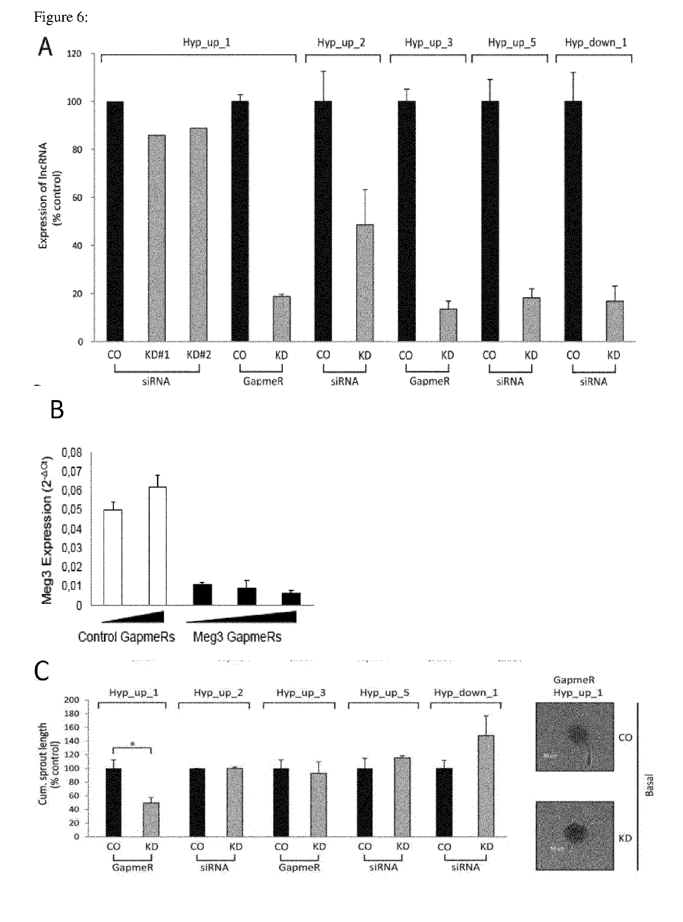

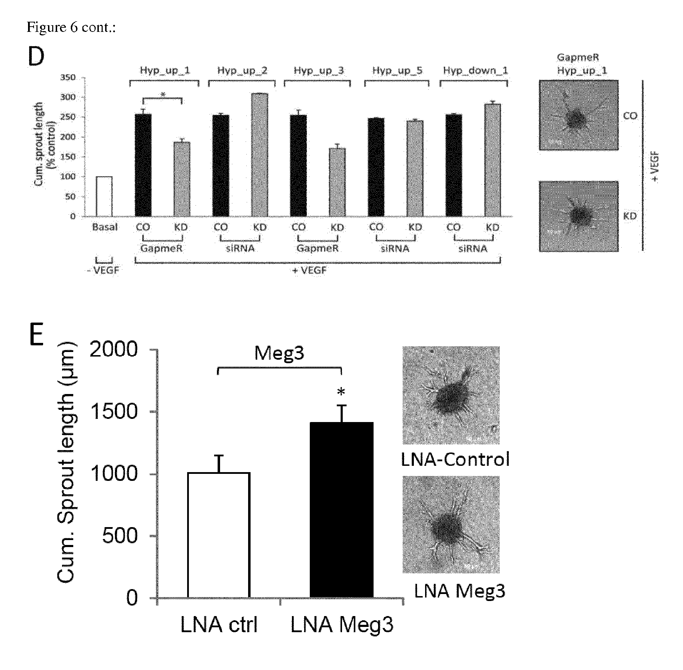

- **Figure 6:**: Effect of hypoxia-regulated lncRNAs on angiogenic sprouting of endothelial cells. A/B) GapmeRs (50nM) or siRNAs (60nM) targeting lnc-Hyp_up_1, lnc-Hyp_up_2, lnc-Hyp_up_3, Inc-Hyp_up_5, and lnc-Hyp_down_1 were transfected into HUVEC and lncRNA levels were quantified by qRT-PCR after 48 hours. For lnc-Meg3 silencing, HUVEC were transfected with increasing concentrations (5 to 50 nM) of GapmeRs and lnc-Meg3 expression was quantified by qRT-PCR. C) GapmeRs or siRNAs were transfected 48 hours before spheroids of HUVEC were allowed to sprout under basal conditions in a 3-dimensional matrix for 24 hours. Cumulative sprout length per spheroid was quantified and representative images are shown. D/E) GapmeRs or siRNAs were transfected 48 hours before spheroids of HUVEC were allowed to sprout under VEGF-stimulated conditions (50ng/ml, 24h) in a 3-dimensional matrix for 24 hours. Cumulative sprout length per spheroid was quantified and representative images are shown. CO = control siRNAs or GapmeRs; KD = knockdown with siRNAs or GapmeRs as indicated.*p<0.05 versus respective control.

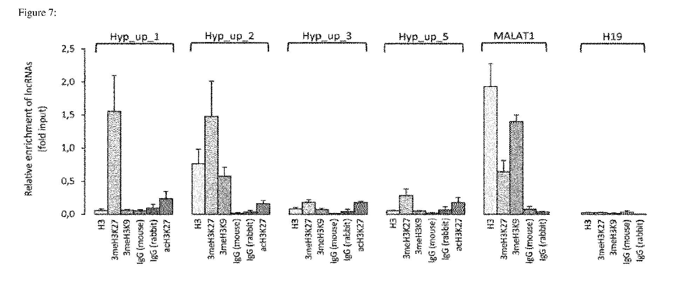

- **Figure 7:**: Histone association of candidate lncRNAs. Cell lysate was prepared from HUCEV cultured under normoxic conditions and used for RNA immunoprecipitations (RIP) using antibodies against histone H3, 3meH3K27, 3meH3K9, and acH3K27. As control, rabbit IgG was used. Co-precipitated lncRNAs (as indicated) were analyzed by qRT-PCR. Data are expressed as fold input. N>3.

SEQ ID NO 1 - 59 show the sequences of the lncRNA of the invention.

### EXAMPLES

### Example 1: Laminar flow regulated lncRNAs

To identify flow-induced lncRNAs, the inventors compared RNA sequencing data from HUVEC that were cultured under static conditions or were exposed to laminar shear stress for 72 h (20 dynes/cm²). As expected, expression levels of lncRNAs were lower compared to coding RNAs (Figure 1A). However, the change in gene expression was similar between coding and non-coding transcripts. Flow induced a > 2-fold up-regulation of 19 % of all lncRNAs analyzed (Figure 1B), whereas 15 % of lncRNAs decreased more than 2-fold. For further analysis, the inventors selected 5 candidates which are highly expressed in HUVECs, and of which 4 were increased by >1.5-fold and one was significantly suppressed by flow (Figure 1C/D). The flow dependent regulation of the selected lncRNAs was confirmed by qRT-PCR (Figure 1D). The inventors determined whether the selected lncRNAs are regulated by the flow-induced transcription factor KLF2 and found that lnc-KPNA2, lnc-SERTAD2 and lnc-00520 are induced after KLF2 overexpression in HUVECs.

### Example 2: lncRNA regulate endothelial cell function

The function of the selected lncRNAs was next studied by using siRNAs, which reduced expression of the targeted lnc-RNF151 and lnc-SERTAD2 (Figure 2A). Furthermore, lnc-KPNA2 was targeted by GapmeRs, which are RNase H-inducing antisense oligonucleotides (Figure 2B). Endothelial cell function was measured by using the spheroid outgrowth assay (Figure 2C/D). Silencing of lnc-SERTAD2 and lnc-KPNA2 reduced spheroid sprouting, whereas silencing of lnc-RNF151 had no effect. Since flow elicits a profound anti-inflammatory effect in endothelial cells, the inventors additionally determined the effect of lncRNAs on TNFa-induced inflammatory activation of ECs (Figure 3A). The inventors show that silencing of lnc-SERTAD2 reduces TNFa-induced expression of VCAM1, whereas silencing of lnc-RNF151 did not affect the inflammatory response. Since lnc-KPNA2 was highest expressed at baseline and showed a significant effect on EC functions, the inventors selected this lncRNA for further functional analysis.

### Example 3: Lnc-KPNA2 regulates the mevalonate pathway

To determine the molecular mechanism by which lnc-KPNA2 controls EC functions, the inventors performed gene expression profiling using microarrays (Figure 4A). KEGG analysis showed that metabolic pathways are critically regulated by lnc-KPNA2 silencing (Figure 4B). Of note, the mevalonate pathway, which is targeted by statins and critically controls EC functions, was significantly regulated by silencing of lnc-KPNA2 (Figure 3B). Particularly, aceto-acetyl-CoA transferase (ACAT2), HMG-CoA-synthase (HMGCS1), and HMG-CoA-reductase (HMGCR) were significantly increased after lnc-KPNA2 silencing (Figure 4C). These results were confirmed by qRT-PCR (Figure 4D). Since statins mediate their endothelial protective effect at least in parts by blocking the HMG-CoA-reductase, the inventors investigated whether the pharmacological inhibition of this enzyme may rescue the impaired function of lnc-KPNA2-silenced ECs. Indeed, atorvastatin partially reversed the inhibition of spheroid sprouting after lnc-KPNA2 GapmeR-treatment (Figure 4E).

In summary laminar flow regulates several lncRNAs, of which lnc-KPNA2 exhibit pro-angiogenic and anti-inflammatory activities in cultured endothelial cells. Inhibition of lnc-KPNA2 reduced spheroid sprouting and increased basal and TNFα-induced expression of adhesion molecules. Inhibition of lnc-SERTAD2 reduced spheroid sprouting and TNF-induced expression of VCAM1 suggesting that this lncRNA also promotes angiogenesis but enhances endothelial inflammatory activation.

### Example 4: Hypoxia-regulated lncRNAs

To systematically determine the regulation of lncRNAs by hypoxia, the inventors performed deep sequencing of RNA from endothelial cells after exposure to hypoxia (0.2 %) for 12 or 24 h. The inventors showed that 71 noncoding RNAs were significantly (p<0.01) up-regulated and 78 lncRNAs were down-regulated after 24 h hypoxia. Based on expression levels (FPKM > 1) and regulation by hypoxia, the inventors selected 6 uncharacterized candidates named lnc-Hyp_up_1 to lnc-Hyp_up_5 and lnc-Hyp_down_1 as well as lnc-Meg3 for further analysis (Figure 5A/B). Of the 6 candidates, 3 are annotated as lincRNAs and 3 are annotated as antisense transcripts. However, the antisense transcripts are not all complementary sequences to the mRNA. For example, lnc-Hyp_up_1 is transcribed from the reverse strand of the GATA6 locus, but it does not overlap with GATA6 mRNA. The inventors confirmed the up-regulation of lnc-Hyp_up_1, lnc-Hyp_up_5 and lnc-Hyp_up_5 and the down-regulation of lnc-Hyp_down_1 by using two sets of primers targeting different regions of the lncRNAs (Figure 5C). Only one of the two primer sets showed an increased expression of lnc-Hyp_up_2 and lnc-Hyp_up_4 (Figure 5C), suggesting that different isoforms may exist for these two lncRNAs. For lnc-Meg3 the inventors confirmed up-regulation by using a hypoxia time course experiment, ranging from 6 h to 24 h (Figure 5B). Depending on their subcellular localization, lncRNAs may comprise different functions. Whereas nuclear lncRNAs can change gene expression levels by altering chromatin states through recruitment chromatin modifying enzymes or modify alternative splicing, cytoplasmic localized lncRNAs may act as sponges for miRNAs or proteins. To gain first insights into the putative mode of action, the inventors determined the nuclear versus cytoplasmic localization of the hypoxia-regulated lncRNAs. The inventors showed that lnc-Hyp_up_1, lnc-Hyp_up_2, and lnc-Hyp_up_5 are predominately localized to the nucleus, whereas the other candidates were detected in the cytoplasm and the nucleus (Figure 5D).

Hypoxia controls the angiogenic function of endothelial cells. Therefore, it was next investigated whether the candidate lncRNAs affect endothelial cell sprouting as an in vitro measurement for the angiogenic response. Inhibition of lnc-Hyp_up_2, lnc-Hyp_up_5 and lnc-Hyp_down_1 was achieved by siRNAs (Figure 6A, data not shown) and alternatively by LNA™ GapmeRs, which are known to enter the nucleus and induce lncRNA degradation by an RNase H-dependent mechanism. By using GapmeRs the inventors achieved a significant >80% reduction of lnc-Hyp_up_1, lnc-Hyp_up_3, and lnc-Meg3 (Figure 6A/B). Next, the inventors used the established siRNAs or GapmeRs to determine the biological function of the hypoxia-regulated lncRNAs.

It could be shown that basal sprouting is significantly reduced by silencing of lnc-Hyp_up_1, whereas inhibition of lnc-Hyp_down_1 and lnc-Meg3 increased basal sprouting (Figure 6C/E). All other tested hypoxia-regulated lncRNAs showed no effect on basal angiogenic sprouting (Figure 6C). Next it was tested, whether the selected lncRNAs are also involved in VEGF-stimulated angiogenic sprouting of endothelial cells. Endothelial sprouting was significantly decreased after silencing of lnc-Hyp_up_1 and lnc-Hyp_up_5 but was increased by siRNAs directed against lnc-Hyp_down_1 (Figure 6D).

In order to functionally characterize our selected lncRNAs, their association with histones was determined. In general, acetylation of histones H3 and H4 is associated with increased gene expression, whereas the effect of histone methylation on transcription depends on the lysine acceptor site and the number of methyl groups attached. Methylation can occur at several lysine (K) residues and the residues can be mono, di-, or tri-methylated. Histone H3 modifications at K4 and K36 are associated with activation of gene expression, whereas K27 trimethylation (3meH3K27) and K9 trimethylation (3meH3K9) at promoter sites are repressive. Lnc-Hyp_up_1 and lnc-Hyp_up_2 were enriched in RNA immunoprecipitates of 3meH3K27 but not in precipitates of acetylated H3K27 (acH3K27) or 3meH3K9 (Figure 7), suggesting that these lncRNAs are predominantly associated with repressive 3meH3K27 chromatin marks and may be involved in epigenetic gene silencing. Controls with other known non-coding RNAs such as lnc-MALAT1, a nuclear enriched lncRNA, which predominantly interacts with H3 and H3K9me3, and H19, which is predominantly localized to the cytoplasm and does not interact with histone complexes, confirm that the association of the newly identified hypoxia-regulated lncRNAs with modified histones appears to be specific (Figure 7).

In summary it was shown that 1) lnc-Hyp_up_1 is sufficiently expressed (FPKM > 1) under baseline conditions, 2) is significantly up-regulated by hypoxia, 3) controls angiogenic sprouting in vivo, 4) is highly enriched in the nuclear fraction, and 5) is strongly associated with silencing 3meH3K27 histone complexes suggesting a putative epigenetic function. In contrast, lnc-Hyp_down_1 and lnc-Meg3 exhibit an anti-angiogenic activity as demonstrated by the increased sprouting observed after silencing of these lncRNAs.

## Claims

1. A compound for use in the treatment of a disease associated with endothelial cell dysfunction, wherein the compound is an agonist or is an antagonist of a long non-coding RNA (lncRNA) selected from the group consisting of lnc-KPNA2, lnc-SERTAD2, lnc-Hyp_up_1, lnc-Hyp_down_1, lnc-Meg3, lnc-RNF151, lnc-Hyp_up_3, lnc-Hyp_up_5 and lnc-GDF7.

2. The compound for use according to claim 1, wherein the disease associated with endothelial cell dysfunction is pathological angiogenesis, and wherein the compound is
a. An antagonist of a long non-coding RNA (lncRNA) selected from the group consisting of lnc-KPNA2, lnc-SERTAD2, and lnc-Hyp_up_1, or
b. An agonist of an lncRNA selected from lnc-Hyp_down_1 and lnc-Meg3.

3. The compound for use according to claim 2, wherein the pathologic angiogenesis occurs in connection with a proliferative disorder and wherein the proliferative disorder is cancer, preferably selected from the group consisting of liver cancer, lung cancer, breast cancer, colorectal cancer, stomach cancer and melanoma, most preferably wherein the cancer is solid cancer, even more preferably a metastatic solid cancer.

4. The compound for use according to claim 1, wherein the disease associated with endothelial cell dysfunction is a cardiovascular disease, and wherein the compound is
a. An agonist of a long non-coding RNA (lncRNA) selected from the group consisting of lnc-KPNA2, lnc-SERTAD2, and lnc-Hyp_up_1, or
b. An antagonist of an lncRNA selected from lnc-Hyp_down_1 and lnc-Meg3.

5. The compound for use according to claim 4, wherein the cardiovascular disease is a disease associated with a pathological repressed endothelial cell repair, cell growth and/or cell division or is a disease treatable by improving endothelial cell repair, cell growth and/or cell division.

6. The compound according to any of claims 1 to 5, wherein an lncRNA agonist is selected from the group of an lncRNA molecule, or a homolog thereof or an lncRNA expression construct, or a molecule that when contacted with a cell induces or increases the expression and or function of the lncRNA; and/or wherein an antagonist of a lncRNA is selected from, an antagonistic peptide, an antagonistic small mocleucle, or an inhibitory nucleic acid, preferably an antisense oligonucleotide, a miRNA, siRNA, dsRNA, LNA, GapmeR, or a molecule that represses the expression and/or function of a lncRNA.

7. An *in-vitro* or *ex-vivo* method for modulating endothelial cell function comprising contacting an endothelial cell with an agonist or antagonist of a lncRNA selected from the group consisting of lnc-KPNA2, lnc-SERTAD2, lnc-Hyp_up_1, lnc-Hyp_down_1 and lnc-Meg3

8. The method according to claim 7, wherein the endothelial cell function is a function selected from endothelial cell repair, endothelial cell growth and/or endothelial cell division.

9. An *in-vitro* or *ex-vivo* method for the generation of vascular tissue material, the method comprising the steps of contacting an endothelial cell with an agonist of a lncRNA, wherein the lncRNA is selected from the group consisting of lnc-KPNA2, lnc-SERTAD2, and lnc-Hyp_up_1, or an antagonist of a lncRNA selected from lnc-Hyp_down_1 and lnc-Meg3.

10. The method according to claim 9, wherein the vascular tissue material is a blood vessel.

11. The method according to any of claims 9 or 10, wherein an lncRNA agonist is selected from the group of an lnc-RNA molecule, a chemically modified lnc-RNA molecule, or an lncRNA expression construct, or a molecule that when contacted with a cell induces or increases the expression and or function of the lncRNA.

12. The method according to any one of claims 9 to 11, wherein an antagonist of a lncRNA is selected from an inhibitory nucleic acid, such as a miRNA, siRNA, dsRNA, LNA, GapmeR, or a molecule that represses the expression and/or function of a lncRNA.

13. A pharmaceutical composition comprising an agonist or antagonist of a long non-coding RNA (lncRNA) selected from the group consisting of lnc-KPNA2, lnc-SERTAD2, lnc-Hyp_up_1, lnc-Hyp_down_1, lnc-Meg3, lnc-RNF151, lnc-Hyp_up_3, lnc-Hyp_up_5 and lnc-GDF7, optionally further comprising a pharmaceutically acceptable carrier and/or excipient.

14. The pharmaceutical composition according to claim 13, for use for use in the treatment of a disease associated with endothelial cell dysfunction according to any of claims 1 to 6.

15. An *in-vitro* use of an agonist or antagonist of a long non-coding RNA (lncRNA) selected from the group consisting of lnc-KPNA2, lnc-SERTAD2, lnc-Hyp_up_1, lnc-Hyp_down_1, lnc-Meg3, lnc-RNF151, lnc-Hyp_up_3, lnc-Hyp_up_5 and lnc-GDF7, in the modulation of endothelial cell function.
